# EUROPEAN PATENT APPLICATION

(11) **EP 2 289 500 A1**
(43) Date of publication of application: **02.03.2011**
(21) Application number: 10181763.3
(22) Date of filing: 07.12.2005
(51) Int. Cl.: A61K 31/165, A61P 9/00, A61P 25/28

(54) **Retinol binding protein and transthyretin modulators for treating hypertosis, Alzheimer's disease or idiopathic intracranial hypertension**

(30) Priority: 08.12.2004 US 634449 P; 10.03.2005 US 660924 P; 11.03.2005 US 660904 P; 18.04.2005 US 672405 P; 11.07.2005 US 698512 P
(62) Divisional of application: 07020279.1
(71) Applicant: ReVision Therapeutics, Inc., San Diego, CA 92121 (US)
(72) Inventor: Widder, Kenneth, Rancho Santa Fe, California 92067 (US); Lichter, Jay, San Diego, California 92130 (US)
(74) Representative: Cole, William Gwyn

(57) **Abstract**

Described herein are methods and compositions for treating certain retinol-related diseases and conditions by modulation of transthyretin (TTR) and retinol binding protein (RBP) availability in the subject. For example, the methods and compositions provide for therapeutic agents for the treatment and/or prevention of age-related macular degeneration and/or dystrophies, metabolic disorders, idiopathic intracranial hypertension, hyperostosis, and protein misfolding and aggregation diseases. The compositions disclosed may be used as single agent therapy or in combination with other agents or therapies. In addition, described herein are methods and assays for selecting appropriate agents that can modulate the TTR and RBP availability in a subject.

## Description

### RELATED APPLICATIONS

This patent application claims the benefit of (a) U.S. Provisional Application Ser. No. 60/634,449, filed December 8, 2004, (b) U.S. Provisional Application Ser. No. 60/660,924, filed March 10, 2005, (c) U.S. Provisional Application serial number 60/660,904, filed on March 11, 2005, (d) U.S. Provisional Application serial number 60/672,405, filed on April 18, 2005, and (e) U.S. Provisional Application serial number 60/698,512, filed on July 11, 2005; the aforementioned patent applications are herein incorporated by reference in their entirety.

### FIELD OF THE INVENTION

The methods and compositions described herein are directed to the treatment of retinol-related diseases in a subject by modulating the activity or availability of retinol binding protein (RBP) and transthyretin (TTR) in the subject.

### BACKGROUND OF THE INVENTION

Retinoids are essential for maintenance of normal growth, development, immunity, reproduction, vision and other physiological processes. Conversely, abnormal production or processing of retinoids correlates with the manifestation of disease processes.

For example, more than 100 million of the world's children are vitamin-A deficient, causing blindness and death among these children. Excess vitamin-A levels in target organs and tissues, such as the eye, may also cause blindness in a variety of retinal diseases, including macular degeneration. A large variety of conditions, generally referred to as vitreoretinal diseases, can affect the vitreous and retina that lie on the back part of the eye, including the retinopathies and macular degenerations and dystrophies. Macular degeneration is a group of eye diseases that is the leading cause of blindness for those aged 55 and older in the United States, affecting more than 10 million Americans. Some studies predict a six-fold increase in the number of new cases of macular degeneration over the next decade, taking on the characteristics of an epidemic. Age-related macular degeneration or dystrophy, a particularly debilitating disease, leads to gradual loss of vision and eventually severe damage to the central vision.

Abnormal levels of vitamin A, and/or its associated transport proteins (retinol binding protein (RBP) and transthyretin (TTR)) are also correlated with the manifestation of other diseases, including metabolic disorders. An example is seen in diabetes, where abnormal levels of retinol were seen in both type I and type II diabetic patients, but not normal patients. Other diseases include pseudotumor cerebri (PTC), idiopathic intracranial hypertension (IIH), and bone-related disorders, including cervical spondylosis, spinal hyperostosis, and diffuse idiopathic skeletal hyperostosis (DISH). In addition, vitamin A and/or its associated transport proteins, TTR in particular, may play a role in protein misfolding and aggregation diseases, including Alzheimer's disease and systemic amyloidosis.

Disorders associated with retinoid-related physiological manifestations continue to be a problem throughout the world. Therefore, there is a need to provide for methods and compositions to treat these diseases.

### SUMMARY OF THE INVENTION

Described herein are methods and compositions for identifying and detecting agents which modulate retinol binding protein (RBP) or transthyretin (TTR) levels or activity in a mammal. Also described herein are assays for identifying compounds and therapeutic agents, as well as methods and compositions for treating a subject or patient with retinol-related diseases by administration of compounds or therapeutics agents, wherein said administration results in the modulation of RBP or TTR levels or activity in said patient or subject. Also described herein are methods and compositions for treating a patient with retinol-related diseases by modulating RBP or TTR levels or activity in the patient by administration of such compounds.

In one embodiment, the methods and compositions disclosed herein provide for the modulation of RBP or TTR levels or activity in a mammal comprising administering to the mammal at least once an effective amount of an agent which modulates RBP or TTR transcription in said mammal, wherein said modulation of RBP or TTR levels or activity reduces the formation of all-trans retinal in an eye of a mammal. In one embodiment, the agent is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In yet another embodiment, the methods and compositions disclosed herein provide for modulating RBP or TTR levels or activity in a mammal comprising administering to the mammal at least once an effective amount of an RBP or TTR translation inhibitor, wherein said modulation of RBP or TTR levels or activity reduces the formation of all-trans retinal in an eye of a mammal. The agent may be chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In one embodiment, the methods and compositions disclosed herein provide for modulating RBP or TTR levels or activity in a mammal comprising administering to the mammal at least once an effective amount of an agent which modulates RBP binding to TTR in said mammal, wherein said modulation of RBP or TTR levels or activity reduces the formation of all-trans retinal in an eye of a mammal. The modulating agent can bind to RBP or TTR so as to inhibit the binding of RBP to TTR in the mammal. The modulating agent can also antagonize the binding of retinol to RBP so as to inhibit the binding of RBP or the RBP-agent complex to TTR. The modulating agent may be chosen from the group consisting of: a retinyl derivative, a polyhalogenated aromatic hydrocarbon, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the retinyl derivative of the methods and compositions disclosed herein is a compound having the structure: 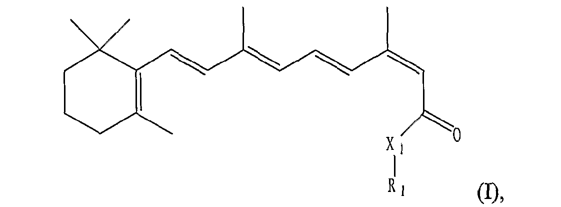 wherein X₁ is selected from the group consisting of NR², O, S, CHR²; R₁ is (CHR²)ₓ-L¹-R³, wherein x is 0, 1, 2, or 3; L¹ is a single bond or -C(O)-; R² is a moiety selected from the group consisting ofH, (C₁-C₄)alkyl, F, (C₁-C₄)fluoroalkyl, (C₁-C₄)alkoxy, -C(O)OH, -C(O)-NE₂, -(C₁-C₄)alkylamine, -C(O)-(C₁-C₄)alkyl, -C(O)-(C₁-C₄)fluoralkyl, -C(O)-(C₁-C₄)alkylamine, and -C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloalkenyl, and a heterocycle; or an active metabolite, or a pharmaceutically acceptable prodrug or solvate thereof.

In one embodiment, the retinyl derivative of the methods and compositions disclosed herein is a compound having the structure: 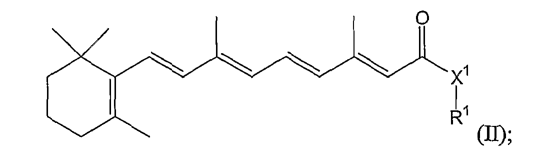 wherein X¹ is selected from the group consisting of NR², O, S, CHR²; R¹ is (CHR²)ₓ-L¹-R³, wherein x is 0, 1, 2, or 3; L¹ is a single bond or -C(O)-; R² is a moiety selected from the group consisting ofH, (C₁-C₄)alkyl, F, (C₁-C₄)fluoroalkyl, (C₁-C₄)alkoxy, -C(O)OH, -C(O)-NH₂, -(C₁-C₄)alkylamine, -C(O)-(C₁-C₄)alkyl, -C(O)-(C₁-C₄)fluoroalkyl, -C(O)-(C₁-C₄)alkylamine, and -C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloalkenyl, and a heterocycle; or an active metabolite, or a pharmaceutically acceptable prodrug or solvate thereof.

In further embodiments (a) X¹ is NR², wherein R² is H or (C₁-C₄)alkyl; (b) x is 0; (c) x is 1 and L¹ is -C(O)-; (d) R³ is an optionally substituted aryl; (e) R³ is an optionally substituted heteroaryl; (f) X¹ is NH and R³ is an optionally substituted aryl, including yet further embodiments in which (i) the aryl group has one substituent, (ii) the aryl group has one substituent selected from the group consisting of halogen, OH, O(C₁-C₄)alkyl, NH(C₁-C₄)alkyl, O(C₁-C₄)fluoroalkyl, and N[(C₁-C₄)alkyl]₂, (iii) the aryl group has one substituent, which is OH, (v) the aryl is a phenyl, or (vi) the aryl is naphthyl; (g) the compound is 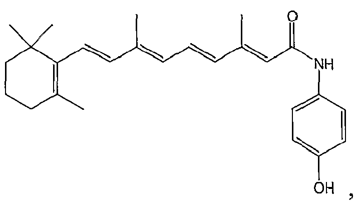 or an active metabolite, or a pharmaceutically acceptable prodrug or solvate thereof; (h) the compound is 4-hydroxyphenylretinamide, or a metabolite, or a pharmaceutically acceptable prodrug or solvate thereof; (i) the compound is 4-methoxyphenylretinamide, or (j) 4-oxo fenretinide, or a metabolite, or a pharmaceutically acceptable prodrug or solvate thereof.

In further embodiments, the administration of a compound of Formula (II) is used to treat ophthalmic conditions by lowering the levels of serum retinol in the body of a patient. In further embodiments (a) the effective amount of the compound is systemically administered to the mammal; (b) the effective amount of the compound is administered orally to the mammal; (c) the effective amount of the compound is intravenously administered to the mammal; (d) the effective amount of the compound is ophthalmically administered to the mammal; (e) the effective amount of the compound is administered by iontophoresis; or (f) the effective amount of the compound is administered by injection to the mammal.

In further embodiments the mammal is a human, including embodiments wherein (a) the human is a carrier of the mutant *ABCA4* gene for Stargardt Disease or the human has a mutant *ELOV4* gene for Stargardt Disease, or has a genetic variation in complement factor H associated with age-related macular degeneration, or (b) the human has an ophthalmic condition or trait selected from the group consisting of Stargardt Disease, recessive retinitis pigmentosa, geographic atrophy (of which scotoma is one non-limiting example), photoreceptor degeneration, dry-form AMD, recessive cone-rod dystrophy, exudative (or wet-form) age-related macular degeneration, cone-rod dystrophy, and retinitis pigmentosa. In further embodiments the mammal is an animal model for retinal degeneration.

In further embodiments, are methods comprising multiple administrations of the effective amount of the agent which modulates RBP binding to TTR in said mammal, including further embodiments in which (i) the time between multiple administrations is at least one week; (ii) the time between multiple administrations is at least one day; and (iii) the compound is administered to the mammal on a daily basis; or (iv) the compound is administered to the mammal every 12 hours. In further or alternative embodiments, the method comprises a drug holiday, wherein the administration of the compound is temporarily suspended or the dose of the compound being administered is temporarily reduced; at the end of the drug holiday, dosing of the compound is resumed. The length of the drug holiday can vary from 2 days to 1 year.

In further embodiments are methods comprising administering at least one additional agent selected from the group consisting of an inducer of nitric oxide production, an anti-inflammatory agent, a physiologically acceptable antioxidant, a physiologically acceptable mineral, a negatively charged phospholipid, a carotenoid, a statin, an anti-angiogenic drug, a matrix metalloproteinase inhibitor, 13-*cis-*retinoic acid (including derivatives of 13-*cis*-retinoic acid), 11-*cis*-retinoic acid (including derivatives of 11-*cis*-retinoic acid), 9-*cis*-retinoic acid (including derivatives of 9-*cis*-retinoic acid), and retinylamine derivatives. In further embodiments:
(a) the additional agent is an inducer of nitric oxide production, including embodiments in which the inducer of nitric oxide production is selected from the group consisting of citrulline, ornithine, nitrosated *L-*arginine, nitrosylated *L*-arginine, nitrosated *N*-hydroxy-*L*-arginine, nitrosylated *N*-hydroxy-*L*-arginine, nitrosated *L*-homoarginine and nitrosylated *L-*homoarginine;
(b) the additional agent is an anti-inflammatory agent, including embodiments in which the anti-inflammatory agent is selected from the group consisting of a non-steroidal anti-inflammatory drug, a lipoxygenase inhibitor, prednisone, dexamethasone, and a cyclooxygenase inhibitor;
(c) the additional agent is at least one physiologically acceptable antioxidant, including embodiments in which the physiologically acceptable antioxidant is selected from the group consisting of Vitamin C, Vitamin E, beta-carotene, Coenzyme Q, and 4-hydroxy-2,2,6,6-tetramethylpiperadine-*N*-oxyl, or embodiments in which (i) the at least one physiologically acceptable antioxidant is administered with the agent which modulates RBP binding to TTR in said mammal, or (ii) at least two physiologically acceptable antioxidants are administered with the agent which modulates RBP binding to TTR in said mammal;
(d) the additional agent is at least one physiologically acceptable mineral, including embodiments in which the physiologically acceptable mineral is selected from the group consisting of a zinc (II) compound, a Cu(II) compound, and a selenium (II) compound, or embodiments further comprising administering to the mammal at least one physiologically acceptable antioxidant;
(e) the additional agent is a negatively charged phospholipid, including embodiments in which the negatively charged phospholipid is phosphatidylglycerol;
(f) the additional agent is a carotenoid, including embodiments in which the carotenoid is selected from the group consisting of lutein and zeaxanthin;
(g) the additional agent is a statin, including embodiments in which the statin is selected from the group consisting of rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium, and dihydrocompactin;
(h) the additional agent is an anti-angiogenic drug, including embodiments in which the the anti-angiogenic drug is Rhufab V2, Tryptophanyl-tRNA synthetase, an Anti-VEGF pegylated aptamer, Squalamine, anecortave acetate, Combretastatin A4 Prodrug, Macugen^{™}, mifepristone, subtenon triamcinolone acetonide, intravitreal crystalline triamcinolone acetonide, AG3340, fluocinolone acetonide, and VEGF-Trap;
(i) the additional agent is a matrix metalloproteinase inhibitor, including embodiments in which the matrix metalloproteinase inhibitor is a tissue inhibitors of metalloproteinases, α₂-macroglobulin, a tetracycline, a hydroxamate, a chelator, a synthetic MMP fragment, a succinyl mercaptopurine, a phosphonamidate, and a hydroxaminic acid;
(j) the additional agent is a complement inhibitor, including by way of example only, antibodies against C1, C2, C3, C4, C5, C6, C7, C8, and C9, such as those disclosed in U.S. Pat. Nos. 5,635,178; 5,843,884; 5,847,082; 5,853,722; and in Rollins et al.; Transplantation, 60:1284-1292 (1995) (the contents of all of which are incorporated herein by reference);
(k) the additional agent is a fish oil, including by way of example only, omega 3 fatty acids;
(l) the additional agent is 13-*cis*-retinoic acid (including derivatives of 13-*cis*-retinoic acid), 11-*cis*-retinoic acid (including derivatives of 11-*cis*-retinoic acid), or 9-*cis*-retinoic acid (including derivatives of 9-*cis*-retinoic acid);
(m) the additional agent is a retinylamine derivative, including an all-*trans*-retinylamine derivative, a 13-*cis*-retinylamine derivative, a 11-*cis*-retinylamine derivative, or a 9-*cis-*retinylamine derivative;
(n) the additional agent is administered (i) prior to the administration of the agent which modulates RBP binding to TTR in said mammal, (ii) subsequent to the administration of the agent which modulates RBP binding to TTR in said mammal, (iii) simultaneously with the administration of the agent which modulates RBP binding to TTR in said mammal, or (iv) both prior and subsequent to the administration of agent which modulates RBP binding to TTR in said mammal; or
(o) the additional agent and agent which modulates RBP binding to TTR in said mammal, are administered in the same pharmaceutical composition.

In further embodiments are methods comprising administering extracorporeal rheopheresis to the mammal. In further embodiments are methods comprising administering to the mammal a therapy selected from the group consisting of limited retinal translocation, photodynamic therapy, drusen lasering, macular hole surgery, macular translocation surgery, Phi-Motion, Proton Beam Therapy, Retinal Detachment and Vitreous Surgery, Scleral Buckle, Submacular Surgery, Transpupillary Thermotherapy, Photosystem I therapy, MicroCurrent Stimulation, anti-inflammatory agents, RNA interference, administration of eye medications such as phospholine iodide or echothiophate or carbonic anhydrase inhibitors, microchip implantation, stem cell therapy, gene replacement therapy, ribozyme gene therapy, photoreceptor/retinal cells transplantation, and acupuncture.

In further embodiments are methods comprising the use of laser photocoagulation to remove drusen from the eye of the mammal.

In further embodiments are methods comprising administering to the mammal at least once an effective amount of a second agent which modulates RBP binding to TTR in said mammal, wherein the first compound is different from the second compound.

In further embodiments, an apparatus capable of detecting and/or quantitating retinol-RBP-TTR complex formation is provided, wherein at least a portion of the TTR is fluorescently labeled.

In one embodiment, the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. In another embodiment, the polyhalogenated aromatic hydrocarbon is a hydroxylated polyhalogenated aromatic hydrocarbon metabolite. The hydroxylated polyhalogenated aromatic hydrocarbon metabolite may be a hydroxylated polychlorinated biphenyl metabolite. In yet another embodiment, the diclofenac analogue of the methods and compositions disclosed herein may be chosen from the group consisting of: 2-[(2,6-dichlorophenyl)amino]benzoic acid; 2-[(3,5-dichlorophenyl)amino]benzoic acid; 3,5,-dichloro-4-[(4-nitrophenyl)amino]benzoic acid; 2-[(3,5-dichlorophenyl)amino]benzene acetic acid and 2-[(2,6-dichloro-4-carboxylic acid-phenyl)amino]benzene acetic acid.

In other embodiments, the non-steroidal anti-inflammatory agent of the methods and compositions disclosed herein may be flufenamic acid, diflunisal, a diflunisal analogue, diclofenamic acid, indomethacin, niflumic acid or sulindac. In one embodiment, the diflunisal analogue may be 3',5'-difluorobiphenyl-3-ol; 2',4'-diflurobiphenyl-3-carboxylic acid; 2',4'-difluorobiphenyl-4-carboxylic acid; 2'-fluorobiphenyl-3-carboxylic acid; 2'-fluorobiphenyl-4-carboxylic acid; 3',5'-difluorobiphenyl-3-carboxylic acid; 3',5'-difluorobiphenyl-4-carboxylic acid; 2',6'-difluorobiphenyl-3-carboxylic acid; 2'6'-difluorobiphenyl-4-carboxylic acid; biphenyl-4-carboxylic acid; 4'fluoro-4-hydroxybiphenyl-3-carboxylic acid; 2'-fluoro-4-hydroxybiphenyl-3-carboxylic acid; 3',5'-difluoro-4-hydroxybiphenyl-3-carboxylic acid; 2',4'-dichloro-4-hydroxybiphenyl-3-carboxylic acid; 4-hydroxybiphenyl-3-carboxylic acid; 3'5'-difluoro-4'hydroxybiphenyl-3-carboxylic acid; 3',5'- difluoro-4'hydroxybiphenyl-4-carboxylic acid; 3',5'- dichloro-4'hydroxybiphenyl-3-carboxylic acid; 3',5'- dichloro-4'hydroxybiphenyl-4-carboxylic acid; 3',5'-dichloro-3-formylbiphenyl; 3',5'-dichloro-2-formylbiphenyl; 2',4'-dichlorobiphenyl-3-carboxylic acid; 2',4'-dichlorobiphenyl-4-carboxylic acid; 3',5'-dichlorobiphenyl-3-yl-methanol; 3',5'-dichlorobiphenyl-4-yl-methanol; or 3',5'-dichlorobiphenyl-2-yl-methanol.

In other embodiments, the flavonoid of the methods and compositions disclosed herein may be 3-methyl-4',6-dihydroxy-3',5'-dibromoflavone or 3',5'-dibromo-2',4,4',6-tetrahydroxyaurone. In yet another embodiment, the cardiac agent of the methods and compositions disclosed herein is milrinone.

In another embodiment, the small molecule of the methods and compositions disclosed herein is N-phenylanthranilic acid, methyl red, mordant orange I, bisarylamine, N-benzyl-p-aminobenzoic acid, furosamide, apigenin, resveratrol, biarylamine or dibenzofuran. In one embodiment, the thyroid hormone analogue may be thyroxine-propionic acid, thyroxine-acetic acid, or SKF94901.

The methods and compositions disclosed herein also provide for modulating RBP or TTR levels or activity in a mammal comprising administering to the mammal at least once an effective amount of an agent which increases the clearance rate of RBP or TTR in said mammal, wherein said modulation of RBP or TTR levels or activity reduces the formation of all-*trans* retinal in an eye of a mammal. In one embodiment, the agent may be chosen from the group consisting of: a retinyl derivative, a polyhalogenated aromatic hydrocarbon, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the retinyl derivative is a compound having the structure: 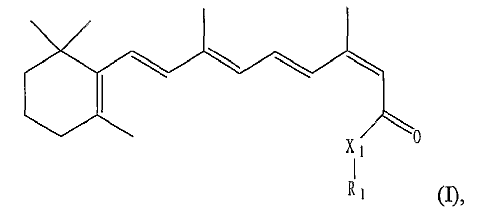 wherein X¹ is selected from the group consisting of NR², O, S, CHR²; R₁ is (CHR²)ₓ-L¹-R³, wherein x is 0, 1, 2, or 3; L¹ is a single bond or -C(O)-; R² is a moiety selected from the group consisting of H, (C₁-C₄)alkyl, F, (C₁-C₄)fluoroalkyl, (C₁-C₄)alkoxy, -C(O)OH, -C(O)-NH₂, -(C₁-C₄)alkylamine, -C(O)-(C₁-C₄)alkyl, -C(O)-(C₁-C₄)fluoralkyl, -C(O)-(C₁-C₄)alkylamine, and -C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloalkenyl, and a heterocycle; or an active metabolite, or a pharmaceutically acceptable prodrug or solvate thereof.

In one embodiment, the retinyl derivative is a compound having the structure: 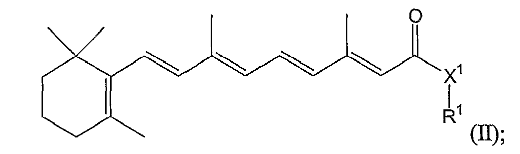 wherein X¹ is selected from the group consisting of NR², O, S, CHR²; R¹ is (CHR²)ₓ-L¹-R³, wherein x is 0, 1, 2, or 3; L¹ is a single bond or -C(O)-; R² is a moiety selected from the group consisting of H, (C₁-C₄)alkyl, F, (C₁-C₄)fluoroalkyl, (C₁-C₄)alkoxy, -C(O)OH, -C(O)-NH₂, -(C₁-C₄)alkylamine, -C(O)-(C₁-C₄)alkyl, -C(O)-(C₁-C₄)fluoroalkyl, -C(O)-(C₁-C₄)alkylamine, and -C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloalkenyl, and a heterocycle; or an active metabolite, or a pharmaceutically acceptable prodrug or solvate thereof.

In further embodiments (a) X¹ is NR², wherein R² is H or (C₁-C₄)alkyl; (b) x is 0; (c) x is 1 and L¹ is -C(O)-; (d) R³ is an optionally substituted aryl; (e) R³ is an optionally substituted heteroaryl; (f) X¹ is NH and R³ is an optionally substituted aryl, including yet further embodiments in which (i) the aryl group has one substituent, (ii) the aryl group has one substituent selected from the group consisting of halogen, OH, O(C₁-C₄)alkyl, NH(C₁-C₄)alkyl, O(C₁-C₄)fluoroalkyl, and N[(C₁-C₄)alkyl]₂, (iii) the aryl group has one substituent, which is OH, (v) the aryl is a phenyl, or (vi) the aryl is naphthyl; (g) the compound is 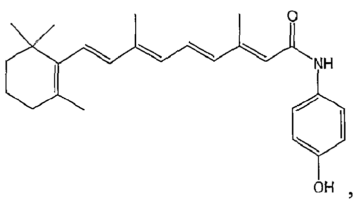 or an active metabolite, or a pharmaceutically acceptable prodrug or solvate thereof; (h) the compound is 4-hydroxyphenylretinamide, or a metabolite, or a pharmaceutically acceptable prodrug or solvate thereof; (i) the compound is 4-methoxyphenylretinamide, or (j) 4-oxo fenretinide, or a metabolite, or a pharmaceutically acceptable prodrug or solvate thereof.

In further embodiments, the administration of a compound of Formula (II) is used to treat ophthalmic conditions by lowering the levels of serum retinol in the body of a patient. In further embodiments (a) the effective amount of the compound is systemically administered to the mammal; (b) the effective amount of the compound is administered orally to the mammal; (c) the effective amount of the compound is intravenously administered to the mammal; (d) the effective amount of the compound is ophthalmically administered to the mammal; (e) the effective amount of the compound is administered by iontophoresis; or (f) the effective amount of the compound is administered by injection to the mammal.

In further embodiments the mammal is a human, including embodiments wherein (a) the human is a carrier of the mutant *ABCA4* gene for Stargardt Disease or the human has a mutant *ELOV4* gene for Stargardt Disease, or has a genetic variation in complement factor H associated with age-related macular degeneration, or (b) the human has an ophthalmic condition or trait selected from the group consisting of Stargardt Disease, recessive retinitis pigmentosa, geographic atrophy (of which scotoma is one non-limiting example), photoreceptor degeneration, dry-form AMD, recessive cone-rod dystrophy, exudative (or wet-form) age-related macular degeneration, cone-rod dystrophy, and retinitis pigmentosa. In further embodiments the mammal is an animal model for retinal degeneration.

In further embodiments, are methods comprising multiple administrations of the effective amount of the agent which increases the clearance rate of RBP or TTR in said mammal, including further embodiments in which (i) the time between multiple administrations is at least one week; (ii) the time between multiple administrations is at least one day; and (iii) the compound is administered to the mammal on a daily basis; or (iv) the compound is administered to the mammal every 12 hours. In further or alternative embodiments, the method comprises a drug holiday, wherein the administration of the compound is temporarily suspended or the dose of the compound being administered is temporarily reduced; at the end of the drug holiday, dosing of the compound is resumed. The length of the drug holiday can vary from 2 days to 1 year.

In further embodiments are methods comprising administering at least one additional agent selected from the group consisting of an inducer of nitric oxide production, an anti-inflammatory agent, a physiologically acceptable antioxidant, a physiologically acceptable mineral, a negatively charged phospholipid, a carotenoid, a statin, an anti-angiogenic drug, a matrix metalloproteinase inhibitor, 13-*cis-*retinoic acid (including derivatives of 13-*cis*-retinoic acid), 11-*cis*-retinoic acid (including derivatives of 11-*cis*-retinoic acid), 9-*cis*-retinoic acid (including derivatives of 9-*cis*-retinoic acid), and retinylamine derivatives. In further embodiments:
(a) the additional agent is an inducer of nitric oxide production, including embodiments in which the inducer of nitric oxide production is selected from the group consisting of citrulline, ornithine, nitrosated *L*-arginine, nitrosylated *L*-arginine, nitrosated *N*-hydroxy-*L*-arginine, nitrosylated *N*-hydroxy-*L*-arginine, nitrosated *L*-homoarginine and nitrosylated *L-*homoarginine;
(b) the additional agent is an anti-inflammatory agent, including embodiments in which the anti-inflammatory agent is selected from the group consisting of a non-steroidal anti-inflammatory drug, a lipoxygenase inhibitor, prednisone, dexamethasone, and a cyclooxygenase inhibitor;
(c) the additional agent is at least one physiologically acceptable antioxidant, including embodiments in which the physiologically acceptable antioxidant is selected from the group consisting of Vitamin C, Vitamin E, beta-carotene, Coenzyme Q, and 4-hydroxy-2,2,6,6-tetramethylpiperadine-*N*-oxyl, or embodiments in which (i) the at least one physiologically acceptable antioxidant is administered with the agent which increases the clearance rate of RBP or TTR in said mammal, or (ii) at least two physiologically acceptable antioxidants are administered with the agent which increases the clearance rate of RBP or TTR in said mammal;
(d) the additional agent is at least one physiologically acceptable mineral, including embodiments in which the physiologically acceptable mineral is selected from the group consisting of a zinc (II) compound, a Cu(II) compound, and a selenium (II) compound, or embodiments further comprising administering to the mammal at least one physiologically acceptable antioxidant;
(e) the additional agent is a negatively charged phospholipid, including embodiments in which the negatively charged phospholipid is phosphatidylglycerol;
(f) the additional agent is a carotenoid, including embodiments in which the carotenoid is selected from the group consisting of lutein and zeaxanthin;
(g) the additional agent is a statin, including embodiments in which the statin is selected from the group consisting of rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium, and dihydrocompactin;
(h) the additional agent is an anti-angiogenic drug, including embodiments in which the the anti-angiogenic drug is Rhufab V2, Tryptophanyl-tRNA synthetase, an Anti-VEGF pegylated aptamer, Squalamine, anecortave acetate, Combretastatin A4 Prodrug, Macugen^{™}, mifepristone, subtenon triamcinolone acetonide, intravitreal crystalline triamcinolone acetonide, AG3340, fluocinolone acetonide, and VEGF-Trap;
(i) the additional agent is a matrix metalloproteinase inhibitor, including embodiments in which the matrix metalloproteinase inhibitor is a tissue inhibitors of metalloproteinases, α₂-macroglobulin, a tetracycline, a hydroxamate, a chelator, a synthetic MMP fragment, a succinyl mercaptopurine, a phosphonamidate, and a hydroxaminic acid;
(j) the additional agent is a complement inhibitor, including by way of example only, antibodies against C1, C2, C3, C4, C5, C6, C7, C8, and C9, such as those disclosed in U.S. Pat. Nos. 5,635,178; 5,843,884; 5,847,082; 5,853,722; and in Rollins et al.; Transplantation, 60:1284-1292 (1995) (the contents of all of which are incorporated herein by reference);
(k) the additional agent is a fish oil, including by way of example only, omega 3 fatty acids;
(l) the additional agent is 13-*cis*-retinoic acid (including derivatives of 13-*cis*-retinoic acid), 11-*cis*-retinoic acid (including derivatives of 11-*cis*-retinoic acid), or 9-*cis*-retinoic acid (including derivatives of 9-*cis*-retinoic acid);
(m) the additional agent is a retinylamine derivative, including an all-*trans*-retinylamine derivative, a 13-*cis*-retinylamine derivative, a 11-*cis*-retinylamine derivative, or a 9-*cis-*retinylamine derivative;
(n) the additional agent is administered (i) prior to the administration of the agent which increases the clearance rate of RBP or TTR in said mammal, (ii) subsequent to the administration of the agent which increases the clearance rate of RBP or TTR in said mammal, (iii) simultaneously with the administration of the agent which increases the clearance rate of RBP or TTR in said mammal, or (iv) both prior and subsequent to the administration of agent which increases the clearance rate of RBP or TTR in said mammal; or
(o) the additional agent and agent which increases the clearance rate of RBP or TTR in said mammal, are administered in the same pharmaceutical composition.

In further embodiments are methods comprising administering extracorporeal rheopheresis to the mammal. In further embodiments are methods comprising administering to the mammal a therapy selected from the group consisting of limited retinal translocation, photodynamic therapy, drusen lasering, macular hole surgery, macular translocation surgery, Phi-Motion, Proton Beam Therapy, Retinal Detachment and Vitreous Surgery, Scleral Buckle, Submacular Surgery, Transpupillary Thermotherapy, Photosystem I therapy, MicroCurrent Stimulation, anti-inflammatory agents, RNA interference, administration of eye medications such as phospholine iodide or echothiophate or carbonic anhydrase inhibitors, microchip implantation, stem cell therapy, gene replacement therapy, ribozyme gene therapy, photoreceptor/retinal cells transplantation, and acupuncture.

In further embodiments are methods comprising the use of laser photocoagulation to remove drusen from the eye of the mammal.

In further embodiments are methods comprising administering to the mammal at least once an effective amount of a second agent which increases the clearance rate of RBP or TTR in said mammal, wherein the first compound is different from the second compound.

In further embodiments, an apparatus capable of detecting and/or quantitating retinol-RBP-TTR complex formation is provided, wherein at least a portion of the TTR is fluorescently labeled.

In one embodiment, the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. In another embodiment, the polyhalogenated aromatic hydrocarbon is a hydroxylated polyhalogenated aromatic hydrocarbon metabolite. The hydroxylated polyhalogenated aromatic hydrocarbon metabolite may be a hydroxylated polychlorinated biphenyl metabolite.

The methods and compositions disclosed herein also provide for modulating RBP or TTR levels or activity in a mammal comprising administering to the mammal at least once an effective amount of an RBP or TTR transcription inhibitor, wherein said modulation of RBP or TTR levels or activity reduces the formation of N-retinylidene-N-retinylethanolamine in an eye of a mammal. In some embodiments, the agent may be chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In yet another embodiment, the methods and compositions disclosed herein provide for modulating RBP or TTR levels or activity in a mammal comprising administering to the mammal at least once an effective amount of an RBP or TTR translation inhibitor, wherein said modulation of RBP or TTR levels or activity reduces the formation of N-retinylidene-N-retinylethanolamine in an eye of a mammal. In some embodiments, the agent may be chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In one embodiment, the methods and compositions disclosed herein provide for modulating RBP or TTR levels or activity in a mammal comprising administering to the mammal at least once an effective amount of an agent which modulates RBP binding to TTR in said mammal, wherein said modulation of RBP or TTR levels or activity reduces the formation of N-retinylidene-N-retinylethanolamine in an eye of a mammal. The modulating agent can bind to RBP or TTR so as to inhibit the binding of RBP to TTR in the mammal. The modulating agent can also antagonize the binding of retinol to RBP so as to inhibit the binding of RBP or the RBP-agent complex to TTR. The agent may be chosen from the group consisting of a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In yet another embodiment, the methods and compositions disclosed herein provide for modulating RBP or TTR levels or activity in a mammal comprising administering to the mammal at least once an effective amount of an agent which increases the clearance rate of RBP or TTR in said mammal, wherein said modulation of RBP or TTR levels or activity reduces the formation of N-retinylidene-N-retinylethanolamine in an eye of a mammal. In some embodiments, the agent may be chosen from the group consisting of a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the methods and compositions disclosed herein provide for modulating RBP or TTR levels or activity in a mammal comprising administering to the mammal at least once an effective amount of an RBP or TTR transcription inhibitor, wherein said modulation of RBP or TTR levels or activity reduces the formation of lipofuscin in an eye of a mammal. The agent may be chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In yet another embodiment, the methods and compositions disclosed herein provide for modulating RBP or TTR levels or activity in a mammal comprising administering to the mammal at least once an effective amount of an RBP or TTR translation inhibitor, wherein said modulation of RBP or TTR levels or activity reduces the formation of lipofuscin in an eye of a mammal. In some embodiments, the agent may be chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In other embodiments, the methods and compositions disclosed herein provide for modulating RBP or TTR levels or activity in a mammal comprising administering to the mammal at least once an effective amount of an agent which modulates RBP binding to TTR in said mammal, wherein said modulation of RBP or TTR levels or activity reduces the formation of lipofuscin in an eye of a mammal. The modulating agent can bind to RBP or TTR so as to inhibit the binding of RBP to TTR in the mammal. The modulating agent can also antagonize the binding of retinol to RBP so as to inhibit the binding of RBP or the RBP-agent complex to TTR. In one embodiment, the agent may be chosen from the group consisting of a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In yet another embodiment, the methods and compositions disclosed herein provide for modulating RBP or TTR levels or activity in a mammal comprising administering to the mammal at least once an effective amount of an agent which increases the clearance rate of RBP or TTR in said mammal, wherein said modulation of RBP or TTR levels or activity reduces the formation of lipofuscin in an eye of a mammal. The agent may be chosen from the group consisting of a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the methods and compositions disclosed herein provide for modulating RBP or TTR levels or activity in a mammal comprising administering to the mammal at least once an effective amount of an RBP or TTR transcription inhibitor, wherein said modulation of RBP or TTR levels or activity reduces the formation of drusen in an eye of a mammal. In some embodiments, the agent may be chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In yet another embodiment, the methods and compositions disclosed herein for modulating RBP or TTR levels or activity in a mammal comprising administering to the mammal at least once an effective amount of an RBP or TTR translation inhibitor, wherein said modulation of RBP or TTR levels or activity reduces the formation of drusen in an eye of a mammal. In some embodiments, the agent may be chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In one embodiment, the methods and compositions disclosed herein provide for modulating RBP or TTR levels or activity in a mammal comprising administering to the mammal at least once an effective amount of an agent which modulates RBP binding to TTR in said mammal, wherein said modulation of RBP or TTR levels or activity reduces the formation of drusen in an eye of a mammal. The modulating agent can bind to RBP or TTR so as to inhibit the binding of RBP to TTR in the mammal. The modulating agent can also antagonize the binding of retinol to RBP so as to inhibit the binding of RBP or the RBP-agent complex to TTR. The agent may be chosen from the group consisting of a retinyl derivative, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In another embodiment, the methods and compositions disclosed herein provide for modulating RBP or TTR levels or activity in a mammal comprising administering to the mammal at least once an effective amount of an agent which increases the clearance rate of RBP or TTR in said mammal, wherein said modulation of RBP or TTR levels or activity reduces the formation of drusen in an eye of a mammal. In some embodiments, the agent may be chosen from the group consisting of a retinyl derivative, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the methods and compositions disclosed herein provide for modulating RBP or TTR levels or activity in a mammal comprising administering to the mammal at least once an effective amount of an RBP or TTR transcription inhibitor, wherein said modulation of RBP or TTR levels or activity prevents age-related macular degeneration or dystrophy in an eye of a mammal. The agent in this embodiment may be chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In another embodiment, the methods and compositions disclosed herein provide for modulating RBP or TTR levels or activity in a mammal comprising administering to the mammal at least once an effective amount of an RBP or TTR translation inhibitor, wherein said modulation of RBP or TTR levels or activity prevents age-related macular degeneration or dystrophy in an eye of a mammal. In one embodiment, the agent is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In yet another embodiment, the methods and compositions disclosed herein provide for modulating RBP or TTR levels or activity in a mammal comprising administering to the mammal at least once an effective amount of an agent which modulates RBP binding to TTR in said mammal, wherein said modulation of RBP or TTR levels or activity prevents age-related macular degeneration or dystrophy in an eye of a mammal. The modulating agent can bind to RBP or TTR so as to inhibit the binding of RBP to TTR in the mammal. The modulating agent can also antagonize the binding of retinol to RBP so as to inhibit the binding of RBP or the RBP-agent complex to TTR. The agent may be chosen from the group consisting of a retinyl derivative, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

The methods and compositions disclosed herein also provide for modulating RBP or TTR levels or activity in a mammal comprising administering to the mammal at least once an effective amount of an agent which increases the clearance rate of RBP or TTR in said mammal, wherein said modulation of RBP or TTR levels or activity prevents age-related macular degeneration or dystrophy in an eye of a mammal. In this embodiment, the agent may be chosen from the group consisting of a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the methods and compositions disclosed herein provide for modulating RBP or TTR levels or activity in a mammal comprising administering to the mammal at least once an effective amount of an RBP or TTR transcription inhibitor, wherein said modulation of RBP or TTR levels or activity protects an eye of a mammal from light. In another embodiment, the agent is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In another embodiment, the methods and compositions disclosed herein provide for modulating RBP or TTR levels or activity in a mammal comprising administering to the mammal at least once an effective amount of an RBP or TTR translation inhibitor, wherein said modulation of RBP or TTR levels or activity protects an eye of a mammal from light. In some embodiments, the agent may be chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In yet another embodiment, the methods and compositions disclosed herein provide for modulating RBP or TTR levels or activity in a mammal comprising administering to the mammal at least once an effective amount of an agent which modulates RBP binding to TTR in said mammal, wherein said modulation of RBP or TTR levels or activity protects an eye of a mammal from light. The modulating agent can bind to RBP or TTR so as to inhibit the binding of RBP to TTR in the mammal. The modulating agent can also antagonize the binding of retinol to RBP so as to inhibit the binding of RBP or the RBP-agent complex to TTR. In this embodiment, the agent may be chosen from the group consisting of a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In another embodiment, the methods and compositions disclosed herein provide for modulating RBP or TTR levels or activity in a mammal comprising administering to the mammal at least once an effective amount of an agent which increases the clearance rate of RBP or TTR in said mammal, wherein said modulation of RBP or TTR levels or activity protects an eye of a mammal from light. In some embodiments, the agent is chosen from the group consisting of a retinyl derivative, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, the methods and compositions disclosed herein provide for modulating retinol binding protein (RBP) or transthyretin (TTR) levels or activity in a mammal comprising administering to the mammal at least once an effective amount of at least one of the compounds chosen from the group consisting of an RBP transcription inhibitor, a TTR transcription inhibitor, an RBP translation inhibitor, a TTR translation inhibitor, an RBP clearance agent, a TTR clearance agent, an RBP antagonist, an RBP agonist, a TTR antagonist and a TTR agonist.

In some embodiments, the RBP transcription inhibitor is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, HNF-4 agonists, HNF-4 antagonists, aptamers, Zn-finger binding proteins, ribozymes and monoclonal antibodies. In other embodiments, the TTR transcription inhibitor is chosen from the group consisting of fatty acid binding protein antagonists, C/EBP agonists, C/EBP antagonists, antisense oligonucleotides, siRNA, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In yet other embodiments, the RBP translation inhibitor is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, HNF-4 agonists, HNF-4 antagonists, aptamers, ribozymes and monoclonal antibodies. In other embodiments, the TTR translation inhibitor is chosen from the group consisting of fatty acid binding protein antagonists, C/EBP agonists, C/EBP antagonists, antisense oligonucleotides, siRNA, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In another embodiment, the RBP clearance agent is chosen from the group consisting of: a retinyl derivative, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody. In yet another embodiment, the TTR clearance agent is chosen from the group consisting of: a thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In another embodiment, the RBP agonist or antagonist is a retinyl derivative. In yet another embodiment, the TTR agonist or antagonist is chosen from the group consisting of a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

The methods and compositions disclosed herein also provide for the treatment of age-related macular degeneration or dystrophy, comprising administering to a mammal at least once an effective amount of a first compound, wherein said first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

The methods and compositions disclosed herein also provide for the reduction of formation of all-trans retinal in an eye of a mammal comprising administering to the mammal at least once an effective amount of a first compound, wherein the first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

In one embodiment, the methods and compositions disclosed herein provide for reducing the formation of N-retinylidene-N-retinylethanolamine in an eye of a mammal comprising administering to the mammal at least once an effective amount of a first compound, wherein said first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

In yet another embodiment, the methods and compositions disclosed herein provide for reducing the formation of lipofuscin in an eye of a mammal comprising administering to the mammal at least once an effective amount of a first compound, wherein said first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

In another embodiment, the methods and compositions disclosed herein provide for reducing the formation of drusen in an eye of a mammal comprising administering to the mammal at least once an effective amount of a first compound, wherein said first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

In one embodiment, the methods and compositions disclosed herein provide for protecting an eye of a mammal from light comprising administering to the mammal at least once an effective amount of a first compound, wherein said first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

In another embodiment, the methods and compositions disclosed herein provide for the treatment of retinol-related diseases, comprising administering to the mammal at least once an effective amount of at least one of the compounds chosen from the group consisting of: an RBP transcription inhibitor, a TTR transcription inhibitor, an RBP translation inhibitor, a TTR translation inhibitor, an RBP clearance agent, a TTR clearance agent, an RBP antagonist, an RBP agonist, a TTR antagonist, a TTR agonist and a retinol binding receptor antagonist. In one embodiment, the RBP transcription inhibitor is chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.

In another embodiment, the TTR transcription inhibitor is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, HNF-4 agonists, HNF-4 antagonists, aptamers, Zn-finger binding proteins, ribozymes and monoclonal antibodies. In yet another embodiment, the RBP translation inhibitor is chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, HNF-4 agonists, HNF-4 antagonists, aptamers, ribozymes and monoclonal antibodies. In still another embodiment, the TTR translation inhibitor is chosen from the group consisting of: antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.

In one embodiment, the RBP clearance agent is chosen from the group consisting of: a retinyl derivative, a polyhalogenated aromatic hydrocarbon, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody. Alternatively, the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. In another embodiment, the polyhalogenated aromatic hydrocarbon may be a hydroxylated polyhalogenated aromatic hydrocarbon metabolite, specifically, a hydroxylated polychlorinated biphenyl metabolite.

In yet another embodiment, the TTR clearance agent is chosen from the group consisting of: a thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, a polyhalogenated aromatic hydrocarbon and an antibody.

In yet another embodiment, the RBP agonist or antagonist may be a retinyl derivative such as N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. In yet another embodiment, the TTR agonist or antagonist is chosen from the group consisting of: a polyhalogenated aromatic hydrocarbon, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody. In one embodiment, the small molecule compound may be resveratrol or biarylamine. In still another embodiment, the retinol binding protein receptor antagonist may be an inhibitor of retinyl palmitate hydrolase, more specifically the retinyl palmitate hydrolase inhibitor may be 3,4,3',4'-tetrachlorobiphenyl.

In yet another embodiment, the methods and compositions disclosed herein provide for administration of a second compound selected from the group consisting of an inducer of nitric oxide production, an antioxidant, an anti-inflammatory agent, a mineral, an anti-oxidant, a carotenoid, a negatively charged phospholipid and a statin. In some embodiments, the retinol-related disease may be diabetes, hyperostosis, idiopathic intracranial hypertension, amyloidosis, Alzheimer's disease, and Alström-Hallgren syndrome.

The methods and compositions disclosed herein also provide for treating type I or type II diabetes in a mammal, comprising administering to the mammal at least once an effective amount of a first compound, wherein said first compound modulates RBP or TTR levels or activity in the mammal. In one embodiment, the first compound may modulate transcription of RBP or TTR in the mammal. In another embodiment, the first compound may modulate translation of RBP or TTR in the mammal. In yet another embodiment, the first compound may modulate RBP or TTR clearance in the mammal. In still another embodiment, the first compound may modulate RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR.

In one embodiment, the methods and compositions disclosed herein further comprise administration of a second compound selected from the group consisting of (a) a glucose-lowering hormone or hormone mimetic (e.g., insulin, GLP-1 or a GLP-1 analog, exendin-4 or liraglutide), (b) a glucose-lowering sulfonylurea (e.g., acetohexamide, chlorpropamide, tolbutamide, tolazamide, glimepiride, a glipizide, glyburide, a micronized gylburide, or a gliclazide), (c) a glucose-lowering biguanide (metformin), (d) a glucose-lowering meglitinide (e.g., nateglinide or repaglinide), (e) a glucose-lowering thiazolidinedione or other PPAR-gamma agonist (e.g., pioglitazone, rosiglitazone, troglitazone, or isagitazone), (f) a glucose-lowering dual-acting PPAR agonist with affinity for both PPAR-gamma and PPAR-alpha (e.g., BMS-298585 and tesaglitazar), (g) a glucose-lowering alpha-glucosidase inhibitor (e.g., acarbose or miglitol), (h) a glucose-lowerinng antisense compound not targeted to glucose-6-phosphatase translocase, (i) an anti-obesity appetite suppressant (e.g. phentermine), (j) an anti-obesity fat absorption inhibitor such as orlistat, (k) an anti-obesity modified form of ciliary neurotrophic factor which inhibits hunger signals that stimulate appetite, (l) a lipid-lowering bile salt sequestering resin (e.g., cholestyramine, colestipol, and colesevelam hydrochloride), (m) a lipid-lowering HMGCoA-reductase inhibitor (e.g., lovastatin, cerivastatin, prevastatin, atorvastatin, simvastatin, and fluvastatin), (n) a nicotinic acid, (o) a lipid-lowering fibric acid derivative (e.g., clofibrate, gemfibrozil, fenofibrate, bezafibrate, and ciprofibrate), (p) agents including probucol, neomycin, dextrothyroxine, (q) plant-stanol esters, (r) cholesterol absorption inhibitors (e.g., ezetimibe), (s) CETP inhibitors (e.g. torcetrapib and JTT-705), (t) MTP inhibitors (eg, implitapide), (u) inhibitors of bile acid transporters (apical sodium-dependent bile acid transporters), (v) regulators of hepatic CYP7a, (w) ACAT inhibitors (e.g. Avasimibe), (x) lipid-lowering estrogen replacement therapeutics (e.g., tamoxigen), (y) synthetic HDL (e.g. ETC-216), or (z) lipid-lowering anti-inflammatories (e.g., glucocorticoids). When the second compound has a different target and/or acts by a different mode of action from the agents described herein (i.e., those that modulate RBP or TTR levels or activity), the administration of the two agents in combination (e.g., simultaneous, sequential or separate administration) is expected to provide additive or synergistic therapeutic benefit to a patient with diabetes. For the same reason, the administration of the two agents in combination (e.g., simultaneous, sequential or separate administration) is expected to allow lower doses of each or either agent relative to the dose of such agent in the absence of the combination therapy while still achieving a desired therapeutic benefit, including by way of example only, reduction in blood glucose and HbA1c control.

In one embodiment, the first compound is administered to a mammal with type II diabetes. In still another embodiment, the first compound increases RBP or TTR clearance in the mammal. In another embodiment, the first compound inhibits RBP binding to TTR. In some embodiments, the first compound may be a retinyl derivative, wherein the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide.

The methods and compositions disclosed herein also provide for treating idiopathic intracranial hypertension in a mammal comprising administering to the mammal at least once an effective amount of a first compound, wherein said first compound modulates RBP or TTR levels or activity in the mammal. In one embodiment, the first compound decreases transcription of RBP or TTR in the mammal. In another embodiment, the first compound decreases translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR. In some embodiments, the first compound may be a retinyl derivative, wherein the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. The methods and compositions disclosed herein may also further comprise administration of a second compound selected from the group consisting of an inducer of nitric oxide production, an antioxidant, an anti-inflammatory agent, a mineral, an anti-oxidant, a carotenoid, a negatively charged phospholipid and a statin.

The methods and compositions disclosed herein also provide for treating hyperostosis in a mammal comprising administering to the mammal at least once an effective amount of a first compound, wherein said first compound modulates RBP or TTR levels or activity in the mammal. In one embodiment, the first compound inhibits transcription of RBP or TTR in the mammal. In another embodiment, the first compound inhibits translation of RBP or TTR in the mammal. In yet another embodiment, the first compound increases RBP or TTR clearance in the mammal. In still another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR. In some embodiments, the first compound may be a retinyl derivative, wherein the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. The methods and compositions disclosed herein may also further comprise administration of a second compound selected from the group consisting of an inducer of nitric oxide production, an antioxidant, an anti-inflammatory agent, a mineral, an anti-oxidant, a carotenoid, a negatively charged phospholipid and a statin.

The methods and compositions disclosed herein also provide for treating amyloidosis in a mammal comprising administering to the mammal at least once an effective amount of a first compound, wherein said first compound modulates RBP or TTR levels or activity in the mammal. In one embodiment, the first compound inhibits transcription or translation of TTR in the mammal. In another embodiment, the first compound increases TTR clearance in the mammal. In yet another embodiment, the first compound inhibits RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR. In some embodiments, the first compound may be a retinyl derivative, wherein the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. The methods and compositions disclosed herein may also further comprise administration of a second compound selected from the group consisting of an inducer of nitric oxide production, an antioxidant, an anti-inflammatory agent, a mineral, an anti-oxidant, a carotenoid, a negatively charged phospholipid and a statin.

The methods and compositions disclosed herein also provide for treating Alzheimer's disease in a mammal comprising administering to the mammal at least once an effective amount of a first compound, wherein said first compound modulates RBP or TTR levels or activity in the mammal. In one embodiment, the first compound increases transcription of RBP or TTR in the mammal. In another embodiment, the first compound increases translation of RBP or TTR in the mammal. In yet another embodiment, the first compound decreases RBP or TTR clearance in the mammal. In still another embodiment, the first compound promotes RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR. In some embodiments, the first compound may be a retinyl derivative, wherein the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. The methods and compositions disclosed herein may also further comprise administration of a second compound selected from the group consisting of an inducer of nitric oxide production, an antioxidant, an anti-inflammatory agent, a mineral, an anti-oxidant, a carotenoid, a negatively charged phospholipid and a statin.

The methods and compositions disclosed herein also provide for treating Alstrom-Hallgren's syndrome in a mammal, comprising administering to the mammal at least once an effective amount of a first compound, wherein said first compound modulates RBP or TTR levels or activity in the mammal. In one embodiment, the first compound modulates transcription of RBP or TTR in the mammal. In another embodiment, the first compound modulates translation of RBP or TTR in the mammal. In yet another embodiment, the first compound modulates RBP or TTR clearance in the mammal. In still another embodiment, the first compound modulates RBP binding to TTR. Such an agent can bind to RBP or TTR so as to inhibit the binding of RBP to TTR in the mammal. Further, such an agent can also antagonize the binding of retinol to RBP so as to inhibit the binding of RBP or the RBP-agent complex to TTR. In some embodiments, the first compound may be a retinyl derivative, wherein the retinyl derivative is N-(4-hydroxyphenyl)retinamide (also referred to herein as "HPR" or "fenretinide" or "4-hydroxyphenylretinamide" or "hydroxyphenyl retinamide"), N-(4-methoxyphenyl)retinamide ("MPR"; the most prevalent metabolite of HPR), or ethylretinamide. The methods and compositions disclosed herein may also further comprise administration of a second compound selected from the group consisting of an inducer of nitric oxide production, an antioxidant, an anti-inflammatory agent, a mineral, an anti-oxidant, a carotenoid, a negatively charged phospholipid and a statin.

In yet other embodiments, an effective amount of a compound of the methods and compositions disclosed herein may be systemically administered to the mammal. In some embodiments, the compound may be administered orally to the mammal. In other embodiments, the compound may be intravenously administered to the mammal. In yet other embodiments, the compound may be ophthalmically administered to the mammal. In another embodiment, the compound may be administered by injection to the mammal.

In any of the aforementioned embodiments, the mammal of the methods and compositions disclosed herein is a human. In yet other embodiments, the methods and compositions disclosed herein may comprise multiple administrations of the effective amount of the compound. In some embodiments, the time between multiple administrations is at least one week. In other embodiments, the time between multiple administrations is at least one day. In yet another embodiment, the compound is administered to the mammal on a daily basis.

The methods and compositions disclosed herein may also further comprise administering an inducer of nitric oxide production to the mammal. In yet other embodiments, the methods and compositions disclosed herein may further comprise administering an anti-inflammatory agent to the mammal. In one embodiment, the methods and compositions disclosed herein may further comprise administering to the mammal at least one antioxidant. The antioxidant of the methods and compositions disclosed herein may be selected from the group consisting of Vitamin C, Vitamin E, beta-carotene, Coenzyme Q, and 4-hydroxy-2,2,6,6-tetramethylpiperadine-N-oxyl.

In another embodiment, the methods and compositions disclosed herein may further comprise the administration of at least one antioxidant with the compounds disclosed herein. In yet another embodiment, the methods and compositions disclosed herein may further comprise administering to the mammal at least one mineral. In this embodiment, the mineral may be selected from the group consisting of a zinc (II) compound, a Cu(II) compound, and a selenium (II) compound. In another embodiment, the minerals of the methods and compositions disclosed herein may be further administered with at least one antioxidant.

In yet another embodiment, the methods and compositions disclosed herein may further comprise administering to the mammal a carotenoid. The carotenoid in this embodiment may be selected from the group consisting of lutein and zeaxanthin. In one embodiment, the methods and compositions disclosed herein may further comprise administering to the mammal a negatively charged phospholipid. In this embodiment, the negatively charged phospholipid may be phosphatidyl glycerol. In another embodiment, the methods and compositions disclosed herein may further comprise administering to the mammal a statin. The statin in the methods and compositions disclosed herein may be chosen from the group consisting of rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium, and dihydrocompactin

In other embodiments, the compound of the methods and compositions disclosed herein may be administered to the mammal every 12 hours. In some embodiments, the methods and compositions disclosed herein further comprise administering rheophoresis to the mammal. In another embodiment, the methods and compositions disclosed herein further comprise monitoring formation of drusen in the eye of the mammal. In yet another embodiment, the methods and compositions disclosed herein further comprise measuring levels of lipofuscin in the eye of the mammal. In still another embodiment, the methods and compositions disclosed herein further comprise measuring visual acuity in the eye of the mammal. In one embodiment, the methods and compositions disclosed herein further comprise measuring the auto-fluorescence of A2E and precursors of A2E.

In some embodiments, the macular degeneration is Stargardt Disease. In other embodiments, the macular degeneration is dry form age-related macular degeneration. In one embodiment, the human is a carrier of the gene for Stargardt Disease. The method and compositions disclosed may also further comprise determining whether the mammal is a carrier of the gene for Stargardt Disease.

In some embodiments, a pharmaceutical composition for the treatment of macular degeneration may comprising the compounds of the methods and compositions disclosed herein and a pharmaceutically acceptable carrier. In one embodiment, the pharmaceutically acceptable carrier is suitable for ophthalmic administration.

Other objects, features and advantages of the methods and compositions described herein will become apparent from the following detailed description. It should be understood, however, that the detailed description and the specific examples, while indicating specific embodiments, are given by way of illustration only, since various changes and modifications within the spirit and scope of the invention will become apparent to those skilled in the art from this detailed description.

All publications and patent applications mentioned in this specification are herein incorporated by reference to the same extent as if each individual publication or patent application was specifically and individually indicated to be incorporated by reference.

### BRIEF DESCRIPTION OF THE DRAWINGS

The novel features of the methods and compositions disclosed herein are set forth with particularity in the appended claims. A better understanding of the features and advantages will be obtained by reference to the following detailed description that sets forth illustrative embodiments, in which the principles disclosed herein are utilized, and the accompanying drawings of which:

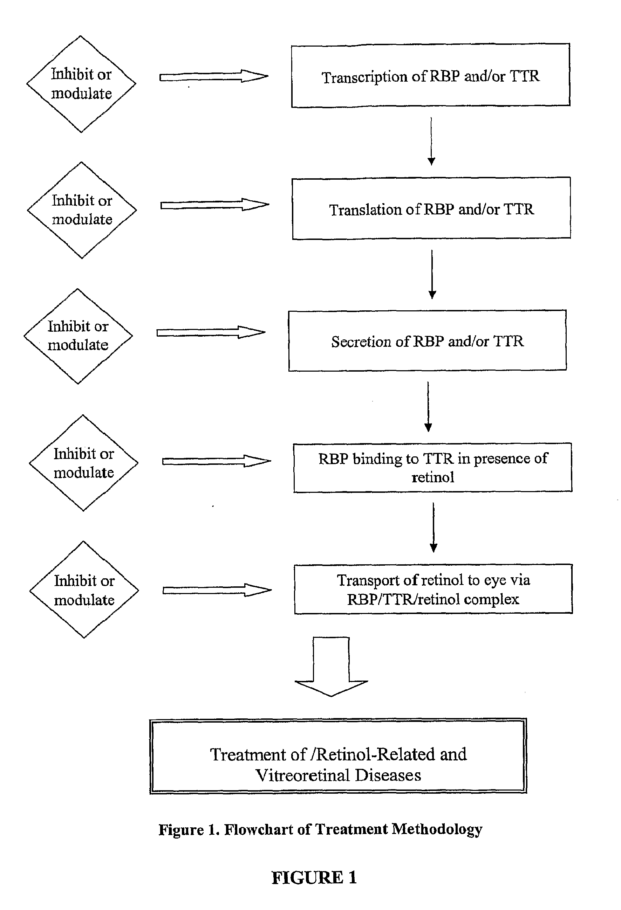

FIG. 1 illustrates a flowchart for the treatment of retinol-related and/or vitreoretinal diseases using the methods and compositions described herein.

FIG. 2 illustrates the relationship of serum HPR levels to serum retinol levels and ocular levels of retinoids and A2E.

FIG. 3 illustrates the effect of administering HPR to wild type mice on (A) serum retinol levels and (B) ocular retinoid levels.

FIG. 4 illustrates an example of a FRET spectrum taken of an RBP-TTR complex in the absence and presence of HPR, wherein the TTR has been labeled with a fluorescence moiety.

FIG. 5 illustrates an example of dose dependent inhibition of retinol-RBP-TTR complex formation by HPR as determined using the FRET methods described herein.

FIG. 6 illustrates a comparison of the inhibition of retinol-RBP-TTR complex formation using HPR, 13-cis-retirioic acid and all-trans-retinoic acid as determined using the FRET methods described herein.

FIGS. 7a-7c illustrate various reverse phase LC analyses of acetonitrile extracts of serum. The serum was obtained from mice administered with either DMSO (FIG. 7a), 10 mg/kg N-4-(hydroxyphenyl)retinamide (HPR) (FIG 7b), or 20 mg/kg HPR (FIG. 7c) for 14 days.

FIG. 8 illustrates the analysis of serum retinol as a function of fenretinide concentration.

FIG. 9a illustrates a control binding assay for the interaction between retinol and retinol-binding protein as measured by fluorescence quenching.

FIG. 9b illustrates a binding assay for the interaction between retinol and retinol-binding protein in the presence of HPR (2 µM) as measured by fluorescence quenching.

FIG. 10a illustrates the effect of HPR on A2PE-H₂ biosynthesis in *abca4* null mutant mice.

FIG. 10b illustrates the effect of HPR on A2E biosynthesis in *abca4* null mutant mice.

FIG. 11 illustrates the binding of *N*-4-(methoxyphenyl)retinamide (MPR) to retinol binding protein (RBP) as measured by fluorescence quenching.

FIG. 12 illustrates the modulation of TTR binding to RBP-MPR as measured by size exclusion chromatography and UV/Visible spectrophotometry.

FIG. 13 illustrates the analysis of A2PE-H₂ and A2E levels as a function of fenretinide dose and treatment period (panels A-F) and lipofuscin autofluorescence in the RPE of *ABCA4* null mutant mice as a function of fenretinide treatment (panels G-I).

FIG. 14 illustrates a correlation plot relating fenretinide concentration to reductions in retinol, A2PE-H₂ and A2E in *ABCA4* null mutant mice.

FIG. 15 illustrates retinoid composition in light adapted DMSO- and HPR-treated mice (panel A); the affect of HPR on the regeneration of visual chromophore (panel B); the effect of HPR on bleached chromophore recycling (panel C); and electrophysiological measurements of rod function (panel D), rod and cone function (panel E), and recovery from photobleaching (panel F).

FIG. 16 illustrates light microscopy images of the retinas from DMSO- and HPR-treated animals.

FIG. 17 illustrates absorbance and fluorescence chromatograms from eyecup extracts of control mice (panel A), and of mice previously maintained on HPR therapy (panel B) following a 12-day drug holiday; absorbance and fluorescence chromatograms from eyecup extracts of control mice (panel C), and of mice previously maintained on HPR therapy (panel D) following a 28-day drug holiday; the histogram presents the relative A2E levels for the mice described in panels A-D.

FIG. 18 illustrates the relative concentration of A2E, A2PE and A2PE-H₂ in three lines of mice at three months of age.

### DETAILED DESCRIPTION OF THE INVENTION

Reference will now be made in detail to embodiments of the methods and compositions disclosed herein. Examples of the embodiments are illustrated in the following Examples section.

Unless defined otherwise, all technical and scientific terms used herein have the same meaning as is commonly understood by one of skill in the art to which this invention belongs. All patents and publications referred to herein are incorporated by reference.

As used herein, the term "ABCA4 gene" refers to a gene encoding the rim protein or RmP. The ABCA4 gene is also known as the ABCR gene.

As used herein, the term "anti-oxidant" refers to a synthetic or natural substance that can prevent, delay or otherwise inhibit the oxidation of a compound or biological substance.

As used herein, the term "deconvoluting" refers to the process of converting data, information and/or images into (at least in part) constituent components. For example, a fluorescence or absorbance spectrum that features a complex wave form can be mathematically deconvoluted into the separate absorbance or fluorescence peaks that comprise the complex wave form. Suitable mathematical procedures and algorithms are well-known in the art, and suitable software packages for deconvoluting data, information and/or images are commercially available.

As used herein, the term "disruption of the visual cycle" or the like refers to any means for modulating the activity, directly or indirectly, of at least one enzyme involved in the visual cycle.

As used herein, the term "dispersing" refers to suspending a substance in another medium. Dispersing can include steps for homogenizing, fractionating, breaking up, fluidizing or decreasing the size of a substance in order to facilitate the suspending step.

As used herein, a retinyl derivative refers to a compound that can be produced by reacting one of the various *cis* or *trans* retinal isomers with another compound or series of compounds.

As used herein, the term "age-related macular degeneration or dystrophy" or "ARMD" refers to a debilitating disease, which include wet and dry forms of ARMD. The dry form of ARMD, which accounts for about 90 percent of all cases, is also known as atrophic, nonexudative, or drusenoid macular degeneration. With the dry form of ARMED, drusen typically accumulate in the retinal pigment epithelium (RPE) tissue beneath/within the Bruch's membrane. Vision loss can then occur when drusen interfere with the function of photoreceptors in the macula. The dry form of ARMD results in the gradual loss of vision over many years. The dry form of ARMD can lead to the wet form of ARMD. The wet form of ARMD can progress rapidly and cause severe damage to central vision. The macular dystrophies include Stargardt Disease, also known as Stargardt Macular Dystrophy or Fundus Flavimaculatus, which is the most frequently encountered juvenile onset form of macular dystrophy.

As used herein, the term "mammal" refers to all mammals including humans. Mammals include, by way of example only, humans, non-human primates, cows, dogs, cats, goats, sheep pigs, rats, mice and rabbits.

As used herein, the term "biological sample" refers to plasma, blood, urine, feces, tissue, mucus, tears or saliva.

As used herein, the term "effective amount" refers to the total amount of the therapeutic agent in the pharmaceutical formulation or method that is sufficient to show a meaningful subject or patient benefit.

As used herein, the term "modulation" means either an increase or a decrease in the levels or expression of a nucleic acid or polypeptide, or in the binding or other functional characteristics of the nucleic acid or polypeptide.

As used herein, the term "ophthalmic disease or condition" refers to any disease or condition involving the eye or related tissues. Non-limiting examples include diseases or conditions involving degeneration of the retina and/or macula, including the retinal and/or macular dystrophies and the retinal and/or macular degenerations.

As used herein, the term "immobilized" refers to the covalent or non-covalent attachment of a chemical or biological species to a support.

As used herein, the term "primate" refers to the highest order of mammals; includes man, apes and monkeys.

As used herein, the term "vitreoretinal disease" refers to any disease or condition involving the vitreous and retina, including, by way of example only, diabetic retinopathy, macular degeneration, retinopathy of prematurity, and retinitis pigmentosa.

As used herein, the term "retinol-related disease" refers to any disease or condition associated with abnormal levels of vitamin A, retinol and its related transport proteins, including diseases associated with abnormal levels of retinol binding protein and transthyretin, in a patient.

As used herein, the term "risk" refers to the probability that an event will occur.

### The Visual Cycle

The vertebrate retina contains two types of photoreceptor cells. Rods are specialized for vision under low light conditions. Cones are less sensitive, provide vision at high temporal and spatial resolutions, and afford color perception. Under daylight conditions, the rod response is saturated and vision is mediated entirely by cones. Both cell types contain a structure called the outer segment comprising a stack of membranous discs. The reactions of visual transduction take place on the surfaces of these discs. The first step in vision is absorption of a photon by an opsin-pigment molecule, which involves 11-*cis* to all-trans isomerization of the retinal chromophore. Before light sensitivity can be regained, the resulting all-*trans*-retinal must dissociate from the opsin apoprotein and isomerize to 11-*cis-*retinal.

All-*trans*-retinal is a visual cycle retinoid which upon condensation with phosphatidylethanolamine produces the diretinal species *N*-retinylidene-*N*-retinylethanolamine. 11-*cis-*retinal is the photoreactive portion of rhodopsin, which is converted to all-*trans*-retinal when a photon of light in the active absorption band strikes the molecule. This process goes through a sequence of chemical reactions as 11-*cis*-retinal isomerizes to all-*trans*-retinal. During this series of chemical steps, the nerve fiber, which is attached to that particular rod or cone, undergoes a stimulus that is perceived in the brain as a visual signal.

### Visual Cycle for Regeneration of Rhodopsin

Rhodopsin, G protein-coupled receptor, has two physiological pathways: phototransduction and/or recovery from bleaching (return of activated components to the dark state) and the retinoid cycle
(production of 11-*cis*-retinal). Vertebrate phototransduction is initiated by a photochemical reaction whereby 11-*cis*-retinal bound to its opsin moiety (rhodopsin = opsin + 11-*cis*-retinal) undergoes isomerization to all-*trans*-retinal, producing conformation changes in opsin. In vertebrates, restoration of a photosensitive receptor conformation (return to the dark state) requires the formation of 11-*cis*-retinal from all-*trans*-retinal via the retinoid cycle. The entire cycle of isomerization and pigment regeneration in humans occurs on a time scale of minutes for rhodopsin, and significantly faster for cone pigments. Reduction of all *trans*-retinal to all-*trans*-retinol takes place in photoreceptor outer segments whereas all other reactions, including isomerization, occur within retinal pigment epithelial cells (RPE). The all-*trans*-retinylidene Schiff base hydrolyzes and all-*trans*-retinal dissociates from the binding pocket of opsin, yet the molecular steps leading to its release from the opsin-binding pocket remain not fully explained. Removal of all-*trans*-retinal from the disks may be facilitated by an ATP-binding cassette transporter (ABCA4), mutations in which are causative of an array of retinal disorders including Stargardt's Disease, cone-rod dystrophy, retinitis pigmentosa and possibly macular degeneration.

Further, all-*trans*-retinal is reduced to all-*trans*-retinol by NADPH-dependent all-*trans*-retinol dehydrogenase, a membrane-associated enzyme that belongs to large gene family of short-chain alcohol dehydrogenases (SCAD). All-trans-retinol translocates to the RPE via a poorly defined process, perhaps involving components like IRBP and RBP present in the interphotoreceptor matrix (IPM), or passive diffusion driven by trapping retinoids (e.g., insoluble fatty acid retinyl esters) in RPE. Esterification in the RPE involves the transfer of an acyl group from lecithin to retinol and is catalyzed by lecithin:retinol acyltransferase (LRAT). These esters may be substrates for an as yet unknown enzyme termed isomerohydrolase, which would use the energy of retinyl ester hydrolysis to isomerize all-*trans*-retinol to 11-*cis*-retinol and thus, drive the reaction forward. Alternatively, these two reactions may proceed separately, i.e., the ester may be first hydrolyzed by a retinyl ester hydrolase and then isomerized to 11-*cis*-retinol through an intermediate. 11-*cis*-retinol would then be oxidized to 11-*cis*-retinal in a reaction catalyzed by NAD- and NADP-dependent 11-*cis*-retinol dehydrogenases, which are other short chain dehydrogenase family members. Finally 11-*cis*-retinal moves back to the rod photoreceptors, either in IRBP-dependent or -independent fashion, where it joins with opsin to regenerate visual pigment.

Further information regarding the anatomical organization of the vertebrate eye, the visual cycle for regeneration of rhodopsin, and the biogenesis of A2E-oxiranes is provided in U.S. Pat. App. No. 11/150,641, filed June 10, 2005, PCT Patent Application No. US 2005/29455, filed August 17, 2005 and U.S. Provisional Pat. 60/622,213, filed October 25, 2004, the contents of which are incorporated by reference in their entirety.

### Macular or Retinal Degenerations and Dystrophies.

Macular degeneration (also referred to as retinal degeneration) is a disease of the eye that involves deterioration of the macula, the central portion of the retina. Approximately 85% to 90% of the cases of macular degeneration are the "dry" (atrophic or non-neovascular) type. In dry macular degeneration, the deterioration of the retina is associated with the formation of small yellow deposits, known as drusen, under the macula; in addition, the accumulation of lipofuscin in the RPE leads to geographic atrophy. This phenomena leads to a thinning and drying out of the macula. The location and amount of thinning in the retina caused by the drusen directly correlates to the amount of central vision loss. Degeneration of the pigmented layer of the retina and photoreceptors overlying drusen become atrophic and can cause a slow loss of central vision.

In "wet" macular degeneration new blood vessels form (i.e., neovascularization) to improve the blood supply to retinal tissue, specifically beneath the macula, a portion of the retina that is responsible for our sharp central vision. The new vessels are easily damaged and sometimes rupture, causing bleeding and injury to the surrounding tissue. Although wet macular degeneration only occurs in about 10 percent of all macular degeneration cases, it accounts for approximately 90% of macular degeneration-related blindness. Neovascularization can lead to rapid loss of vision and eventual scarring of the retinal tissues and bleeding in the eye. This scar tissue and blood produces a dark, distorted area in the vision, often rendering the eye legally blind. Wet macular degeneration usually starts with distortion in the central field of vision. Straight lines become wavy. Many people with macular degeneration also report having blurred vision and blank spots in their visual field. Growth promoting proteins called vascular endothelial growth factor, or VEGF, have been targeted for triggering this abnormal vessel growth in the eye. This discovery has lead to aggressive research of experimental drugs that inhibit or block VEGF. Studies have shown that anti-VEGF agents can be used to block and prevent abnormal blood vessel growth. Such anti-VEGF agents stop or inhibit VEGF stimulation, so there is less growth of blood vessels. Such anti-VEGF agents may also be successful in anti-angiogenesis or blocking VEGF's ability to induce blood vessel growth beneath the retina, as well as blood vessel leakiness.

Stargardt Disease is a macular dystrophy that manifests as a recessive form of macular degeneration with an onset during childhood. See e.g., Allikmets et al., Science, 277:1805-07 (1997); Lewis et al., Am. J. Hum. Genet., 64:422-34 (1999); Stone et al., Nature Genetics, 20:328-29 (1998); Allikmets, Am. J. Hum. Gen., 67:793-799 (2000); Klevering, et al, Ophthalmology, 111:546-553 (2004). Stargardt Disease is characterized clinically by progressive loss of central vision and progressive atrophy of the RPE overlying the macula. Mutations in the human *ABCA4* gene for Rim Protein (RmP) are responsible for Stargardt Disease. Early in the disease course, patients show delayed dark adaptation but otherwise normal rod function. Histologically, Stargardt Disease is associated with deposition of lipofuscin pigment granules in RPE cells.

Mutations in *ABCA4* have also been implicated in recessive retinitis pigmentosa, see, e.g., Cremers et al., Hum. Mol. Genet., 7:355-62 (1998), recessive cone-rod dystrophy, see id., and nonexudative age-related macular degeneration, see e.g., Allikmets et al., Science, 277:1805-07 (1997); Lewis et al., Am. J. Hum. Genet., 64:422-34 (1999), although the prevalence of *ABCA4* mutations in AMD is still uncertain. See Stone et al., Nature Genetics, 20:328-29 (1998); Allikmets, Am. J. Hum. Gen., 67:793-799 (2000); Klevering, et al, Ophthalmology, 111:546-553 (2004). Similar to Stargardt Disease, these diseases are associated with delayed rod dark-adaptation. See Steinmetz et al., Brit. J. Ophthalm., 77:549-54 (1993). Lipofuscin deposition in RPE cells is also seen prominently in AMD, see Kliffen et al., Microsc. Res. Tech., 36:106-22 (1997) and some cases ofretinitis pigmentosa. See Bergsma et al., Nature, 265:62-67 (1977).

In addition, there are several types of macular degenerations that affect children, teenagers or adults that are commonly known as early onset or juvenile macular degeneration. Many of these types are hereditary and are looked upon as macular dystrophies instead of degeneration. Some examples of macular dystrophies include: Cone-Rod Dystrophy, Corneal Dystrophy, Fuch's Dystrophy, Sorsby's Macular Dystrophy, Best Disease, and Juvenile Retinoschisis, as well as Stargardt Disease.

An eye doctor examining a patient at this stage may note the presence of these drusen, even though most people have no symptoms. When drusen have been noted on examination, monitoring will be needed over time. Many people over the age of 60 will have some drusen.

### Metabolic Disorders

Metabolic disorders, including type I and type II diabetes mellitus, have also been associated with abnormal retinol levels.

### Type I Diabetes (Insulin-Dependent Diabetes Mellitus)

Type I diabetes is a severe form of diabetes. If left untreated, type I diabetes results in ketosis of the patient and rapid degeneration. Approximately 10-20% of diabetic patients are classified as type I, comprising mainly young individuals. Non-obese adults also comprise type I diabetic patients, although at fewer numbers.

Type I diabetes is a catabolic disorder, where circulating levels of insulin are virtually absent and plasma glucagon levels elevated. Type I diabetes is believed to have auto-immune origins, possibly resulting from an infectious or toxic environmental insult to the pancreatic B cells in affected individuals. In support of the auto-immune theory, autoantibodies to insulin and islet cells have been detected in type I diabetes patients, as compared to non-diabetic individuals.

Lower levels of retinol, with observed decreases in retinol binding protein (RBP) levels and increased urinary excretion of RBP, has been correlated with type I diabetes in juveniles. See Basu, TK, et al. Am. J. Clin. Nutr. 50:329-331 (1989); Durbey, SW et al., Diabetes Care 20:84-89 (1997). The lower levels of retinol and RBP are accompanied by a concomitant decrease in zinc metabolism, a factor necessary for the synthesis of RBP in hepatic cells. See Cunningham, JJ, et al. Metabolism 42:1558-1562 (1994). In contrast, tocopherol, or vitamin E levels, are unchanged in type I diabetic patients. See Basu, TK et al (1989).

The lower levels of retinol are observed despite elevated levels of vitamin A in hepatic storage cells. See Tuitoek PJ, et al. Br. J. Nutr. 75: 615-622 (1996). Studies demonstrating the linkage between vitamin A status and insulin secretion show that only insulin treatment can relieve the suppressed levels of vitamin A in type I diabetic subjects. Tuitoek, PJ et al., J. Clin. Biochem. Nutr. 19:165-169 (1996). In contrast, dietary supplementation of vitamin A does not normalize metabolic availability of vitamin A. *Id.*

These studies demonstrate the interconnection between vitamin A and insulin regulation of glucose transport into muscle and adipocyte cells. Further studies have strengthened this interconnection by demonstrating the requirement of vitamin A for normal insulin secretion. See Chertow, BS, et al., J. Clin. Invest. 79:163-169 (1987). Retinol was shown to be necessary for insulin release from vitamin A-deficient perfused islet cells. *Id.* In vivo experiments demonstrated that vitamin-A deficient rats had impaired glucose-induced acute insulin release, which only improved with vitamin A repletion. *Id*. Vitamin A may exert its effects on insulin secretion through activation of transglutaminase activity in islet and insulin-secreting cells, see Driscoll HK, et al., Pancreas 15:69-77 (1997), and is needed for fetal islet development and prevention of glucose intolerance in adults, see Matthews, KA et al., J. Nutr. 134:1958-1963 (2004), further strengthening the role of vitamin A and retinol in insulin release and regulation of blood glucose levels in diabetic patients.

### Type II Diabetes (Non-Insulin Dependent Diabetes Mellitus)

Type II diabetes comprises a heterogeneous group of the milder forms of diabetes. Type II diabetes usually occurs in adults, but occasionally may have its onset in childhood.

Type II diabetics classically exhibit insulin insensitivity in response to elevated plasma glucose levels. Up to 85% of type II diabetics are obese, having an insensitivity to endogenous insulin that is positively correlated with the presence of an abdominal distribution of fat. Causes of insulin insensitivity are linked with post-receptor defect in insulin action. This is associated with over distended cellular storage depots (e.g. distended adipocytes and overnourished liver and muscle cells) and a reduced ability to clear nutrients from the circulation after meals. The subsequent hyperinsulinism can also result in a further downregulation of cellular insulin receptors. Furthermore, glucose transporter proteins (e.g. GLUT4) are also downregulated upon continuous activation, leading to an aggravation of hyperglycemic conditions in patients.

In contrast to type I diabetes, type II diabetic patients exhibit elevated levels of RBP selectively, with normal to increased levels of retinol observed. See Sasaki, H et al., "Am. J. Med. Sci. 310:177-82 (1995); Basualdo CG, et al. J. Am Coll. Nutr. 16:39-45 (1997); Abahausain, MA et al., Eur. J. Clin. Nutr. 53: 630-635 (1999). Retinoic acid (all *trans* RA and 13-*cis* RA) levels were also decreased in patients with type II diabetes. Yamakoshi, Y et al., Biol. Pharm. Bull 25:1268-1271 (2002). Levels of other vitamins, including vitamin E (tocopherol) and carotenoids were unchanged in both diabetic and control groups, as well as levels of zinc, albumin and TTR, which are known to influence vitamin A metabolism. *Id.*

This selective increase in RBP levels in type II diabetics, combined with the selective decrease of RBP in type I diabetics, supports the role of RBP and vitamin A in insulin control of blood glucose levels. The increased RBP levels have been attributed to the increased insulin levels (hyperinsulinemia) in diabetic patients. Basualdo et al. (1997). RBP levels have also been linked to the severity of hyperglycemia in patients. *Id.* Retinoids have previously been shown to increase insulin sensitivity in humans. See Hartmann, D. et al. Eur. J. Clin. Pharmacol. 42:523-8 (1992). The inverse correlation of RBP levels with insulin sensitivity in type I and type II diabetics indicates a therapeutic means of controlling insulin sensitivity in mammalian subjects.

### Idiopathic Intracranial Hypertension (IIH)

IIH, also known as pseudotumor cerebri (PTC), is a condition of high pressure in the fluid around the brain without an identifiable causative agent. The condition exists mostly in women in their childbearing years. The symptoms often start or worsen during a period of weight gain. Typical symptoms include headaches, pulse synchronous tinnitus and visual problems (papilledema), which may lead to severe and permanent visual loss in untreated cases.

Although the aetiology of IIH is unknown, investigators have looked at excess vitamin A levels as a candidate because the symptoms and signs of hypervitaminosis A mimic those of IIH. Studies have shown that serum retinol levels are significantly higher in patients with IIH than in control groups, despite the showing of no significant differences in vitamin A ingestion or retinyl ester concentration in both groups. See Jacobson, DM et al., Neurology, 54:2192-3 (1999).

### Bone-Related Disorders

Hyperostosis is a condition where an excessive growth of bone occurs. This condition may lead to formation of a mass projecting from a normal bone, seen in numerous musculoskeletal disorders. Diffuse idiopathic skeletal hyperostosis (DISH) is a form of hyperostosis, characterized by flowing calcification and ossification of vertebral bodies. Radiographic abnormalities in DISH patients are observed most commonly in the thoracic spine, leading to the presence of a radiodense shield in front of the vertebral column. Ossification of the posterior longitudinal ligament (OPLL) is also associated with increased frequency in patients with DISH, in addition to cervical cord compromise as a result of hyperostosis or ossification of spinal ligaments. Other disorders accompanying hyperostosis or DISH patients includes acute fracture and pseudarthrosis of the spine.

Although the pathogenesis of DISH and OPLL are presently unknown, both disorders have been associated with high levels of serum retinol and RBP. See Kodama, T et al., In vivo 12:339-344 (1998); Kilcoyne, RF, J. Am. Acad. Dermatol. 19:212-216 (1988), suggesting a possible role for vitamin A in the pathogenesis of DISH and OPLL. Other studies have shown the occurrence of congenital functional RBP deficiency with abnormal levels of retinol and RBP levels in a hyperostosis patient. De Bandt, M., et al., J. Rheumatol. 22:1395-8 (1995). Medical accounts also report the occurrence of hypervitaminosis A with degenerative joint disease in an elderly patient. See Romero, JB et al., Bull Hosp. Jt. Dis. 54:169-174 (1996).

### Protein Misfolding and Aggregation Diseases

Protein misfolding and aggregation has been linked to several diseases, generally known as the amyloidoses, including Alzeheimer's disease, Parkinson's disease and systemic amyloidosis. These diseases occur with misfolding of the secondary protein structure, in which a normally soluble protein forms insoluble extracellular fibril deposits of β-sheet-rich structures referred to as amyloid fibrils, which causes organ dysfunction. Twenty different fibril proteins, including transthyretin (TTR), have been described in human amyloidosis, each with a different clinical picture.

Wild-type TTR proteins are involved in the development of senile systemic amyloidosis, a sporadic disorder resulting from the deposition of TTR fibrils in cardiac tissues. Mutant TTR proteins, in contrast, are associated with familial amyloidotic polyneuropathy and cardiomyopathy, which deposits primarily affect the peripheral and autonomic nervous system, and heart. The mechanisms responsible for tissue selectivity deposition are currently unknown. In amyloidosis formation, TTR associates with fibril formation in its monomer form. Compounds which promote stabilization of TTR tetramers, such as the small molecules resveratrol and biarylamine, inhibit amyloid fibril formation in vitro. See Reixach, N. et al., PNAS 101:2817-2822 (2004).

Transthyretin is also implicated in Alzheimer's disease, but in contrast to the formation of amyloid fibrils in amyloidosis, TTR inhibits amyloid beta protein formation both in vitro and in vivo. See Schwartzman, AL et al., Amyloid. 11:1-9 (2004); Stein, TD and Johnson, JA, J. Neurosci. 22:7380-7388 (2002). Vitamin A also has been shown to exhibit anti-amyloidogenic and amyloid-beta fibril destabilizing effects in vitro. See Ono, K., et al., Exp. Neurol. 189:380-392 (2004).

### Alström-Hallgren Syndrome

Alström-Hallgren syndrome (also known as Alström syndrome) is a rare autosomal recessive disorder affecting children at a very early age. Symptoms include blindness or severe vision impairment in infancy associated with cone-rod dystrophy, deafness, obesity onset during the first year, development of type II diabetes mellitus and severe insulin resistance, acanthosis nigricans (development of dark patches of skin) hypergonadotrophic hypogonadism and thyroid deficiencies.

Mutations linked to Alström syndrome were localized to a 14.9 cM region on chromosome 2p. Collin, GB et al., Hum. Mol. Gen. 6:213-219 (1997). Other than treating individual symptomatic manifestations of the disease, there are currently no therapeutic treatments available for Alström syndrome patients.

### Modulation of Vitamin A levels

Vitamin A (all-trans retinol) is a vital cellular nutrient which cannot be synthesized *de novo* and therefore must be obtained from dietary sources. Vitamin A is a generic term which may designate any compound possessing the biological activity, including binding activity, of retinol. One retinol equivalent (RE) is the specific biologic activity of 1 µg of all-trans retinol (3.33 IU) or 6 µg (10 IU) of beta-carotene. Beta-carotene, retinol and retinal (vitamin A aldehyde) all possess effective and reliable vitamin A activity. Each of these compounds are derived from the plant precursor molecule, carotene (a member of a family of molecules known as carotenoids). Beta-carotene, which consists of two molecules of retinal linked at their aldehyde ends, is also referred to as the provitamin form of vitamin A.

Ingested β-carotene is cleaved in the lumen of the intestine by β -carotene dioxygenase to yield retinal. Retinal is reduced to retinol by retinaldehyde reductase, an NADPH requiring enzyme within the intestines, and thereafter esterified to palmitic acid.

Following digestion, retinol in food material is transported to the liver bound to lipid aggregates. See Bellovino et al., Mol. Aspects Med., 24:411-20 (2003). Once in the liver, retinol forms a complex with retinol binding protein (RBP) and is then secreted into the blood circulation. Before the retinol-RBP holoprotein can be delivered to extra-hepatic target tissues, such as by way of example, the eye, it must bind with transthyretin (TTR). Zanotti and Berni, Vitam. Horm., 69:271-95 (2004). It is this secondary complex which allows retinol to remain in the circulation for prolonged periods. Association with TTR facilitates RBP release from hepatocytes, and prevents renal filtration of the RBP-retinol complex. The retinol-RBP-TTR complex is delivered to target tissues where retinol is taken up and utilized for various cellular processes. Delivery of retinol to cells through the circulation by the RBP-TTR complex is the maj or pathway through which cells and tissue acquire retinol.

Retinol uptake from its complexed retinol-RBP-TTR form into cells occurs by binding of RBP to cellular receptors on target cells. This interaction leads to endocytosis of the RBP-receptor complex and subsequent release of retinol from the complex, or binding of retinol to cellular retinol binding proteins (CRBP), and subsequent release of apoRBP by the cells into the plasma. Other pathways contemplate alternative mechanisms for the entry of retinol into cells, including uptake of retinol alone into the cell. See Blomhoff (1994) for review.

The methods and compositions described herein are useful for the modulation of vitamin A levels in a mammalian subject. In particular, modulation of vitamin A levels can occur through the regulation of retinol binding protein (RBP) and transthyretin (TTR) availability or activity in a mammal. The methods and compositions described herein provide for the modulation of RBP and TTR levels or activity in a mammalian subject, and subsequently modulation of vitamin A levels. Increases or decreases in vitamin A levels in a subject can have effects on retinol availability in target organs and tissues. Therefore, providing a means of modulating retinol or retinol derivative availability may correspondingly modulate disease conditions caused by a lack of or excess in local retinol or retinol derivative concentrations in the target organs and tissues.

For example, A2E, the major fluorophore of lipofuscin, is formed in macular or retinal degeneration or dystrophy, including age-related macular degeneration and Stargardt Disease, due to excess production of the visual-cycle retinoid, all-trans-retinaldehyde, a precursor of A2E. Reduction of vitamin A and all-trans retinaldehyde in the retina, therefore, would be beneficial in reducing A2E and lipofuscin build-up, and treatment of age-related macular degeneration. Studies have confirmed that reducing serum retinol may have a beneficial effect of reducing A2E and lipofuscin in RPE. For example, animals maintained on a vitamin A deficient diet have been shown to demonstrate significant reductions in lipofuscin accumulation. Katz et al., Mech. Ageing Dev., 35:291-305 (1986); Katz et al., Mech. Ageing Dev., 39:81-90 (1987); Katz et al., Biochim. Biophys. Acta, 924:432-41 (1987). Further evidence that reducing vitamin A levels may be beneficial in the progression of macular degeneration and dystrophy was shown by Radu and colleagues, where reduction in ocular vitamin A levels resulted in reductions in both lipofuscin and A2E. Radu et al., Proc. Natl. Acad. Sci. USA, 100:4742-7 (2003); Radu et al., Proc. Natl. Acad. Sci. USA, 101:5928-33 (2004).

Administration of the retinoic acid analog, N-4-(hydroxyphenyl)retinamide (HPR or fenretinide), has been shown to cause reductions in serum retinol and RBP. Formelli et al., Cancer Res. 49:6149-52 (1989); Formelli et al., J. Clin Oncol., 11:2036-42 (1993); Torrisi et al., Cancer Epidemiol. Biomarkers Prev., 3:507-10 (1994). In vitro studies have demonstrated that HPR interferes with the normal interaction of TTR with RBP. Malpeli et al., Biochim. Biophys. Acta 1294: 48-54 (1996); Holven et al., Int. J. Cancer 71:654-9 (1997).

Modulators (e.g. HPR) that inhibit delivery of retinol to cells either through interruption of binding of retinol to apo RBP or holo RBP (RBP + retinol) to its transport protein, TTR, or the increased renal excretion of RBP and TTR, therefore, would be useful in decreasing serum vitamin A levels, and buildup of retinol and its derivatives in target tissues such as the eye.

Similarly, modulators which reduce the availability of the retinol transport proteins, retinol binding protein (RBP) and transthyretin (TTR), would also be useful in decreasing serum vitamin A levels, and buildup of retinol and its derivatives and physical manifestations in target tissues, such as the eye. TTR, for example, has been shown to be a component of Drusen constituents, suggesting a direct involvement of TTR in age-related macular degeneration. Mullins, RF, FASEB J. 14:835-846 (2000); Pfeffer BA, et al., Molecular Vision 10:23-30 (2004).

The same approach to modulation of RBP and/or TTR levels or activity in a mammal is expected to find use in the treatment of metabolic disorders, such as type I or type II diabetes, IIH, bone-related disorders, such as hyperostosis, protein misfolding and aggregation diseases, such as systemic amyloidoses and Alzheimer's disease, and Alström-Hallgren syndrome.

One embodiment of the methods and compositions disclosed herein, therefore, provides for the modulation of RBP or TTR levels or activity in a mammal by administering to a mammal at least once an effective amount of at least one of the compounds chosen from the group consisting of an RBP transcription inhibitor, a TTR transcription inhibitor, an RBP translation inhibitor, a TTR translation inhibitor, an RBP clearance agent, a TTR clearance agent, an RBP antagonist, an RBP agonist, a TTR antagonist, a TTR agonist and a retinol binding protein receptor antagonist.

### Retinol Binding Protein (RBP) and Transthyretin (TTR)

Retinol binding protein, or RBP, is a single polypeptide chain, with a molecular weight of approximately 21 kD. RBP has been cloned and sequenced, and its amino acid sequence determined. Colantuni et al., Nuc. Acids Res., 11:7769-7776 (1983). The three-dimensional structure of RBP reveals a i specialized hydrophobic pocket designed to bind and protect the fat-soluble vitamin retinol. Newcomer et al., EMBO J., 3:1451-1454 (1984). In in vitro experiments, cultured hepatocytes have been shown to synthesize and secrete RBP. Blaner, W.S., Endocrine Rev., 10:308-316 (1989). Subsequent experiments have demonstrated that many cells contain mRNA for RBP, suggesting a widespread distribution of RBP synthesis throughout the body. See Blaner (1989). Most of the RBP secreted by the liver contains retinol in a 1:1 molar ratio, and retinol binding to RBP is required for normal RBP secretion.

In cells, RBP tightly binds to retinol in the endoplasmic reticulum, where it is found in high concentrations. Binding of retinol to RBP initiates a translocation of retinol-RBP from endoplasmic reticulum to the Golgi complex, followed by secretion of retinol-RBP from the cells. RBP secreted from hepatocytes also assists in the transfer of retinol from hepatocytes to stellate cells, where direct secretion of retinol-RBP into plasma takes place.

In plasma, approximately 95% of the plasma RBP is associated with transthyretin (TTR) in a 1:1 mol/mol ratio, wherein essentially all of the plasma vitamin A is bound to RBP. TTR is a well-characterized plasma protein consisting of four identical subunits with a molecular weight of 54,980. The full three-dimensional structure, elucidated by X-ray diffraction, reveals extensive β-sheets arranged tetrahedrally. Blake et al., J. Mol. Biol., 121:339-356 (1978). A channel runs through the center of the tetramer in which is located two binding sites for thyroxine. However, only one thyroxine molecule appears to be bound normally to TTR due to negative cooperativity. The complexation of TTR to RBP-retinol is thought to reduce the glomerular filtration of retinol, thereby increasing the half-life of retinol and RBP in plasma by about threefold.

### Modulation of TTR and RBP Transcription and Translation

Mice lacking RBP have an impaired retinal function and vitamin A availability. Quardro, L, et al. EMBO J. 18:4633-4644 (1999), herein incorporated by reference in its entirety. Although RBP-/- mice can acquire and store retinol in hepatocytes, they lack the ability to mobilize these hepatic retinol stores, causing a tenuous vitamin A status and making the mice entirely dependent on a regular dietary intake of vitamin A. Quardro (1999). Similarly, retinol levels are also depressed in transthyretin deficient mice, having low levels of circulating retinol and RBP, Epiksopou, V., et al. Proc. Natl. Acad. Sci 90:2375-2379 (1993); van Bennekum, A.M., et al., J. Biol. Chem. 276:1107-1113 (2001), demonstrating that TTR maintains normal levels of retinol and retinol metabolites in plasma.

Methods and compositions which modulate RBP or TTR in a subject, therefore, directly affect retinol binding and subsequent delivery of retinol to the eye. If an agent lowers the delivery of retinol to the eye of a person with a vitreoretinal disease, such as the retinopathies and macular degenerations, then lower amounts of all-*trans*-retinal will be generated in such an eye, which also lowers the amount of the A2E generated in the same eye. Because A2E is cytotoxic to the cells of the eye, in particular to the cells comprising the retina of an eye, decreased amounts of A2E in the eye of a patient with vitreoretinal disease is expected to provide benefit. Thus, modulation (in particular, down regulation) of serum levels of RBP and TTR is expected to provide benefit to patients with various vitreoretinal conditions and diseases, including but not limited to the retinopathies and the macular degenerations. Furthermore, such modulation is also expected to produce benefit for patients in, for example, treatment of metabolic disorders, such as type I or type II diabetes, IIH, bone-related disorders, such as hyperostosis, protein misfolding and aggregation diseases, such as systemic amyloidoses and Alzheimer's disease, and Alström-Hallgren syndrome. Methods of promoting lower serum levels of TTR and RBP include, by way of example only, down regulation of TTR and/or RBP transcription, down regulation of TTR and/or RBP translation, inhibition of TTR and/or RBP post-translational modification, promoting the intracellular degradation of RBP and/or TTR, inhibiting the extra-cellular secretion of RBP and/or TTR, and/or enhancing the serum clearance rates of TTR and/or RBP.

One embodiment of the methods and compositions disclosed herein is the modulation of TTR or RBP levels or activity by any means that affects the transcription of TTR or RBP, and thus expression of the respective mRNA transcript in cells. Thus, the expression of RBP or TTR receptor may be downregulated, by for example, antisense oligonucleotides to an mRNA coding for RBP or TTR, or by down-regulation of transcription of such an mRNA, or by modulation of mRNA transport, processing, degradation, etc. Such down-regulation or modulation may make use of methods known in the art, for example, by use of inhibitors of transcription.

Translation of retinol binding protein receptor from RBP and TTR mRNA may also be regulated as a means of down-regulating the expression of this protein. Such down-regulation or modulation may make use of methods known in the art, for example, by use of non-specific or specific inhibitors of RBP or TTR translation.

For example, modulation of RBP transcription or translation may occur through the administration of specific or non-specific inhibitors to RBP transcription or translation. The 5' transcriptional regulatory region of human RBP has been cloned and sequenced. See D'Onofrio, C., et al. EMBO J. 4:1981-1989 (1985); Colontuoni, V., et al., EMBO J. 6:631-636 (1987), both of which are incorporated by reference herein. Mouse RBP expression has been shown to be regulated by retinoic acid, wherein both all-trans retinoic acid and 9-cis retinoic acid have been shown to induce RBP mRNA expression in a dose- and time-dependent manner. Jessen, KA, and Satre, MA, Mol. Cell Biochem. 211:85-94 (2000). Therefore, one embodiment disclosed herein is the use of retinoic acid agonists and antagonists, such as RXR and RAR antagonists or retinyl methyl ether (see Sani, BP, et al. Biochem. Biophys. Res. Commun., 223: 293-298 (1996), herein incorporated by reference in its entirety), for the modulation of RBP transcription or translation in a cell. Other transcriptional and translation regulators of RBP include estrogen, progesterone, testosterone and dexamethasone (see Eberhardt, DM, et al., Biol. Reprod. 60:714-720 (1999); Bucco RA, et al., Endocrinology 37:3111-3122 (1996); McKearin, D.M., et al., J. Biol. Chem 263:3261-3265 (1988)). HNF-4, a member of the zinc-finger binding protein family, also regulates expression of RBP and TTR. Duncan, S.A., et al. Development 124:279-287 (1997); Hayashi, Y., et al., J. Clin. Pathol.: Mol. Pathol. 52:19-24 (1999), both of which are herein incorporated by reference. Therefore, HNF-4 agonists and antagonist, and Zn-finger binding proteins may be useful in the modulation of RBP or TTR transcription or translation.

TTR is regulated by a variety of hepatic specific transcription factors, including hepatic nuclear factor (HNF) 1, HNF-3, HNF-4 and HNF-6. See Hayashi, Y, et al., J. Clin. Pathol.: Mol. Pathol. 52:19-24 (1999); Samadani, U., et al., Mol. Cell Biol. 16:6273-6284 (1996), both of which are herein incorporated by reference in its entirety. CCAAT/enhancer binding protein (C/EBP) and fatty acid binding proteins have also been implicated in playing a role in TTR transactivation in hepatocytes. See Hayashi (1999); Puskas, L.G., et al. Proc. Natl. Acad. Sci. 100:1580-1585 (2003), herein incorporated by reference in its entirety.

Other transcriptional and translational regulators of RBP or TTR transcription or translation include siRNA, ribozymes, antibodies, antisense oligonucleotides or aptamers.

In one embodiment, short interfering RNAs (siRNAs) may modulate RBP or TTR transcription or translation through RNA interference (RNAi) or post-transcriptional gene silencing (PTGS) (see, for example, Ketting et al. (2001) Genes Develop. 15:2654-2659). siRNA molecules can target homologous mRNA molecules for destruction by cleaving the mRNA molecule within the region spanned by the siRNA molecule. Accordingly, siRNAs capable of targeting and cleaving homologous TTR or RBP mRNA, and therefore are useful for the modulation of TTR or RBP levels or activity in a subject.

In another embodiment, ribozymes may be used in the modulation of RBP or TTR transcription or translation. Ribozymes are enzymatic RNA molecules capable of catalyzing the specific cleavage of RNA. The mechanism of ribozyme action involves sequence-specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage. For this sequence, see, e.g., U.S. Pat. No. 5,093,246. While ribozymes that cleave mRNA at site-specific recognition sequences can be used to destroy mRNAs encoding RBP or TTR, the use of hammerhead ribozymes may also be used. Hammerhead ribozymes cleave mRNAs at locations dictated by flanking regions that form complementary base pairs with the target mRNA. The sole requirement is that the target mRNA has the following sequence of two bases: 5'-UG-3'. The construction and production of hammerhead ribozymes is well known in the art. The ribozymes disclosed herein may also include RNA endoribonucleases (hereinafter "Cech-type ribozymes") such as the one that occurs naturally in Tetrahymena thermophila (known as the IVS, or L-19 IVS RNA). The Cech-type ribozymes have an eight base pair active site that hybridizes to a target RNA sequence where after cleavage of the target RNA takes place. The methods and compositions herein encompasses those Cech-type ribozymes that target eight base-pair active site sequences that are present in the genes encoding RBP or TTR.

In yet another embodiment, antibodies may be used to modulate TTR or RBP transcription or translation in a subject. The term "antibody" as used herein refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant regions, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. Within each IgG class, there are different isotypes (e.g., IgG₁, IgG₂, etc.). Typically, the antigen-binding region of an antibody will be the most critical in determining specificity and affinity of binding.

An exemplary immunoglobulin (antibody) structural unit comprises a tetramer. Each tetramer is composed of two identical pairs of polypeptide chains, each pair having one light chain (about 25 kD) and one heavy chain (about 50-70 kD). The N-terminus of each chain defines a variable region of about 100-110 or more amino acids primarily responsible for antigen recognition. The terms "variable light chain" (V_{L}) and variable heavy chain (V_{H}) refer to these light and heavy chains respectively.

Methods for preparing antibodies are well known in the art. See, for example, Kohler & Milstein (1975) Nature 256:495-497; Harlow & Lane (1988) Antibodies: a Laboratory Manual, Cold Spring Harbor Lab., Cold Spring Harbor, N.Y.). The genes encoding the heavy and light chains of an antibody of interest can be cloned from a cell, e.g., the genes encoding a monoclonal antibody can be cloned from a hybridoma and used to produce a recombinant monoclorial antibody. Gene libraries encoding heavy and light chains of monoclonal antibodies can also be made from hybridoma or plasma cells. Random combinations of the heavy and light chain gene products generate a large pool of antibodies with different antigenic specificity. Techniques for the production of single chain antibodies or recombinant antibodies (U.S. Pat. No. 4,946,778; U.S. Pat. No. 4,816,567) can be adapted to produce antibodies used in the fusion proteins and methods of the instant invention. Also, transgenic mice, or other organisms such as other mammals, may be used to express human or humanized antibodies. Alternatively, phage display technology can be used to identify antibodies and heteromeric Fab fragments that specifically bind to selected antigens.

Screening and selection of preferred antibodies can be conducted by a variety of methods known to the art. Initial screening for the presence of monoclonal antibodies specific to a target antigen may be conducted through the use of ELISA-based methods, for example. A secondary screen is preferably conducted to identify and select a desired monoclonal antibody for use in construction of the multi-specific fusion proteins of the invention. Secondary screening may be conducted with any suitable method known to the art.

The modulator disclosed herein may also comprise one or more antisense compounds, including antisense RNA and antisense DNA, which are, by way of example only, capable of reducing the endogenous level of RBP or TTR within a subject. Thus, a modulator capable of lowering the level of expression of RBP or TTR in a cell such that endogenous TTR or RBP levels or activity are reduced, is included. Preferably, the antisense compounds comprise sequences complementary to RBP or TTR nucleic acids.

In one embodiment, the antisense compounds are oligomeric antisense compounds, particularly oligonucleotides. The antisense compounds specifically hybridize with one or more nucleic acids encoding RBP or TTR. As used herein, the term "nucleic acid encoding RBP or TTR" encompasses DNA encoding RBP or TTR, RNA (including pre-mRNA and mRNA) transcribed from such DNA, and also cDNA derived from such RNA.

The specific hybridization of an oligomeric compound with its target nucleic acid interferes with the normal function of the nucleic acid. This modulation of function of a target nucleic acid by compounds which specifically hybridize to it is generally referred to as "antisense". The functions of DNA to be interfered with include replication and transcription. The functions of RNA to be interfered with include all vital functions such as, for example, translocation of the RNA to the site of protein translation, translation of protein from the RNA, splicing of the RNA to yield one or more mRNA species, and catalytic activity which may be engaged in or facilitated by the RNA. The overall effect of such interference with target nucleic acid function is modulation of the expression of retinol binding protein receptor or a retinoic acid synthesis enzyme (including retinol dehydrogenase and retinal dehydrogenase). Antisense constructs are described in detail in U.S. Pat. No. 6,100,090 (Monia et al), and Neckers et al., 1992, Crit Rev Oncog 3(1-2):175-231, the teachings of which are specifically incorporated by reference herein.

In another embodiment, aptamers are used to modulate RBP or TTR transcription or translation in a subject. Aptamers refer to reagents generated in a selection from a combinatorial library (typically in vitro) wherein a target molecule, generally although not exclusively a protein or nucleic acid, is used to select from a combinatorial pool of molecules, generally although not exclusively oligonucleotides, those that are capable of binding to the target molecule. The selected reagents can be identified as primary aptamers. The term "aptamer" includes not only the primary aptamer in its original form, but also secondary aptamers derived from (i.e., created by minimizing and/or modifying) the primary aptamer. Aptamers, therefore, must behave as ligands, binding to their target molecule. See Stull and Szoka, Pharmaceutical Res. 12(4):465-483 (1995). In the methods and compositions disclosed herein, aptamers that bind to either nucleic acid or proteins involved in transcription or translation, or regulation of transcription or translation, may be used to modulate RBP or TTR transcription or translation in a subject.

A combination of two or more modulators may be used, for example, a combination of RBP modulator and a modulator of TTR transcription or translation. Such multiple treatments may be administered simultaneously or sequentially, for example, in rotation.

### Modulation of RBP or TTR Binding or Clearance in a Subject

Before retinol bound to RBP is transported in the blood stream for delivery to the eye, it must be complexed with TTR. It is this secondary complex which allows retinol to remain in the circulation for prolonged periods. In the absence of TTR, the retinol-RBP complex would be rapidly excreted in the urine. Similarly, in the absence of RBP, retinol transport in the blood stream and uptake by cells would be diminished.

Another embodiment of the invention, therefore, is to modulate availability of RBP or TTR for complexing to retinol or retinol-RBP in the blood stream by modulating RBP or TTR binding characteristics or clearance rates. As mentioned above, the TTR binding to RBP holoprotein decreases the clearance rate of RBP and retinol. Therefore, by modulating either RBP or TTR availability or activity, retinol levels may likewise be modulated in a subject in need thereof.

For example, antagonists of retinol binding to RBP may be used in the methods and compositions disclosed herein. An antagonist of retinol binding to RBP may include retinol derivatives or analogs which compete with the binding of retinol to RBP. Alternatively, an antagonist may comprise a fragment of an RBP which competes with native RBP for retinol binding, but does not allow retinol delivery to cells. This may include regions important for RBP binding to retinol binding protein receptor on cells. Alternatively, or in addition to, an immunoglobulin capable of binding to RBP or another protein, for example, on the cell surface, may be used so long as it interferes with the ability of RBP to bind to retinol and/or the uptake of retinol by the binding of RBP to retinol binding protein receptor. As above, the immunoglobulin may be a monoclonal or a polyclonal antibody.

As mentioned above, one means by which RBP binding to retinol may be modulated is to competitively bind RBP agonists or antagonists, such as retinol analogues. Therefore, one embodiment of the methods and compositions disclosed herein provides for RBP agonists or RBP antagonists in modulating RBP levels or activity. For example, administration of the retinoic acid analog, N-4-(hydroxyphenyl)retinamide (HPR or fenretinide), has been shown to cause profound reductions in serum retinol and RBP. Formelli et al., Cancer Res. 49:6149-52 (1989); Formelli et al., J. Clin Oncol., 11:2036-42 (1993); Torrisi et al., Cancer Epidemiol. Biomarkers Prev., 3:507-10 (1994). In vitro studies have demonstrated that HPR interferes with the normal interaction of TTR with RBP. See Malpeli et al., Biochim. Biophys. Acta 1294: 48-54 (1996); Holven et al., Int. J. Cancer 71:654-9 (1997).

Other examples of potential modulators of RBP levels or activity include derivatives of vitamin A, such as tretinoin (all trans-retinoic acid) and isotretinoin (13-cis-retinoic acid), which are used in the treatment of acne and certain other skin disorders. Other derivatives include ethylretinamide. In some aspects of the methods and compositions disclosed herein, it is contemplated that derivatives of retinol, retinyl derivatives and related retinoids may be used alone, or in combination with, other derivatives of retinol or related retinoids.

Further potential modulators of RBP levels or activity include retinyl derivatives having the structure of Formula (I) and Formula (II): 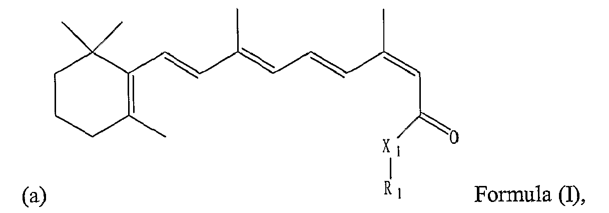 wherein X₁ is selected from the group consisting of NR², O, S, CHR²; R¹ is (CHR²)ₓ-L¹-R³, wherein x is 0, 1, 2, or 3; L¹ is a single bond or -C(O)-; R² is a moiety selected from the group consisting of H, (C₁-C₄)alkyl, F, (C₁-C₄)fluoroalkyl, (C₁-C₄)alkoxy, -C(O)OH, -C(O)-NH₂, - (C₁-C₄)alkylamine, -C(O)-(C₁-C₄)alkyl, -C(O)-(C₁-C₄)fluorallcyl, -C(O)-(C₁-C₄)alkylamine, and -C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cyloalkenyl, and a heterocycle; or an active metabolite, or a pharmaceutically acceptable prodrug or solvate thereof; or 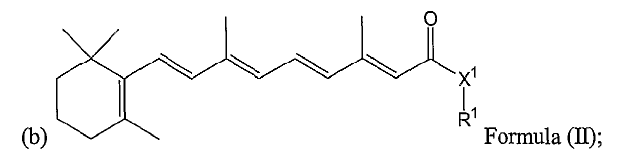 wherein X₁ is selected from the group consisting of NR², O, S, CHR²; R¹ is (CHR²)ₓ-L¹-R³, wherein x is 0, 1, 2, or 3; L¹ is a single bond or -C(O)-; R² is a moiety selected from the group consisting of H, (C₁-C₄)alkyl, F, (C₁-C₄)fluoroalkyl, (C₁-C₄)alkoxy, -C(O)OH, -C(O)-NH₂, -(C₁-C₄)alkylamine, -C(O)-(C₁-C₄)alkyl, -C(O)-(C₁-C₄)fluoroalkyl, -C(O)-(C₁-C₄)alkylamine, and - C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloalkenyl, and a heterocycle; or an active metabolite, or a pharmaceutically acceptable prodrug or solvate thereof.

Fenretinide (hereinafter referred to as hydroxyphenyl retinamide) is one example of a compound having the structure of Formula (II) and is particularly useful in the compositions and methods disclosed herein. As will be explained below, fenretinide may be used as a modulator of retinol-RBP binding. In some aspects of the methods and compositions described herein, derivatives of fenretinide may be used instead of, or in combination with, fenretinide. As used herein, a "fenretinide derivative" refers to a compound whose chemical structure is chemically derived from fenretinide.

In some embodiments, derivatives of fenretinide that may be used include, but are not limited to, C-glycoside and arylamide analogues of N-(4-hydroxyphenyl) retinamide-O-glucuronide, including but not limited to 4-(retinamido)phenyl-C-glucuronide, 4-(retinamido)phenyl-C-glucoside, 4-(retinamido)phenyl-C-xyloside, 4-(retinamido)benzyl-C-glucuronide, 4-(retinamido)benzyl-C-glucoside, 4-(retinamido)benzyl-C-xyloside; and retinoyl β-glucuronide analogues such as, for example, 1-(β-D-glucopyranosyl) retinamide and 1-(D-glucopyranosyluronosyl) retinamide, described in U.S. Pat. Nos. 5,516,792, 5,663,377, 5,599,953, 5,574,177, and Bhatnagar et al., Biochem. Pharmacol., 41:1471-7 (1991), each incorporated herein by reference.

In other embodiments, other vitamin A derivatives may be used, including those disclosed in U.S. Pat. No. 4,743,400, incorporated herein by reference. These retinoids include, for example, all-trans retinoyl chloride, all-trans-4-(methoxyphenyl) retinamide (methoxyphenyl retinamide), 13-cis-4-(hydroxyphenyl) retinamide and all-trans-4-(ethoxyphenyl) retinamide. U.S. Pat. No. 4,310,546, incorporated herein by reference, describes N-(4-acyloxyphenyl)-all-trans retinamides, such as, for example, N-(4-acetoxyphenyl)-all-trans-retinamide, N-(4-propionyloxyphenyl)-all-trans-retinamide and N-(4-n-butyryloxyphenyl- )-all-trans-retinamide, all of which are contemplated for use in certain embodiments.

Other vitamin A derivatives or metabolites, such as N-(1H-tetrazol-5-yl)retinamide, N-ethylretinamide, 13-cis-N-ethylretinamide, N-butylretinamide, etretin (acitretin), etretinate, tretinoin (all-trans-retinoic acid) or isotretinoin (13-cis-retinoic acid) may be contemplated for use in certain embodiments. See U.S. Provisional Patent Applications Nos. 60/582,293 and 60/602,675; see also Turton et al., Int. J. Exp. Pathol., 73:551-63 (1992), all herein incorporated by reference).

Similarly, modulation of TTR binding may occur with competitive binders to TTR ligand binding, such as thyroxine or tri-iodothyronine or their respective analogs, or to RBP binding on TTR. TTR is a tetrameric protein comprised of identical 127 amino acid β-sheet sandwich subunits, and its three-dimensional configuration is known. Blake, C., et al., J. Mol. Biol. 61:217-224 (1971); Blake, C. et al., J. Mol. Biol. 121:339-356 (1978). TTR complexes to holo-RBP, and increase retinol and RBP half-lives by preventing glomerular filtration of RBP and retinol. Modulating TTR binding to holo RBP, therefore, may modulate RBP and retinol levels by decreasing the half-life of these compositions.

The three-dimensional structure of TTR complexed with holo RBP shows that TTR's natural ligand, thyroxine, does not interfere with binding to RBP holoprotein. Monaco, H.L., et al. Science, 268:1039-1041 (1995). However, studies involving competitive inhibitors to thyroxine binding have shown that disruption of the TTR-RBP holoprotein complex can occur, resulting in decrease plasma retinol levels in the subject. For example, metabolites to 3,4,3',4'-tetrachlorobiphenyl reduces RBP binding sites on TTR, and inhibits formation of the TTR-RBP holoprotein complex. See Brouwer, A., et al. Chem. Biol. Interact., 68:203-17 (1988); Brouwer, A., et al., Toxicol. Appl. Pharmacol. 85:310-312 (1986). Therefore, one embodiment of the methods and compositions disclosed herein include the use of hydroxylated polyhalogenated aromatic hydrocarbon metabolites for the modulation of TTR or RBP availability.

By way of example only, other TTR modulators include diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.

In one embodiment, non-steroidal inflammatory agents may be used as TTR modulators, including but not limited to flufenamic acid, mefenamic acid, meclofenamic acid, diflunisal, diclofenac, diclofenamic acid, sulindac and indomethacin. See Peterson, S.A., et al., Proc. Natl. Acad. Sci. 95:12956-12960 (1998); Purkey, H.E., et al., Proc. Natl. Acad. Sci. 98:5566-5571 (2001), both of which are incorporated herein by reference in their entirety.

Diclofenac analogues may also be used in conjunction with the methods and compositions disclosed herein. Some examples include 2-[(2,6-dichlorophenyl)amino]benzoic acid; 2-[(3,5-dichlorophenyl)amino]benzoic acid; 3,5,-dichloro-4-[(4-nitrophenyl)amino]benzoic acid; 2-[(3,5-dichlorophenyl)amino]benzene acetic acid and 2-[(2,6-dichloro-4-carboxylic acid-phenyl)amino]benzene acetic acid. See Oza, V.B. et al., J. Med. Chem. 45:321-332 (2002), hereby incorporated by reference in its entirety. Similary, diflunisal analogues may also be used in conjunction with the methods and compositions disclosed herein. Some examples include 3',5'-difluorobiphenyl-3-ol; 2',4'-diflurobiphenyl-3-carboxylic acid; 2',4'-difluorobiphenyl-4-carboxylic acid; 2'-fluorobiphenyl-3-carboxylic acid; 2'-fluorobiphenyl-4-carboxylic acid; 3',5'-difluorobiphenyl-3-carboxylic acid; 3',5'-difluorobiphenyl-4-carboxylic acid; 2',6'-difluorobiphenyl-3-carboxylic acid; 2'6'-difluorobiphenyl-4-carboxylic acid; biphenyl-4-carboxylic acid; 4'fluoro-4-hydroxybiphenyl-3-carboxylic acid; 2'-fluoro-4-hydroxybiphenyl-3-carboxylic acid; 3',5'-difluoro-4-hydroxybiphenyl-3-carboxylic acid; 2',4'-dichloro-4-hydroxybiphenyl-3-carboxylic acid; 4-hydroxybiphenyl-3-carboxylic acid; 3'5'-difluoro-4'hydroxybiphenyl-3-carboxylic acid; 3',5'- difluoro-4'hydroxybiphenyl-4-carboxylic acid; 3',5'-dichloro-4'hydroxybiphenyl-3-carboxylic acid; 3',5'- dichloro-4'hydroxybiphenyl-4-carboxylic acid; 3',5'-dichloro-3-formylbiphenyl; 3',5'-dichloro-2-formylbiphenyl; 2',4'-dichlorobiphenyl-3-carboxylic acid; 2',4'-dichlorobiphenyl-4-carboxylic acid; 3',5'-dichlorobiphenyl-3-yl-methanol; 3',5'-dichlorobiphenyl-4-yl-methanol; or 3',5'-dichlorobiphenyl-2-yl-methanol. See Adamski-Werner, S.L., et al., J. Med. Chem. 47:355-374 (2004), the teachings of which are hereby incorporated by reference in its entirety. Bivalent inhibitors, which link small molecule analogues into one compound, may also be used in conjunction with the methods and compositions disclosed herein. Green, N.S., et al., J. Am. Chem. Soc., 125:13404-13414 (2003).

Flavonoids and related compounds have also been shown to compete with thyroxine for binding to TTR. By way of example only, some flavonoids that may be used in conjunction with the methods and compositions disclosed herein include 3-methyl-4',6-dihydroxy-3',5'-dibromoflavone or 3',5'-dibromo-2',4,4',6-tetrahydroxyaurone. Flavenoids and flavanoids, which are related to flavonoids, may also be used as modulators of TTR binding. In addition, cardiac agents have been shown to compete with thyroxine for binding to TTR. See Pedraza, P., et al., Endocrinology 137:4902-4914 (1996), herein incorporated by reference. These agents include, by way of example only, milrinone and amrinone. See Davis, PJ, et al., Biochem. Pharmacol. 36:3635-3640 (1987); Cody, V., Clin. Chem. Lab. Med. 40:1237-1243 (2002).

Additionally, hormone analogues, agonists and antagonists have been shown to be effective competitive inhibitors for thyroid hormone, including thyroxine and tri-iodothyronine. For example, diethylstilbestrol, an estrogen antagonist, has been shown to bind to and inhibit thyroxine binding. See Morais-de-Sa, E., et al., J. Biol. Chem. Epub. (Oct. 6, 2004), incorporated herein by reference in its entirety. Thyroxine-proprionic acid, thyroxine acetic acid and SKF-94901 are some examples of thyroxine analogs which may act as modulators of TTR binding. See Cody, V. (2002). In addition, retinoic acid has also been shown to inhibit thyroxine binding to human transthyretin. Smith, TJ, et al., Biochim. Biophys. Acta, 1199:76 (1994).

Other embodiments include the use of small molecule inhibitors as modulators of TTR binding. Some examples include N-phenylanthranilic acid, methyl red, mordant orange I, bisarylamine, N-benzyl-p-aminobenzoic acid, furosamide, apigenin, resveratrol, dibenzofuran, niflumic acid, or sulindac. See Baures, P.W., et al. Bioorg. & Med. Chem. 6:1389-1401 (1998), incorporated by reference herein.

Modulators for use herein are also intended to include, a protein, polypeptide or peptide including, but not limited to, a structural protein, an enzyme, a cytokine (such as an interferon and/or an interleukin), an antibiotic, a polyclonal or monoclonal antibody, or an effective part thereof, such as an Fv fragment, which antibody or part thereof may be natural, synthetic or humanised, a peptide hormone, a receptor, a signalling molecule or other protein; a nucleic acid, as defined below, including, but not limited to, an oligonucleotide or modified oligonucleotide, an antisense oligonucleotide or modified antisense oligonucleotide, cDNA, genomic DNA, an artificial or natural chromosome (e.g. a yeast artificial chromosome) or a part thereof, RNA, including mRNA, tRNA, rRNA or a ribozyme, or a peptide nucleic acid (PNA); a virus or virus-like particles; a nucleotide or ribonucleotide or synthetic analogue thereof, which may be modified or unmodified; an amino acid or analogue thereof, which may be modified or unmodified; a non-peptide (e.g., steroid) hormone; a proteoglycan; a lipid; or a carbohydrate. Small molecules, including inorganic and organic chemicals, which bind to and occupy the active site of the polypeptide thereby making the catalytic site inaccessible to substrate such that normal biological activity is prevented, are also included. Examples of small molecules include but are not limited to small peptides or peptide-like molecules.

### Detection of modulator activity

The compounds and compositions disclosed herein can also be used in assays for detecting perturbations in RBP or TTR availability through conventional means. For example, a subject may be treated with any of the compounds or compositions disclosed herein, and RBP or TTR levels quantified using conventional assay techniques. See Sundaram, M., et al., Biochem. J. 362:265-271 (2002). For example, a typical non-competitive sandwich assay is an assay disclosed in U.S. Pat. No. 4,486,530, incorporated herein by reference. In this method, a sandwich complex, for example an immune complex, is formed in an assay medium. The complex comprises the analyte, a first antibody, or binding member, that binds to the analyte and a second antibody, or binding member that binds to the analyte or a complex of the analyte and the first antibody, or binding member. Subsequently, the sandwich complex is detected and is related to the presence and/or amount of analyte in the sample. The sandwich complex is detected by virtue of the presence in the complex of a label wherein either or both the first antibody and the second antibody, or binding members, contain labels or substituents capable of combining with labels. The sample may be plasma, blood, feces, tissue, mucus, tears, saliva, or urine, for example for detecting modulation of clearance rates for RBP or TTR. For a more detailed discussion of this approach see U.S. Pat. Nos. Re 29,169 and 4,474,878, the relevant disclosures of which are incorporated herein by reference.

In a variation of the above sandwich assay, the sample in a suitable medium is contacted with labeled antibody or binding member for the analyte and incubated for a period of time. Then, the medium is contacted with a support to which is bound a second antibody, or binding member, for the analyte. After an incubation period, the support is separated from the medium and washed to remove unbound reagents. The support or the medium is examined for the presence of the label, which is related to the presence or amount of analyte. For a more detailed discussion of this approach see U.S. Pat. No. 4,098,876, the relevant disclosure of which is incorporated herein by reference.

The modulators disclosed herein may also be used in in vitro assays for detecting perturbations in RBP or TTR activity. For example, the modulator may be added to a sample comprising RBP, TTR and retinol to detect complex disruption. A component, for example, RBP, TTR, retinol or the modulator, may be labeled to determine if disruption of complex formation occurs. Complex formation and subsequent disruption may be detected and/or measured through conventional means, such as the sandwich assays disclosed above. Other detection systems may also be used to detect modulation of RBP or TTR binding, for example, FRET detection of RBP-TTR-retinol complex formation. See U.S. Provisional Patent Application No. 60/625,532 "Fluorescence Assay for Modulators of Retinol Binding," herein incorporated by reference in its entirety.

In vitro gene expression assays may also be used to detect modulation of transcription or translation of RBP or TTR by the modulators disclosed herein. For example, as described in Wodicka et al., Nature Biotechnology 15 (1997), (hereby incorporated by reference in its entirety), because mRNA hybridization correlates to gene expression level, hybridization patterns can be compared to determine differential gene expression. As a non-limiting example, hybridization patterns from samples treated with the modulators may be compared to hybridization patterns from samples which have not been treated or which have been treated with a different compound or with different amounts of the same compound. The samples may be analyzed using DNA array technology, see U.S. Patent No. 6,040,138, herein incorporated by reference in its entirety. Gene expression analysis of RBP or TTR activity may also be analyzed using recombinant DNA technology by analyzing the expression of reporter proteins driven by RBP or TTR promoter regions in an in vitro assay. See, e.g., Rapley and Walker, Molecular Biomethods Handbook (1998); Wilson and Walker, Principals and Techniques of Practical Biochemistry (2000), hereby incorporated by reference in its entirety.

In vitro translation assays may also be used to detect modulation or translation of RBP or TTR by the modulators disclosed herein. By way of example only, modulation of translation by the modulators may be detected through the use of cell-free protein translation systems, such as E. coli extract, rabbit reticulocyte lysate and wheat germ extract, see Spirin, A.S., Cell-free protein synthesis bioreactor (1991), herein incorporated by reference in its entirety, by comparing translation of proteins in the presence and absence of the modulators disclosed herein. Modulator effects on protein translation may also be monitored using protein gel electrophoretic or immune complex analysis to determine qualitative and quantitative differences after addition of the modulators.

In addition, other potential modulators which include, but are not limited to, small molecules, polypeptides, nucleic acids and antibodies, may also be screened using the in vitro detection methods described above. For example, the methods and compositions described herein may be used to screen small molecule libraries, nucleic acid libraries, peptide libraries or antibody libraries in conjunction with the teachings disclosed herein. Methods for screening libraries, such as combinatorial libraries and other libraries disclosed above, can be found in U.S. Pat. Nos. 5,591,646; 5,866,341; and 6,343,257, which are hereby incorporated by reference in its entirety.

### In vivo detection of modulator activity

In addition to the in vitro methods disclosed above, the methods and compositions disclosed herein may also be used in conjunction with in vivo detection and/or quantitation of modulator activity on TTR or RBP availability. For example, labeled TTR or RBP may be injected into a subject, wherein a candidate modulator added before, during or after the injection of the labeled TTR or RBP. The subject may be a mammal, for example a human; however other mammals, such as primates, horse, dog, sheep, goat, rabbit, mice or rats may also be used. A biological sample is then removed from the subject and the label detected to determine TTR or RBP availability. A biological sample may comprise, but is not limited to, plasma, blood, urine, feces, mucus, tissue, tears or saliva. Detection of the labeled reagents disclosed herein may take place using any of the conventional means known to those of ordinary skill in the art, depending upon the nature of the label. Examples of monitoring devices for chemiluminescence, radiolabels and other labeling compounds can be found in U.S. Pats. No. 4,618,485; 5,981,202, the relevant disclosures of which are herein incorporated by reference.

### Treatment Methods, Dosages and Combination Therapies

The compositions containing the compound(s) described herein can be administered for prophylactic and/or therapeutic treatments. The term "treating" is used to refer to either prophylactic and/or therapeutic treatments. In therapeutic applications, the compositions are administered to a patient already suffering from a disease, condition or disorder, in an amount sufficient to cure or at least partially arrest the symptoms of the disease, disorder or condition. Amounts effective for this use will depend on the severity and course of the disease, disorder or condition, previous therapy, the patient's health status and response to the drugs, and the judgment of the treating physician. It is considered well within the skill of the art for one to determine such therapeutically effective amounts by routine experimentation (*e.g*., a dose escalation clinical trial).

In prophylactic applications, compositions containing the compounds described herein are administered to a patient susceptible to or otherwise at risk of a particular disease, disorder or condition. Such an amount is defined to be a "prophylactically effective amount or dose." In this use, the precise amounts also depend on the patient's state of health, weight, and the like. It is considered well within the skill of the art for one to determine such prophylactically effective amounts by routine experimentation (e.g., a dose escalation clinical trial).

The terms "enhance" or "enhancing" means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system. When used in a patient, amounts effective for this use will depend on the severity and course of the disease, disorder or condition, previous therapy, the patient's health status and response to the drugs, and the judgment of the treating physician.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the compounds may be given continuously or temporarily suspended for a certain length of time (i.e., a "drug holiday").

Once improvement of the patient's conditions has occurred, a maintenance dose is administered if necessary. Subsequently, the dosage or the frequency of administration, or both, can be reduced, as a function of the symptoms, to a level at which the improved disease, disorder or condition is retained. Patients can, however, require intermittent treatment on a long-term basis upon any recurrence of symptoms.

The amount of a given agent that will correspond to such an amount will vary depending upon factors such as the particular compound, disease condition and its severity, the identity (e.g., weight) of the subject or host in need of treatment, but can nevertheless be routinely determined in a manner known in the art according to the particular circumstances surrounding the case, including, e.g., the specific agent being administered, the route of administration, the condition being treated, and the subject or host being treated. In general, however, doses employed for adult human treatment will typically be in the range of 0.02-5000 mg per day, preferably 1-1500 mg per day. The desired dose may conveniently be presented in a single dose or as divided doses administered simultaneously (or over a short period of time) or at appropriate intervals, for example as two, three, four or more sub-doses per day.

In certain instances, it may be appropriate to administer at least one of the compounds described herein (or a pharmaceutically acceptable salt, ester, amide, prodrug, or solvate) in combination with another therapeutic agent. By way of example only, if one of the side effects experienced by a patient upon receiving one of the compounds herein is inflammation, then it may be appropriate to administer an anti-inflammatory agent in combination with the initial therapeutic agent. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit. By way of example only, in a treatment for macular degeneration involving administration of one of the compounds described herein, increased therapeutic benefit may result by also providing the patient with other therapeutic agents or therapies for macular degeneration. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of at least one compound that modulates RBP or TTR levels or activity with nitric oxide (NO) inducers, statins, negatively charged phospholipids, anti-oxidants, minerals, anti-inflammatory agents, anti-angiogenic agents, matrix metalloproteinase inhibitors, and carotenoids. In several instances, suitable combination agents may fall within multiple categories (by way of example only, lutein is an anti-oxidant and a carotenoid). Further, the compounds that modulate RBP or TTR levels or activity may also be administered with additional agents that may provide benefit to the patient, including by way of example only cyclosporin A.

In addition, the compounds that modulates RBP or TTR levels or activity may also be used in combination with procedures that may provide additional or synergistic benefit to the patient, including, by way of example only, the use of extracorporeal rheopheresis (also known as membrane differential filtration), the use of implantable miniature telescopes, laser photocoagulation of drusen, and microstimulation therapy.

The use of anti-oxidants has been shown to benefit patients with macular degenerations and dystrophies. See, e.g., Arch. Ophthalmol., 119: 1417-36 (2001); Sparrow, et al., J. Biol. Chem., 278:18207-13 (2003). Examples of suitable anti-oxidants that could be used in combination with at least one compound that modulates RBP or TTR levels or activity include vitamin C, vitamin E, beta-carotene and other carotenoids, coenzyme Q, 4-hydroxy-2,2,6,6-tetramethylpiperidine-*N*-oxyl (also known as Tempol), lutein, butylated hydroxytoluene, resveratrol, a trolox analogue (PNU-83836-E), and bilberry extract.

The use of certain minerals has also been shown to benefit patients with macular degenerations and dystrophies. See, e.g., Arch. Ophthalmol.,119: 1417-36 (2001). Examples of suitable minerals that could be used in combination with at least one compound that modulates RBP or TTR levels or activity include copper-containing minerals, such as cupric oxide (by way of example only); zinc-containing minerals, such as zinc oxide (by way of example only); and selenium-containing compounds.

The use of certain negatively-charged phospholipids has also been shown to benefit patients with macular degenerations and dystrophies. See, e.g., Shaban & Richter, Biol. Chem., 383:537-45 (2002); Shaban, et al., Exp. Eye Res., 75:99-108 (2002). Examples of suitable negatively charged phospholipids that could be used in combination with at least one compound that modulates RBP or TTR levels or activity include cardiolipin and phosphatidylglycerol. Positively-charged and/or neutral phospholipids may also provide benefit for patients with macular degenerations and dystrophies when used in combination with compounds that modulates RBP or TTR levels or activity.

The use of certain carotenoids has been correlated with the maintenance of photoprotection necessary in photoreceptor cells. Carotenoids are naturally-occurring yellow to red pigments of the terpenoid group that can be found in plants, algae, bacteria, and certain animals, such as birds and shellfish. Carotenoids are a large class of molecules in which more than 600 naturally occurring carotenoids have been identified. Carotenoids include hydrocarbons (carotenes) and their oxygenated, alcoholic derivatives (xanthophylls). They include actinioerythrol, astaxanthin, canthaxanthin, capsanthin, capsorubin, β-8'-apo-carotenal (apo-carotenal), β-12'-apo-carotenal, α-carotene, β-carotene, "carotene" (a mixture of α- and β-carotenes), γ-carotenes, β -cyrptoxanthin, lutein, lycopene, violerythrin, zeaxanthin, and esters of hydroxyl- or carboxyl-containing members thereof. Many of the carotenoids occur in nature as *cis-* and *trans*-isomeric forms, while synthetic compounds are frequently racemic mixtures.

In humans, the retina selectively accumulates mainly two carotenoids: zeaxanthin and lutein. These two carotenoids are thought to aid in protecting the retina because they are powerful antioxidants and absorb blue light. Studies with quails establish that groups raised on carotenoid-deficient diets had retinas with low concentrations of zeaxanthin and suffered severe light damage, as evidenced by a very high number of apoptotic photoreceptor cells, while the group with high zeaxanthin concentrations had minimal damage. Examples of suitable carotenoids for in combination with at least one compound that modulates RBP or TTR levels or activity include lutein and zeaxanthin, as well as any of the aforementioned carotenoids.

Suitable nitric oxide inducers include compounds that stimulate endogenous NO or elevate levels of endogenous endothelium-derived relaxing factor (EDRF) in vivo or are substrates for nitric oxide synthase. Such compounds include, for example, L-arginine, L-homoarginine, and *N-*hydroxy-*L*-arginine, mcluding their nitrosated and nitrosylated analogs (e.g., nitrosated L-arginine, nitrosylated L-arginine, nitrosated *N*-hydroxy-*L*-arginine, nitrosylated *N*-hydroxy-*L*-arginine, nitrosated *L*-homoarginine and nitrosylated *L*-homoarginine), precursors of *L-*arginine and/or physiologically acceptable salts thereof, including, for example, citrulline, ornithine, glutamine, lysine, polypeptides comprising at least one of these amino acids, inhibitors of the enzyme arginase (*e.g*., *N*-hydroxy-*L-*arginine and 2(S)-amino-6-boronohexanoic acid) and the substrates for nitric oxide synthase, cytokines, adenosine, bradykinin, calreticulin, bisacodyl, and phenolphthalein. EDRF is a vascular relaxing factor secreted by the endothelium, and has been identified as nitric oxide or a closely related derivative thereof (Palmer et al, Nature, 327:524-526 (1987); Ignarro et al, Proc. Natl. Acad. Sci. USA, 84:9265-9269 (1987)).

Statins serve as lipid-lowering agents and/or suitable nitric oxide inducers. In addition, a relationship has been demonstrated between statin use and delayed onset or development of macular degeneration. G. McGwin, et al., British Journal of Ophthalmology, 87:1121-25 (2003). Statins can thus provide benefit to a patient suffering from an ophthalmic condition (such as the macular degenerations and dystrophies, and the retinal dystrophies) when administered in combination with compounds that modulates RBP or TTR levels or activity. Suitable statins include, by way of example only, rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium (which is the hemicalcium salt of atorvastatin), and dihydrocompactin.

Suitable anti-inflammatory agents with which the compounds that modulates RBP or TTR levels or activity may be used include, by way of example only, aspirin and other salicylates, cromolyn, nedocromil, theophylline, zileuton, zafirlukast, montelukast, pranlukast, indomethacin, and lipoxygenase inhibitors; non-steroidal antiinflammatory drugs (NSAIDs) (such as ibuprofen and naproxin); prednisone, dexamethasone, cyclooxygenase inhibitors (*i*.*e*., COX-1 and/or COX-2 inhibitors such as Naproxen™, or Celebrex™); statins (by way of example only, rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium (which is the hemicalcium salt of atorvastatin), and dihydrocompactin); and disassociated steroids.

Suitable matrix metalloproteinases (MMPs) inhibitors may also be administered in combination with compounds that modulates RBP or TTR levels or activity in order to treat ophthalmic conditions or symptoms associated with macular or retinal degenerations. MMPs are known to hydrolyze most components of the extracellular matrix. These proteinases play a central role in many biological processes such as normal tissue remodeling, embryogenesis, wound healing and angiogenesis. However, excessive expression of MMP has been observed in many disease states, including macular degeneration. Many MMPs have been identified, most of which are multidomain zinc endopeptidases. A number of metalloproteinase inhibitors are known (see for example the review of MMP inhibitors by Whittaker M. et al, Chemical Reviews 99(9):2735-2776 (1999)). Representative examples of MMP Inhibitors include Tissue Inhibitors of Metalloproteinases (TIMPs) (e.g., TIMP-1, TIMP-2, TIMP-3, or TIMP-4), α₂-macroglobulin, tetracyclines (e.g., tetracycline, minocycline, and doxycycline), hydroxamates (e.g., BATIMASTAT, MARIMISTAT and TROCADE), chelators (e.g., EDTA, cysteine, acetylcysteine, D-penicillamine, and gold salts), synthetic MMP fragments, succinyl mercaptopurines, phosphonamidates, and hydroxaminic acids. Examples of MMP inhibitors that may be used in combination with compounds that modulates RBP or TTR levels or activity include, by way of example only, any of the aforementioned inhibitors.

The use of antiangiogenic or anti-VEGF drugs has also been shown to provide benefit for patients with macular degenerations and dystrophies. Examples of suitable antiangiogenic or anti-VEGF drugs that could be used in combination with at least one compound that modulates RBP or TTR levels or activity include Rhufab V2 (Lucentis^{™}), Tryptophanyl-tRNA synthetase (TrpRS), Eye001 (Anti-VEGF Pegylated Aptamer), squalamine, Retaane^{™} 15mg (anecortave acetate for depot suspension; Alcon, Inc.), Combretastatin A4 Prodrug (CA4P), Macugen^{™}, Mifeprex^{™} (mifepristone - ru486), subtenon triamcinolone acetonide, intravitreal crystalline triamcinolone acetonide, Prinomastat (AG3340 - synthetic matrix metalloproteinase inhibitor, Pfizer), fluocinolone acetonide (including fluocinolone intraocular implant, Bausch & Lomb/Control Delivery Systems), VEGFR inhibitors (Sugen), and VEGF-Trap (Regeneron/Aventis).

Other pharmaceutical therapies that have been used to relieve visual impairment can be used in combination with at least one compound that modulates RBP or TTR levels or activity. Such treatments include but are not limited to agents such as Visudyne^{™} with use of a non-thermal laser, PKC 412, Endovion (NeuroSearch A/S), neurotrophic factors, including by way of example Glial Derived Neurotrophic Factor and Ciliary Neurotrophic Factor, diatazem, dorzolamide, Phototrop, 9-*cis*-retinal, eye medication (including Echo Therapy) including phospholine iodide or echothiophate or carbonic anhydrase inhibitors, AE-941 (AEterna Laboratories, Inc.), Sima-027 (Sima Therapeutics, Inc.), pegaptanib (NeXstar Pharmaceuticals/Gilead Sciences), neurotrophins (including, by way of example only, NT-4/5, Genentech), Cand5 (Acuity Pharmaceuticals), ranibizumab (Genentech), INS-37217 (Inspire Pharmaceuticals), integrin antagonists (including those from Jerini AG and Abbott Laboratories), EG-3306 (Ark Therapeutics Ltd.), BDM-E (BioDiem Ltd.), thalidomide (as used, for example, by EntreMed, Inc.), cardiotrophin-1 (Genentech), 2-methoxyestradiol (Allergan/Oculex), DL-8234 (Toray Industries), NTC-200 (Neurotech), tetrathiomolybdate (University of Michigan), LYN-002 (Lynkeus Biotech), microalgal compound (Aquasearch/Albany, Mera Pharmaceuticals), D-9120 (Celltech Group plc), ATX-S10 (Hamamatsu Photonics), TGF-beta 2 (Genzyme/Celtrix), tyrosine kinase inhibitors (Allergan, SUGEN, Pfizer), NX-278-L (NeXstar Pharmaceuticals/Gilead Sciences), Opt-24 (OPTIS France SA), retinal cell ganglion neuroprotectants (Cogent Neurosciences), N-nitropyrazole derivatives (Texas A&M University System), KP-102 (Krenitsky Pharmaceuticals), and cyclosporin A. *See* U.S. Patent Application Publication No. 20040092435.

For the treatment of diabetes, the methods and compositions disclosed herein further comprise administration of a second compound selected from the group consisting of (a) a glucose-lowering hormone or hormone mimetic (e.g., insulin, GLP-1 or a GLP-1 analog, exendin-4 or liraglutide), (b) a glucose-lowering sulfonylurea (e.g., acetohexamide, chlorpropamide, tolbutamide, tolazamide, glimepiride, a glipizide, glyburide, a micronized gylburide, or a gliclazide), (c) a glucose-lowering biguanide (metformin), (d) a glucose-lowering meglitinide (e.g., nateglinide or repaglinide), (e) a glucose-lowering thiazolidinedione or other PPAR-gamma agonist (e.g., pioglitazone, rosiglitazone, troglitazone, or isagitazone), (f) a glucose-lowering dual-acting PPAR agonist with affinity for both PPAR-gamma and PPAR-alpha (e.g., BMS-298585 and tesaglitazar), (g) a glucose-lowering alpha-glucosidase inhibitor (e.g., acarbose or miglitol), (h) a glucose-lowerinng antisense compound not targeted to glucose-6-phosphatase translocase, (i) an anti-obesity appetite suppressant (e.g. phentermine), (j) an anti-obesity fat absorption inhibitor such as orlistat, (k) an anti-obesity modified form of ciliary neurotrophic factor which inhibits hunger signals that stimulate appetite, (1) a lipid-lowering bile salt sequestering resin (e.g., cholestyramine, colestipol, and colesevelam hydrochloride), (m) a lipid-lowering HMGCoA-reductase inhibitor (e.g., lovastatin, cerivastatin, prevastatin, atorvastatin, simvastatin, and fluvastatin), (n) a nicotinic acid, (o) a lipid-lowering fibric acid derivative (e.g., clofibrate, gemfibrozil, fenofibrate, bezafibrate, and ciprofibrate), (p) agents including probucol, neomycin, dextrothyroxine, (q) plant-stanol esters, (r) cholesterol absorption inhibitors (e.g., ezetimibe), (s) CETP inhibitors (e.g. torcetrapib and JTT-705), (t) MTP inhibitors (eg, implitapide), (u) inhibitors of bile acid transporters (apical sodium-dependent bile acid transporters), (v) regulators of hepatic CYP7a, (w) ACAT inhibitors (e.g. Avasimibe), (x) lipid-lowering estrogen replacement therapeutics (e.g., tamoxigen), (y) synthetic HDL (e.g. ETC-216), or (z) lipid-lowering anti-inflammatories (e.g., glucocorticoids). When the second compound has a different target and/or acts by a different mode of action from the agents described herein (i.e., those that modulate RBP or TTR levels or activity), the administration of the two agents in combination (e.g., simultaneous, sequential or separate administration) is expected to provide additive or synergistic therapeutic benefit to a patient with diabetes. For the same reason, the administration of the two agents in combination (e.g., simultaneous, sequential or separate administration) is expected to allow lower doses of each or either agent relative to the dose of such agent in the absence of the combination therapy while still achieving a desired therapeutic benefit, including by way of example only, reduction in blood glucose and HbAlc control.

In any case, the multiple therapeutic agents (one of which is one of the compounds described herein) may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks. In addition, the combination methods, compositions and formulations are not to be limited to the use of only two agents; we envision the use of multiple therapeutic combinations. By way of example only, a compound that modulates RBP or TTR levels or activity may be provided with at least one antioxidant and at least one negatively charged phospholipid; or a compound that modulates RBP or TTR levels or activity may be provided with at least one antioxidant and at least one inducer of nitric oxide production; or a compound that modulates RBP or TTR levels or activity may be provided with at least one inducer of nitric oxide productions and at least one negatively charged phospholipid; and so forth.

In addition, the compounds that modulate RBP or TTR levels or activity may also be used in combination with procedures that may provide additional or synergistic benefit to the patient. Procedures known, proposed or considered to relieve visual impairment include but are not limited to 'limited retinal translocation', photodynamic therapy (including, by way of example only, receptor-targeted PDT, Bristol-Myers Squibb, Co.; porfimer sodium for injection with PDT; verteporfin, QLT Inc.; rostaporfin with PDT, Miravent Medical Technologies; talaporfin sodium with PDT, Nippon Petroleum; motexafin lutetium, Pharmacyclics" Inc.), antisense oligonucleotides (including, by way of example, products tested by Novagali Pharma SA and ISIS-13650, Isis Pharmaceuticals), laser photocoagulation, drusen lasering, macular hole surgery, macular translocation surgery, implantable miniature telescopes, Phi-Motion Angiography (also known as Micro-Laser Therapy and Feeder Vessel Treatment), Proton Beam Therapy, microstimulation therapy, Retinal Detachment and Vitreous Surgery, Scleral Buckle, Submacular Surgery, Transpupillary Thermotherapy, Photosystem I therapy, use of RNA interference (RNAi), extracorporeal rheopheresis (also known as membrane differential filtration and Rheotherapy), microchip implantation, stem cell therapy, gene replacement therapy, ribozyme gene therapy (including gene therapy for hypoxia response element, Oxford Biomedica; Lentipak, Genetix; PDEF gene therapy, GenVec), photoreceptor/retinal cells transplantation (including transplantable retinal epithelial cells, Diacrin, Inc.; retinal cell transplant, Cell Genesys, Inc.), and acupuncture.

Further combinations that may be used to benefit an individual include using genetic testing to determine whether that individual is a carrier of a mutant gene that is known to be correlated with certain ophthalmic conditions. By way of example only, defects in the human *ABCA4* gene are thought to be associated with five distinct retinal phenotypes including Stargardt disease, cone-rod dystrophy, age-related macular degeneration and retinitis pigmentosa. See e.g., Allikmets et al., Science, 277:1805-07 (1997); Lewis et al., Am. J. Hum. Genet., 64:422-34 (1999); Stone et al., Nature Genetics, 20:328-29 (1998); Allikmets, Am. J. Hum. Gen., 67:793-799 (2000); Klevering, et al, Ophthalmology, 111:546-553 (2004). Such patients are expected to find therapeutic and/or prophylactic benefit in the methods described herein.

In addition to the aforementioned ingredients, the formulations disclosed herein may further include one or more optional accessory ingredient(s) utilized in the art of pharmaceutical formulations, i.e., diluents, buffers, flavoring agents, colorants, binders, surface active agents, thickeners, lubricants, suspending agents, preservatives (including antioxidants) and the like.

The compound may also be administered multiply to the subject, with time between multiple administrations comprising at least several hours, or one day, or up to one week or more. The compound may also be administered every twelve hours, on a daily basis, every two days, every three days, on a weekly basis, or any other suitable period that would be effective for modulation of vitamin A levels.

The subject, in conjunction with administration of the compounds above, may also be monitored for physiological manifestations of retinol-related disease processes. For example, the subject may be monitored for physiological manifestations of age-related macular degenerations or dystrophies, including the formation of drusen in the eye of the subject, measuring the levels of lipofuscin in the eye of the subject, measuring the auto-fluorescence of A2E and precursors of A2E, and measuring N-retinylidene-N-reinylethanolamine levels in the eye of the subject. Furthermore, the subject will also be monitored for changes or perturbations in vitamin A levels, as well as RBP and TTR levels or activity in a biological sample.

### EXAMPLES

The following ingredients, processes and procedures for practicing the methods disclosed herein correspond to that described above. The procedures below describe with particularity a presently preferred embodiment of the process for the detection and screening of modulators to retinol binding. Any methods, materials, reagents or excipients which are not particularly described will be generally known and available those skilled in the assay and screening arts.

### Example 1: Identification of compounds that inhibit gene expression of TTR

The identified test compound may be administered to a culture of human cells transfected with a TTR expression construct and incubated at 37 °C. for 10 to 45 minutes. A culture of the same type of cells that have not been transfected is incubated for the same time without the test compound to provide a negative control.

RNA is then isolated from the two cultures as described in Chirgwin et al., Biochem. 18, 5294-99, 1979). Northern blots are prepared using 20 to 30 µg total RNA and hybridized with a ³²P-labeled TTR-specific probe. Probes for detecting TTR mRNA transcripts have been described previously. A test compound that decreases the TTR-specific signal relative to the signal obtained in the absence of the test compound is identified as an inhibitor of TTR gene expression.

### Example 2: Identification of compounds that bind to RBP and/or inhibit gene expression of RBP

The identified test compound may be administered to a culture of human cells transfected with an RBP expression construct and incubated at 37 °C. for 10 to 45 minutes. A culture of the same type of cells that have not been transfected is incubated for the same time without the test compound to provide a negative control.

RNA is then isolated from the two cultures as described in Chirgwin et al., Biochem. 18, 5294-99, 1979). Northern blots are prepared using 20 to 30 µg total RNA and hybridized with a ³²P-labeled RBP-specific probe. A test compound that decreases the RBP-specific signal relative to the signal obtained in the absence of the test compound is identified as an inhibitor of RBP gene expression.

### Example 3: Detecting the presence of A2E and/or Precursors.

In *abcr⁻*/*⁻* and wild type mice, the levels of A2E in the RPE are determined by HPLC and levels of A2E can be determined by using a confocal scanning laser ophthalmoscope and measuring their absorption at 430 nm.

### Example 4: Testing for Protection from Light Damage

The following study is adapted from Sieving, P.A., et al, Proc. Natl. Acad. Sci., 98:1835-40 (2001). For chronic light-exposure studies, Sprague-Dawley male 7-wk-old albino rats are housed in 12:12 h light/dark cycle of 5 lux fluorescent white light. For acute light-exposure studies, rats are dark-adapted overnight and before being exposed to the bleaching light before ERG measurements. Rats exposed to 2,000 lux white fluorescent light for 48 h. ERGs are recorded 7 d later, and histology is performed immediately.

Rats are euthanized and eyes are removed and sliced. Column cell counts of outer nuclear layer thickness and rod outer segment (ROS) length are measured every 200 µm across both hemispheres, and the numbers are averaged to obtain a measure of cellular changes across the entire retina. The levels of A2E in the RPE are determined by HPLC and levels of A2E can be determined by using a confocal scanning laser ophthalmoscope and measuring their absorption at 430 nm.

### Example 5: Monitoring the Effectiveness of Ophthalmic Treatment, Therapies or Drugs

Assessing the effectiveness of treatments, therapies or drugs which have an effect on macular or retinal degenerations and dystrophies can be a three step process which involves 1) taking initial measurements of a subject, such as the formation of drusen in the eye of the subject, size and number of geographic atrophy in the eye of the subject, measuring the levels of lipofuscin in the eye of the subject by measuring auto-fluorescence of A2E or lipofuscin and precursors of A2E, or measuring N-retinylidene-N-reinylethanolamine levels in the eye of the subject. 2) providing treatment, therapy or drug to the subject, 3) taking measurements of the formation of drusen in the eye of the subject, size and number of geographic atrophy in the eye of the subject, measuring the levels of lipofuscin in the eye of the subject by measuring the auto-fluorescence of A2E or lipofuscin and precursors of A2E, or measuring N-retinylidene-N-reinylethanolamine levels in the eye of the subject after step (2), and assessing results which would indicate that the treatment, therapy or drug may have a desired effect. A desired result may include a decrease or suspension in the formation of drusen, the levels of lipofuscin in the eye of the subject the auto-fluorescence of A2E and precursors of A2E, or N-retinylidene-N-reinylethanolamine levels in the eye(s) of the subject. Reiteration of steps 2-3 may be administered with or without intervals of non-treatment. Subjects may include but are not limited to mice and/or rats and/or human patients.

### Example 6: Monitoring the Effectiveness of TTR or RBP Modulators on Diabetic Patients

TTR or RBP modulators can be tested in well-established mouse models, including NOD (non-obese diabetic) mouse, as well as Biobreeding (BB), and streptozotocin-induced diabetic rats. See U.S. Patent No. 6,770,272, incorporated herein in its entirety, and Tuitoek, PJ, et al., Int. J. Vitam. Nutr. Res. 66:101-5 (1996). The compounds can be tested against the formation of diabetes in mice or rats, or administered in mice with established diabetic symptoms.

Briefly, TTR or RBP may be administered by intraperitoneal injection into 6-week old mice prior to the formation of diabetic symptomology. The mice can be checked at 25 weeks of age, wherein a decrease of diabetic incidence in control animals versus treatment groups indicates a potential therapeutic candidate in diabetes treatment.

The TTR or RBP modulators can also be administered to human patients to inhibit the development of diabetes. The compounds can be formulated for oral, intravenous, subcutaneous, intramuscular, transdermal or inhalation administration in a pharmaceutically acceptable carrier (e.g., saline). The therapeutic compositions can be administered to the patient upon discovery of anti-beta cell autoimmunity and/or subtle pre-diabetic changes in glucose metabolism (i.e. blunted early i.v. glucose tolerance test), and administration is repeated every day or at a frequency as low as once per week, depending upon the patient's response. The preferred dosage of the modulators can be determined by using standard techniques to monitor glucose levels, anti-beta cells autoantibody level, or abnormalities in glucose tolerance tests of the human being treated.

### Example 7: In-Vivo Analyses of the Relationship of Serum HPR Levels to the Levels of Serum Retinol, and Ocular Retinoids and A2E

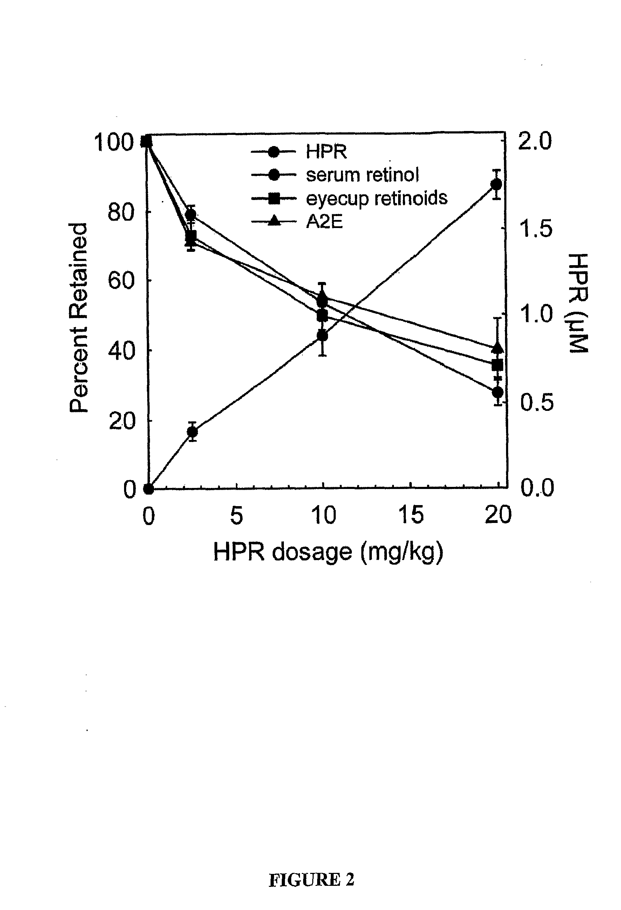

In order to explore the role of HPR in the visual cycle, the *in vivo* effects of HPR in mice have been examined. Thus, HPR was administered to ABCA4 null mutant mice (5 - 20 mg/kg, i.p. in DMSO) for periods of 28 days. Control mice received only the DMSO vehicle. At the end of the treatment period, the concentrations of retinol and HPR in serum and retinoid content in ocular tissues was measured. Profound reductions in serum retinol as a function of increasing serum HPR was observed. This effect was associated with commensurate reductions in ocular retinoids and A2E (a toxic retinoid-based fluorophore). Thus, the calculated percent reduction for each of the measured retinoids, and A2E, was nearly identical (see FIG. 2). These results indicate that reduction of ocular retinoids and A2E resulting from systemic administration of HPR results from reductions in serum retinol levels.

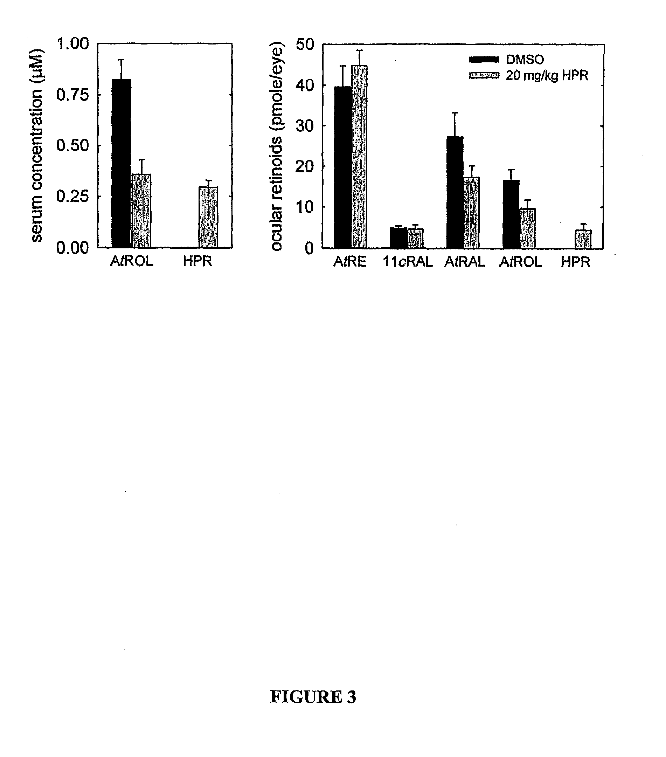

In order to ensure that the observed effects of HPR in ABCA4 null mice were not due to the genetic mutation, HPR (20 mg/kg, i.p. in DMSO) was administered to wild type mice for 5 days. Control mice received only the DMSO vehicle. On the final day of HPR treatment, the mice were exposed to constant illumination (1000 lux for 10 min) in order "stimulate" the visual cycle to generate visual chromophore. Immediately following the illumination period, the animals were sacrificed and the concentrations of retinoids in serum and ocular tissue were determined. The data (see FIG. 3) reveal no significant inhibition in synthesis of either retinyl esters or visual chromophore. As in the previous study, HPR caused a significant reduction in serum retinol (∼ 55%), ocular retinol (∼ 40%) and ocular retinal (∼ 30%). Although HPR did accumulate within ocular tissues during the treatment period (∼ 5 µM), no effect on LRAT or Rpe65/isomerase activities was observed.

Genetic crosses of RBP4^{-/-} mice with ABCA4^{-/-} mice was undertaken to examine the role of RBP in mediation of retinol levels in serum and ocular tissue. Mice from the first generation of this cross (i.e., RBP4/ABCA4^{+/-}) show comparable levels of RBP-retinol reduction as observed in the HPR study when the administered dose was 10 mg/kg (∼ 50-60% reduction in serum RBP-retinol). Moreover, the RBP4/ABCA4^{+/-} mice show commensurate reductions in ocular retinol (∼ 60% reduction). These findings are consistent with data obtained during pharmacological modulation of RBP-retinol with HPR and, therefore, strongly suggest that A2E-based fluorophores will be reduced proportionately. The inhibition of LRAT activity has not been observed in mice receiving acute and chronic doses of HPR.

### Example 8: High-throughput assay for detection of RBP/TTR interaction

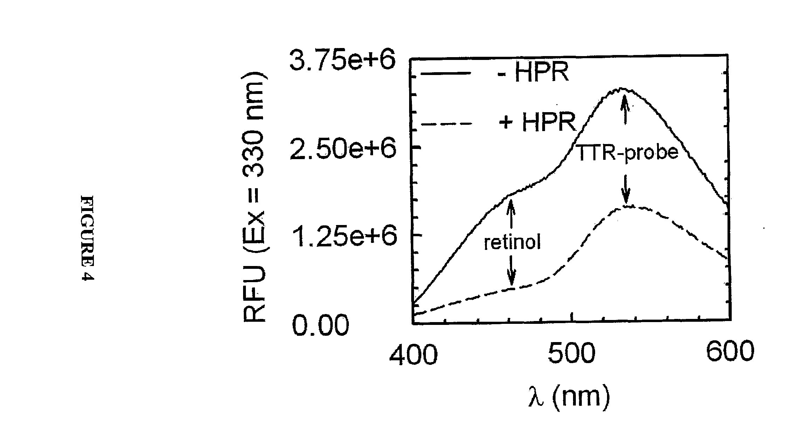

Reduction of serum retinol and RBP are correlated with concomitant reductions in toxic lipofuscin fluorophores. Because compounds that affect RBP-TTR interaction will directly affect fluorophore levels in the eye, a high-throughput screen for small molecules which prevent interaction of RBP with TTR was developed. This screen employs probe-labeled forms of RBP and TTR which participate in a unique fluorescence resonance energy transfer (FRET) event when complexed. Compounds which interfere with RBP-TTR interaction prevent FRET. Sample spectra taken during the course of this type of assay are shown in FIG. 4. These data show interaction of RBP-TTR (0.5 µM unlabeled RBP + 0.5 µM Alexa430-TTR) in the absence (solid line) and presence (dashed line) of HPR (1 µM). The sample is incubated at 37°C for 30 min and then illuminated with 330 nm light. The emission spectra are shown in the range of 400 - 600 nm. HPR binds to RBP and prevents interaction with TTR, and here this property of HPR is utilized here to validate the ability of this screen to detect inhibition of RBP-TTR interaction. The presence of HPR is associated with significantly reduced retinol and TTR-probe fluorescence indicating loss of complexation. Additionally, the design of this assay permits discrimination between compounds which interact with RBP versus those which interact with TTR. Thus, by using two distinct excitation energies (280 nm and 330 nm, for protein and retinol, respectively) and implementing simultaneous monitoring of the retinol and TTR-probe fluorescence, the "target" of a presumptive small molecule can be easily determined.

### Example 9: Assay Validation and Comparison to Conventional Techniques

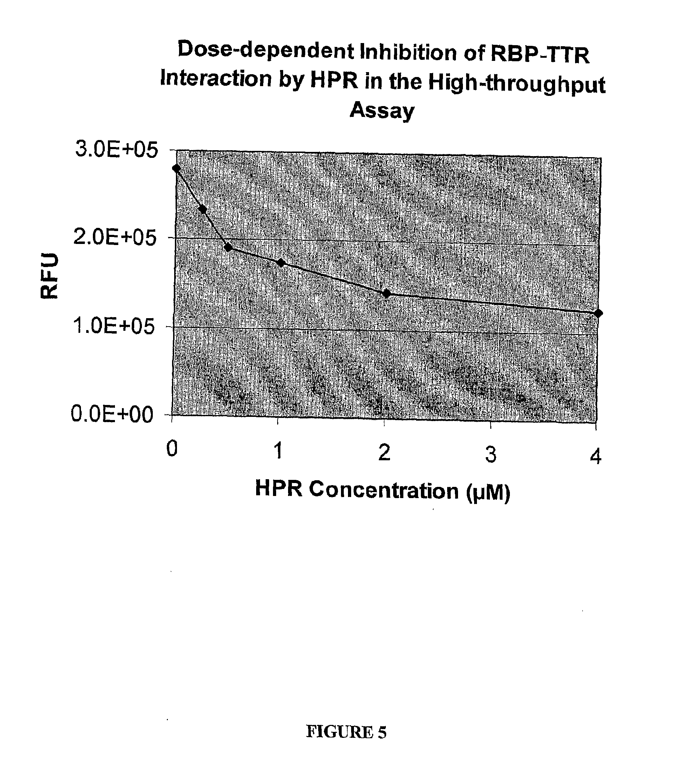

HPR is an effective inhibitor of RBP-TTR interaction as shown by chromatographic and spectrophotometric measurement techniques (*See, e.g.,* Radu RA, Han Y, Bui TV, Nusinowitz S, Bok D, Lichter J, Widder K, Travis GH and Mata NL; Reductions in Serum Vitamin A Arrest Accumulation of Toxic Retinal Fluorophores: A Potential Therapy for Treatment of Lipofuscin-based Retinal Diseases, Invest Ophthalmol. Vis Sci., in press (2005)). Thus, HPR may be used as a positive control to validate the capacity of the high throughput assay to detect inhibitors of RBP-TTR interaction. Accordingly, HPR was employed at varied concentrations (from 0 - 4 µM), using the conditions specified in Example 7, to evaluate the high throughput assay. As shown in FIG. 5, the high throughput assay is effective to detect compounds which, like HPR, inhibit RBP-TTR interaction.

Physiologically, RBP-retinol must complex with TTR in order to achieve a high steady-state concentration of RBP-retinol. This interaction creates a large molecular size complex which resists glomerular filtration and permits delivery of retinol to extra-hepatic target tissues. Inhibition of RBP-TTR interaction results in a reduction of circulating RBP as the relatively small sized RBP-ligand complex would be lost through glomerular filtration. The reduction in circulating RBP then causes a reduction in circulating retinol. This effect has been established *in vivo* for HPR by several investigators. This effect has also been observed *in vivo* using all*-trans* and 13*-cis* retinoic acids (*See, e.g.,* Berni R, Clerici M, Malpeli G, Cleris L, Formelli F; Retinoids: in vitro interaction with retinol-binding protein and influence on plasma retinol, FASEB J. (1993) 7:1179-84).

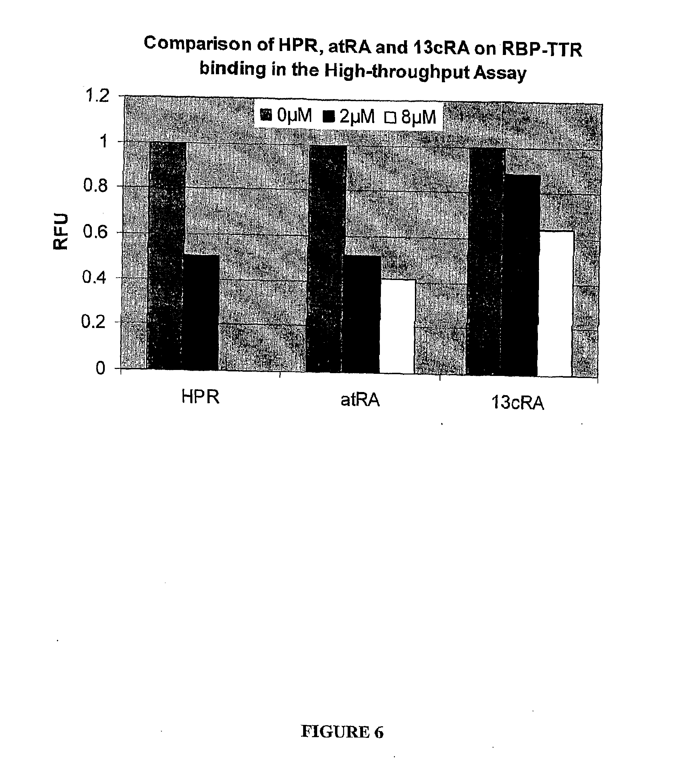

The mechanism of action underlying this effect can be explained by the disruption of RBP-TTR interactions. In order to explore this possibility and to further validate the RBP-TTR screen, the effects of all-*trans* retinoic and 13-*cis* retinoic acid, using the conditions the conditions specified for analysis of HPR, were examined. The data obtained (see FIG. 6) are entirely consistent with the *in vivo* data. This finding further validates the ability of this assay to detect known physiological inhibitors of RBP-TTR interaction.

### Example 10: Testing for the Efficacy of Compounds Which Modulate RBP or TTR Levels or Activity to Treat Macular Degeneration - Fenretinide As An Illustrative Compound

For pre-testing, all human patients undergo a routine ophthalmologic examination including fluorescein angiography, measurement of visual acuity, electrophysiologic parameters and biochemical and rheologic parameters. Inclusion criteria are as follows: visual acuity between 20/160 and 20/32 in at least one eye and signs of AMD such as drusen, areolar atrophy, pigment clumping, pigment epithelium detachment, or subretinal neovascularization. Patients that are pregnant or actively breast-feeding children are excluded from the study.

Two hundred human patients diagnosed with macular degeneration, or who have progressive formations of A2E, lipofuscin, or drusen in their eyes are divided into a control group of about 100 patients and an experimental group of 100 patients. Fenretinide is administered to the experimental group on a daily basis. A placebo is administered to the control group in the same regime as fenretinide is administered to the experimental group.

Administration of fenretinide or placebo to a patient can be either orally or parenterally administered at amounts effective to inhibit the development or reoccurrence of macular degeneration. Effective dosage amounts are in the range of from about 1-4000 mg/m² up to three times a day.

One method for measuring progression of macular degeneration in both control and experimental groups is the best corrected visual acuity as measured by Early Treatment Diabetic Retinopathy Study (ETDRS) charts (Lighthouse, Long Island, NY) using line assessment and the forced choice method (Ferris et al. Am J Ophthalmol, 94:97-98 (1982)). Visual acuity is recorded in logMAR. The change of one line on the ETDRS chart is equivalent to 0.1 logMAR. Further typical methods for measuring progression of macular degeneration in both control and experimental groups include use of visual field examinations, including but not limited to a Humphrey visual field examination, and measuring/monitoring the autofluorescence or absorption spectra of *N*-retinylidene-phosphatidylethanolamine, dihydro-*N*-retinylidene-*N*-retinyl-phosphatidylethanolamine, *N*-retinylidene-*N*-retinyl-phosphatidylethanolamine, dihydro-*N*-retinylidene-*N*-retinyl-ethanolamine, and/or *N-*retinylidene-phosphatidylethanolamine in the eye of the patient. Autofluorescence is measured using a variety of equipment, including but not limited to a confocal scanning laser ophthalmoscope. *See* Bindewald, et al., Am. J. Ophthalmol., 137:556-8 (2004).

Additional methods for measuring progression of macular degeneration in both control and experimental groups include taking fundus photographs, observing changes in autofluorescence over time using a Heidelberg retina angiograph (or alternatively, techniques described in M. Hammer, et al. Ophthalmologe 2004 Apr. 7 [Epub ahead of patent]), and taking fluorescein angiograms at baseline, three, six, nine and twelve months at follow-up visits. Documentation of morphologic changes include changes in (a) drusen size, character, and distribution; (b) development and progression of choroidal neovascularization; (c) other interval fundus changes or abnormalities; (d) reading speed and/or reading acuity; (e) scotoma size; or (f) the size and number of the geographic atrophy lesions. In addition, Amsler Grid Test and color testing are optionally administered.

To assess statistically visual improvement during drug administration, examiners use the ETDRS (LogMAR) chart and a standardized refraction and visual acuity protocol. Evaluation of the mean ETDRS (LogMAR) best corrected visual acuity (BCVA) from baseline through the available post-treatment interval visits can aid in determining statistical visual improvement.

To assess the ANOVA (analysis of variance between groups) between the control and experimental group, the mean changes in ETDRS (LogMAR) visual acuity from baseline through the available post-treatment interval visits are compared using two-group ANOVA with repeated measures analysis with unstructured covariance using SAS/STAT Software (SAS Institutes Inc, Cary, North Carolina).

Toxicity evaluation after the commencement of the study include check ups every three months during the subsequent year, every four months the year after and subsequently every six months. Plasma levels of fenretinide and its metabolite *N*-(4-methoxyphenyl)-retinamide can also be assessed during these visits. The toxicity evaluation includes patients using fenretinide as well as the patients in the control group.

### Example 11: Testing for the Efficacy of Compounds Which Modulate RBP or TTR Levels or Activity to Reduce A2E Production - Fenretinide As An Illustrative Compound

The same protocol design, including pre-testing, administration, dosing and toxicity evaluation protocols, that are described in Example 1 are also used to test for the efficacy of compounds of which modulate RBP and TTR levels or activity in reducing or otherwise limiting the production of A2E in the eye of a patient.

Methods for measuring or monitoring formation of A2E include the use of autofluorescence measurements of *N*-retinylidene-phosphatidylethanolamine, dihydro-*N*-retinylidene-*N*-retinyl-phosphatidylethanolamine, *N*-retinylidene-*N*-retinyl-phosphatidylethanolamine, dihydro-*N*-retinylidene-*N*-retinyl-ethanolamine, and/or *N*-retinylidene-phosphatidylethanolamine in the eye of the patient. Autofluorescence is measured using a variety of equipment, including but not limited to a confocal scanning laser ophthalmoscope, see Bindewald, et al., Am. J. Ophthalmol., 137:556-8 (2004), or the autofluorescence or absorption spectra measurement techniques noted in Example 1. Other tests that can be used as surrogate markers for the efficacy of a particular treatment include the use of visual acuity and visual field examinations, reading speed and/or reading acuity examinations, measurements on the size and number of scotoma and/or geographic atrophic lesions, as described in Example 1. The statistical analyses described in Example 1 is employed.

### Example 12: Testing for the Efficacy of Compounds Which Modulate RBP or TTR Levels or Activity to Reduce Lipofuscin Production - Fenretinide As An Illustrative Compound

The same protocol design, including pre-testing, administration, dosing and toxicity evaluation protocols, that are described in Example 1 are also used to test for the efficacy of compounds that modulate RBP or TTR levels or activity in reducing or otherwise limiting the production of lipofuscin in the eye of a patient. The statistical analyses described in Example 1 may also be employed.

Tests that can be used as surrogate markers for the efficacy of a particular treatment include the use of visual acuity and visual field examinations, reading speed and/or reading acuity examinations, measurements on the size and number of scotoma and/or geographic atrophic lesions, and the measuring/monitoring of autofluorescence of certain compounds in the eye of the patient, as described in Example 1.

### Example 13: Testing for the Efficacy of Compounds Which Modulate RBP or TTR Levels or Activity to Reduce Drusen Production - Fenretinide As An Illustrative Compound

The same protocol design, including pre-testing, administration, dosing and toxicity evaluation protocols, that are described in Example 1 are also used to test for the efficacy of compounds that modulate RBP or TTR levels or activity in reducing or otherwise limiting the production or formation of drusen in the eye of a patient. The statistical analyses described in Example 1 may also be employed.

Methods for measuring progressive formations of drusen in both control and experimental groups include taking fundus photographs and fluorescein angiograms at baseline, three, six, nine and twelve months at follow-up visits. Documentation of morphologic changes may include changes in (a) drusen size, character, and distribution (b) development and progression of choroidal neovascularization and (c) other interval fundus changes or abnormalities. Other tests that can be used as surrogate markers for the efficacy of a particular treatment include the use of visual acuity and visual field examinations, reading speed and/or reading acuity examinations, measurements on the size and number of scotoma and/or geographic atrophic lesions, and the measuring/monitoring of autofluorescence of certain compounds in the eye of the patient, as described in Example 1.

### Example 14: Efficacy of Fenretinide on the Accumulation of Lipofuscin (and/or A2E) in abca4 null Mutant Mice: Phase I - Dose Response and Effect on Serum Retinol.

The effect of HPR on reducing serum retinol in animals and human subjects led us to explore the possibility that reductions in lipofuscin and the toxic bis-retinoid conjugate, A2E, may also be realized. The rationale for this approach is based upon two independent lines of scientific evidence: 1) reduction in ocular vitamin A concentration via inhibition of a known visual cycle enzyme (11-*cis* retinol dehydrogenase) results in profound reductions in lipofuscin and A2E; 2) animals maintained on a vitamin A deficient diet demonstrate dramatic reductions in lipofuscin accumulation. Thus, the objective for this example was to examine the effect of HPR in an animal model which demonstrates massive accumulation of lipofuscin and A2E in ocular tissue, the *abca4* null mutant mouse.

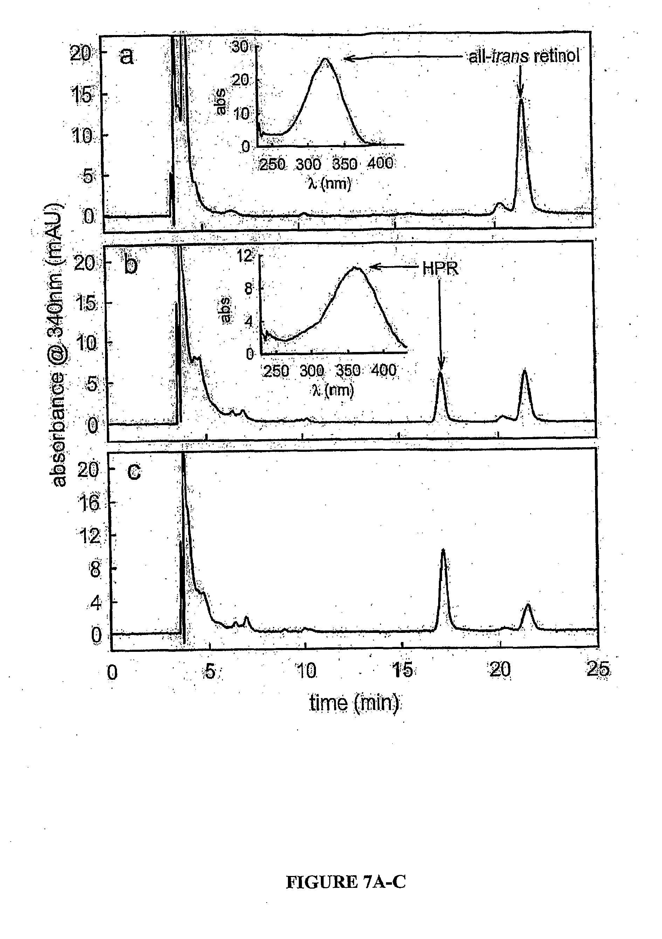

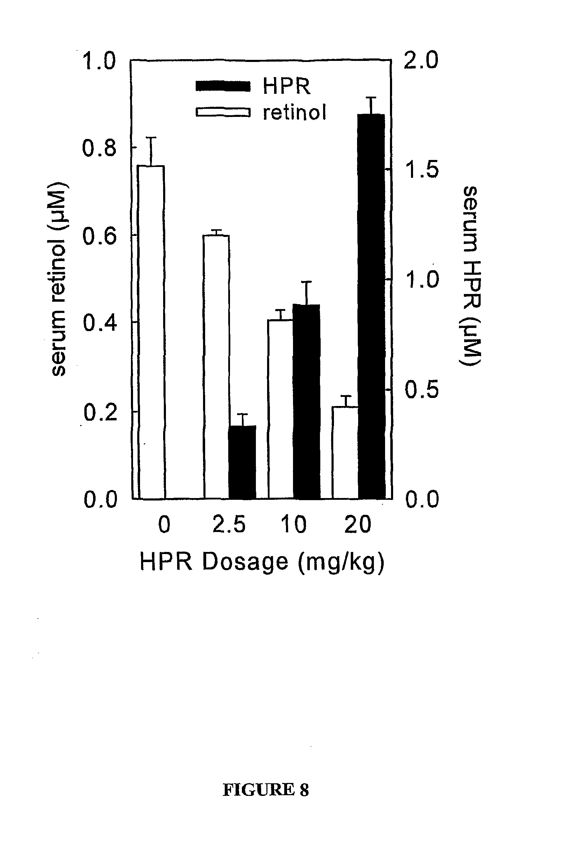

Initial studies began by examining the effect of HPR on serum retinol. Animals were divided into three groups and given either DMSO, 10 mg/kg HPR, or 20 mg/kg HPR for 14 days. At the end of the study period, blood was collected from the animals, sera were prepared and an acetonitrile extract of the serum was analyzed by reverse phase LC/MS. UV-visible spectral and mass/charge analyses were performed to confirm the identity of the eluted peaks. Sample chromatograms obtained from these analyses are shown: Fig. 7a. - extract from an *abca4* null mutant mouse receiving HPR vehicle, DMSO; Fig. 7b. - 10 mg/kg HPR; Fig. 7c. - 20 mg/kg HPR. The data clearly show a dose-dependent reduction in serum retinol. Quantitative data indicate that at 10 mg/kg HPR, all-*trans* retinol is decreased 40%, *see* Fig. 8. For 20 mg/kg HPR, serum retinol is decreased 72%, *see* Fig. 8. The steady state concentrations of retinol and HPR in serum (at 20mg/kg HPR) were determined to be 2.11 µM and 1.75 µM, respectively.

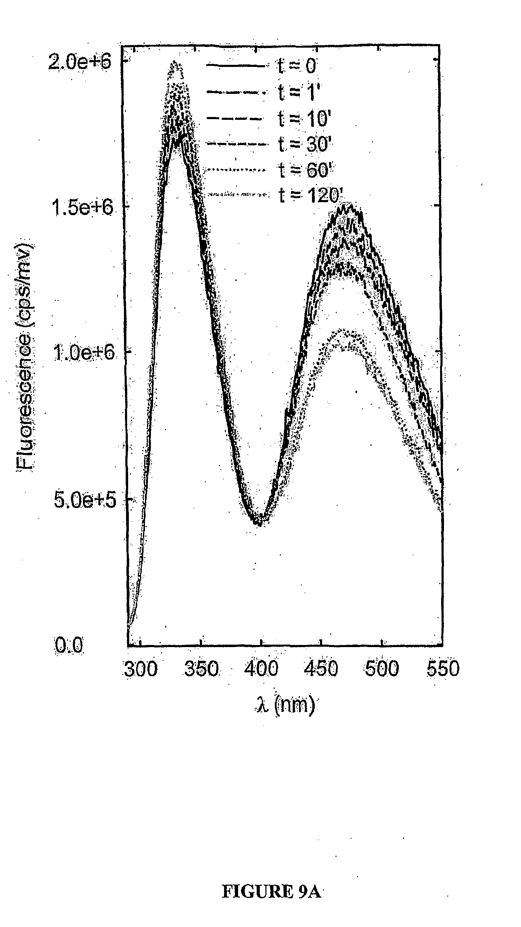

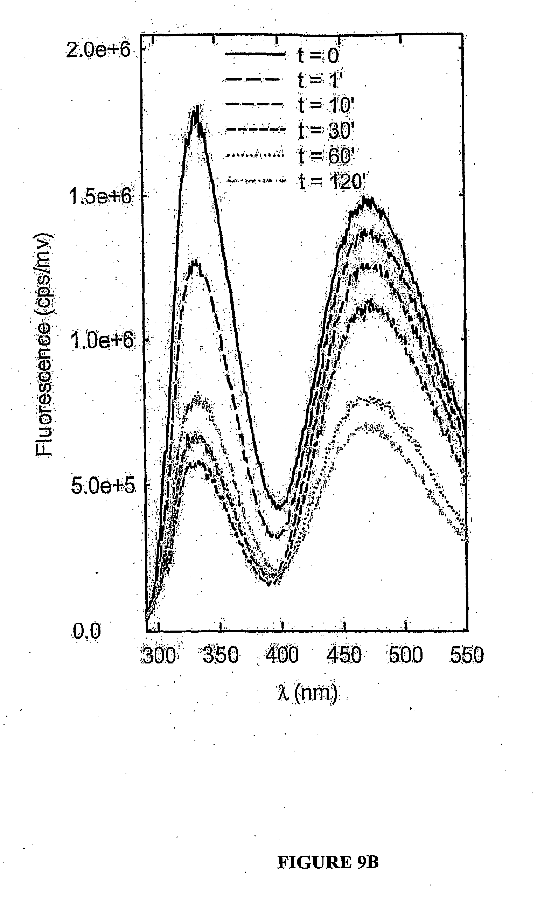

Based upon these findings, we sought to further explore the mechanism(s) of retinol reduction during HPR treatment. A tenable hypothesis is that HPR may displace retinol by competing at the retinol binding site on RBP. Like retinol, HPR will absorb (quench) light energy in the region of protein fluorescence; however, unlike retinol, HPR does not emit fluorescence. Therefore, one can measure displacement of retinol from the RBP holoprotein by observing decreases in both protein (340 nm) and retinol (470 nm) fluorescence. We performed a competition binding assay using RBP-retinol/HPR concentrations which were similar to those determined from the 14 day trial at 20 mg/kg HPR described above. Data obtained from these analyses reveal that HPR efficiently displaces retinol from the RBP-retinol holoprotein at physiological temperature, *see* Fig. 9b. The competitive binding of HPR to RBP was dose-dependent and saturable. In the control assays, decreases in retinol fluorescence were associated with concomitant increases in protein fluorescence, *see* Fig. 9a. This effect was determined to be due to temperature effects as the dissociation constant of RBP-retinol increases (decreased affinity) with increased time at 37C. In summary, these data suggest that increases of HPR beyond equimolar equivalents, relative to RBP holoprotein (e.g., 1.0 µM HPR, 0.5 µM RBP), will cause a significant fraction of retinol to be displaced from RBP *in vivo.*

### Example 15: Efficacy of Fenretinide on the Accumulation of Lipofuscin (and/or A2E) in abca4 null Mutant Mice: Phase II - Chronic Treatment of abca4 Null Mutant Mice.

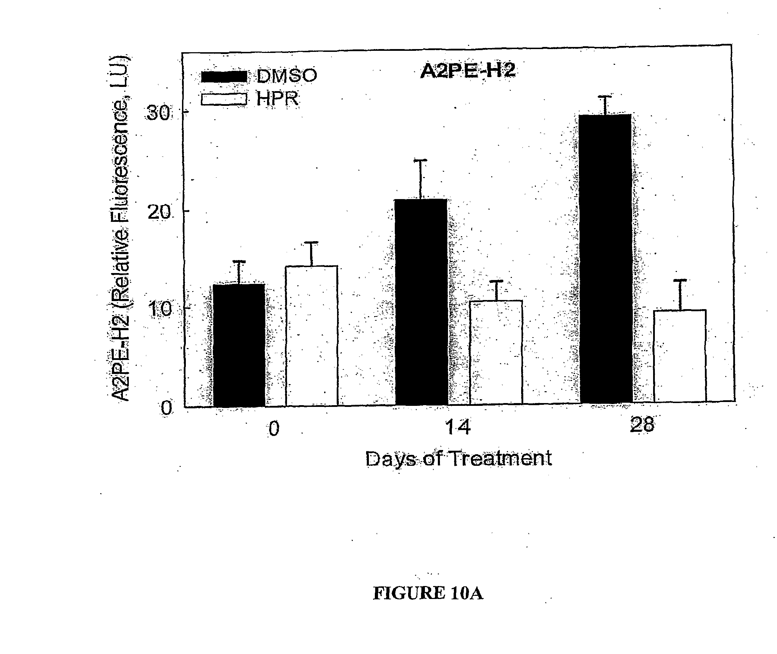

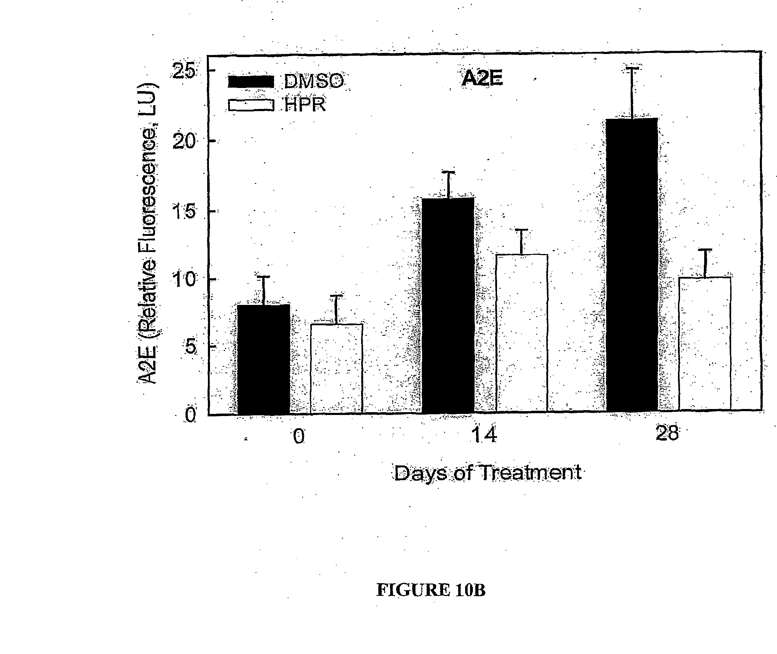

We initiated a one-month study to evaluate the effects of HPR on reduction of A2E and A2E precursors in *abca4* null mutant mice. HPR was administered in DMSO (20 mg/kg, ip) to *abca4* null mutant mice (BL6/129, aged 2 months) daily for a period of 28 days. Control age/strain matched mice received only the DMSO vehicle. Mice were sampled at 0, 14, and 28 days (n = 3 per group), the eyes were enucleated and chloroform-soluble constituents (lipids, retinoids and lipid-retinoid conjugates) were extracted. Mice were sacrificed by cervical dislocation, the eyes were enucleated and individually snap frozen in cryo vials. The sample extracts were then analyzed by HPLC with on-line fluorescence detection. Results from this study show remarkable, early reductions in the A2E precursor, A2PE-H2, *see* Fig. 10a, and subsequent reductions in A2E, *see* Fig. 10b. Quantitative analysis revealed a 70% reduction of A2PE-H2 and 55% reduction of A2E following 28 days of HPR treatment. A similar study may be undertaken to ascertain effects of HPR treatment on the electroretinographic and morphological phenotypes.

### Example 16: Fluorescence Quenching Study of MPR Binding to Retinol Binding Protein (RBP)

Apo-RBP at 0.5 µM was incubated with 0, 0.25, 0.5, 1 and 2 µM of MPR in PBS at room temperature for 1 hour, respectively. As controls, the same concentration of Apo-RBP was also incubated with 1 µM of HPR or 1 µM of atROL. All mixtures contained 0.2% Ethanol (v/v). The emission spectra were measured between 290 nm to 550 nm with excitation wavelength at 280 nm and 3 nm bandpass.

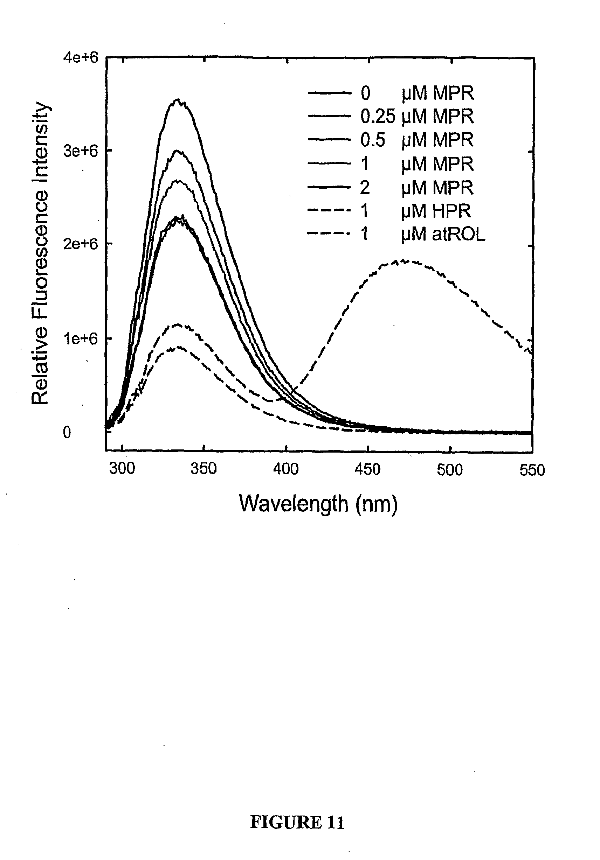

As shown in FIG. 11, MPR exhibited concentration-dependent quenching of RBP fluorescence, and the quenching saturated at 1 µM of MPR for 0.5 µM of RBP. Because the observed fluorescence quenching is likely due to fluorescence resonance energy transfer between protein aromatic residues and bound MPR molecule, MPR is proposed to bind to RBP. The degree of quenching by MPR is smaller than those by atROL and HPR, two other ligands that bind to RBP.

### Example 17: Size Exclusion Study of Transthyretin (TTR) Binding to RBP

Apo-RBP at 10 µM was incubated with 50 µM of MPR in PBS at room temperature for 1 hour. 10 µM of TTR was then added to the solution, and the mixture was incubated for another hour at room temperature. 50 µlof the sample mixtures with and without TTR addition were analyzed by BioRad Bio-Sil SEC125 Gel Filtration Column (300x7.8 mm). In control experiments, atROL-RBP and atROL-RBP-TTR mixture were analyzed in the same manner.

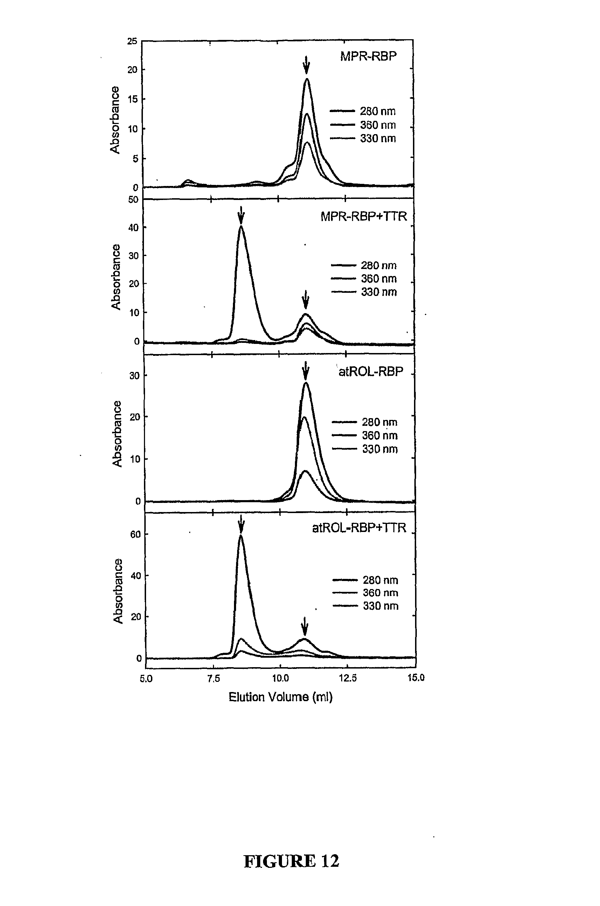

As shown in FIG. 12a, the MPR-RBP sample exhibited an RBP elution peak (at 11 ml) with strong absorbance at 360 nm, indicating RBP binds to MPR; after incubation with TTR, this 360 nm absorbance stayed with the RBP elution peak, while TTR elution peak (at 8.6 ml) did not contain any apparent 360 nm absorbance (see FIG. 12b), indicating MPR-RBP did not bind to TTR. In atROL-RBP control experiment, RBP elution peak showed strong 330 nm absorbance (see FIG. 12c); after incubation with TTR, more than half of this 330 nm absorbance shifted to TTR elution peak (see FIG. 12d), indicating atROL-RBP binds to TTR. Thus, MPR inhibits the binding of TTR to RBP.

### Example 18: Analysis of serum retinol as a function of HPR concentration

ABCA4 null mutant mice were given the indicated dose of HPR in DMSO (i.p.) daily for 28 days (n = 4 mice per dosage group). At the end of the study period, blood samples were taken and serum was prepared. Following acetonitrile precipitation of serum proteins, the concentrations of retinol and HPR were determined from the soluble phase by LC/MS (see FIG. 8). Identity of the eluted compounds was confirmed by UV-vis absorption spectroscopy and co-elution of sample peaks with authentic standards.

### Example 19: Correlation of HPR concentration to reductions in retinol, A2PE-H₂ and A2E in ABCA4 null mutant mice

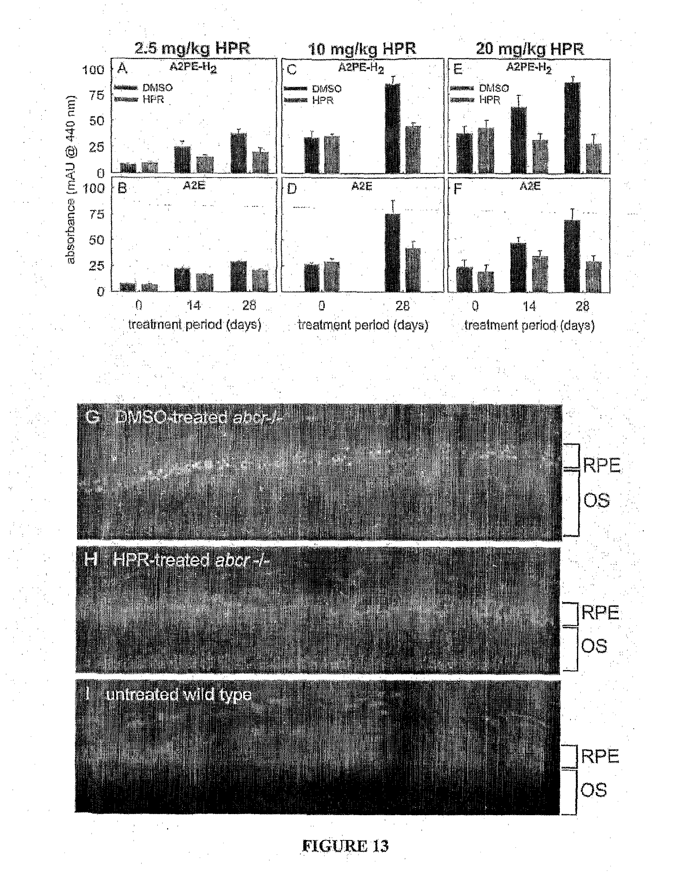

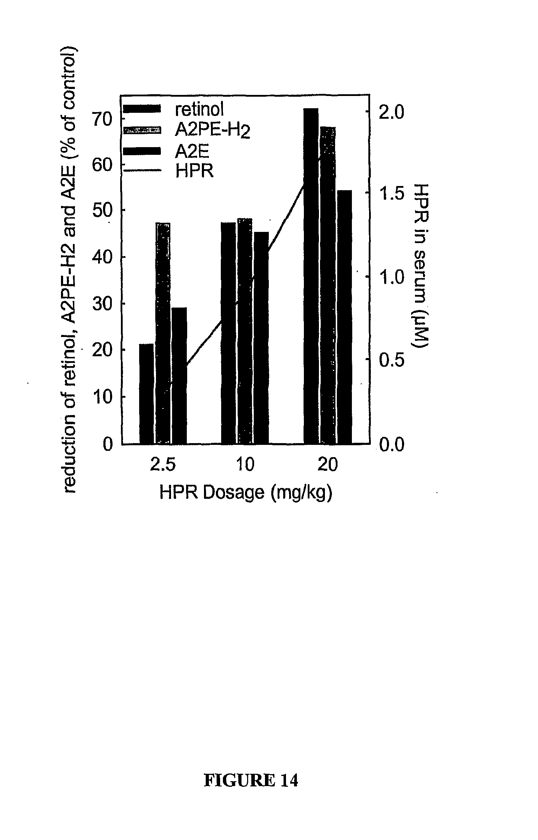

Group averages from the data shown in panels A - G of FIG. 13 in Example 25 (28 day time points) are plotted to illustrate the strong correlation between increases in serum HPR and decreases in serum retinol (see FIG. 14). Reductions in serum retinol are highly correlated with reductions in A2E and precursor compounds (A2PE-H₂). A pronounced reduction in A2PE-H₂ in the 2.5 mg/kg dosage group (∼47%) is observed when the serum retinol reduction is only 20%. The reason for this disproportionate reduction is related to the inherently lower ocular retinoid content in this group of 2-month old animals compared to the other groups. It is likely that if these animals had been maintained on the 2.5 mg/kg dose for a more prolonged period, a greater reduction in A2E would also be realized.

### Example 20: Effects of HPR on Steady State Concentrations of Retinoids, A2E Fluorophores, and Retinal Physiology

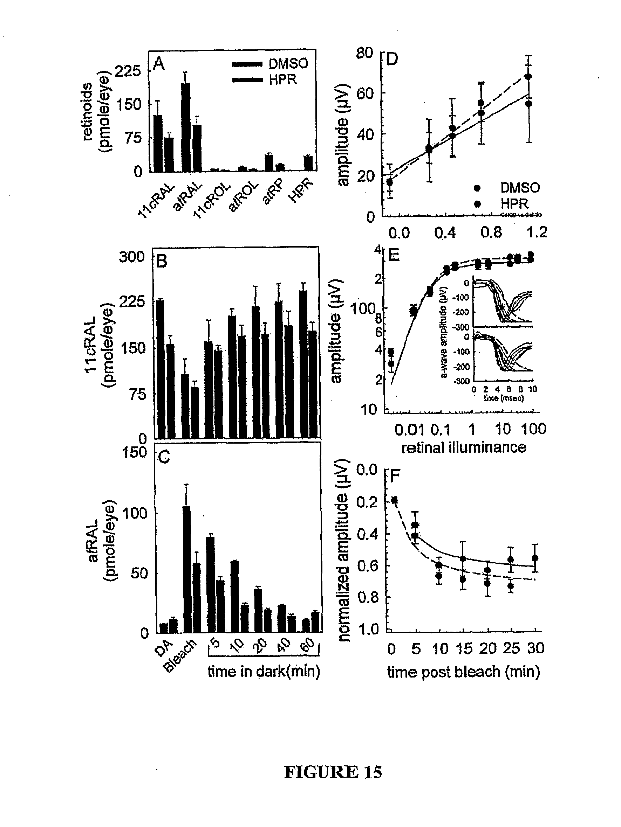

Analysis of retinoid composition in light adapted DMSO- and HPR-treated mice (FIG. 15, panel A) shows approximately 50% reduction of visual cycle retinoids as a result of HPR treatment (10 mg/kg daily for 28 days). Panels B and C of FIG. 15 show that HPR does not affect regeneration of visual chromophore in these mice (panel B is visual chromophore biosynthesis, panel C is bleached chromophore recycling). Panels D - F of FIG. 15 are electrophysiological measurements of rod function (panel D), rod and cone function (panel E) and recovery from photobleaching (panel F). The only notable difference is delayed dark adaptation in the HPR-treated mice (panel F).

*ABCA4* null mutant mice were given the indicated dose of HPR in DMSO or DMSO alone daily for 28 days (n = 16 mice per treatment group). At study onset, mice in the 2.5 mg/kg group were 2 months of age, mice in the other treatment groups were 3 months of age. At the indicated times, representative mice were taken from each group (n = 4) for analysis of A2E precursor compounds (see FIG. 13, A2PE-H₂, panels A, C and E) and A2E (see FIG. 13, panels B, D and F). Eyes were enucleated, hemisected and lipid soluble components were extracted from the posterior pole by chloroform/methanol-water phase partitioning. Sample extracts were analyzed by LC. Identity of the eluted compounds was confirmed by UV-vis absorption spectroscopy and co-elution of sample peaks with authentic standards. Note: limitations in appropriately age and strain-matched mice in the 10 mg/kg group prevented analysis at the 14-day interval. The data show dose-dependent reductions of A2PE-H₂ and A2E during the study period.

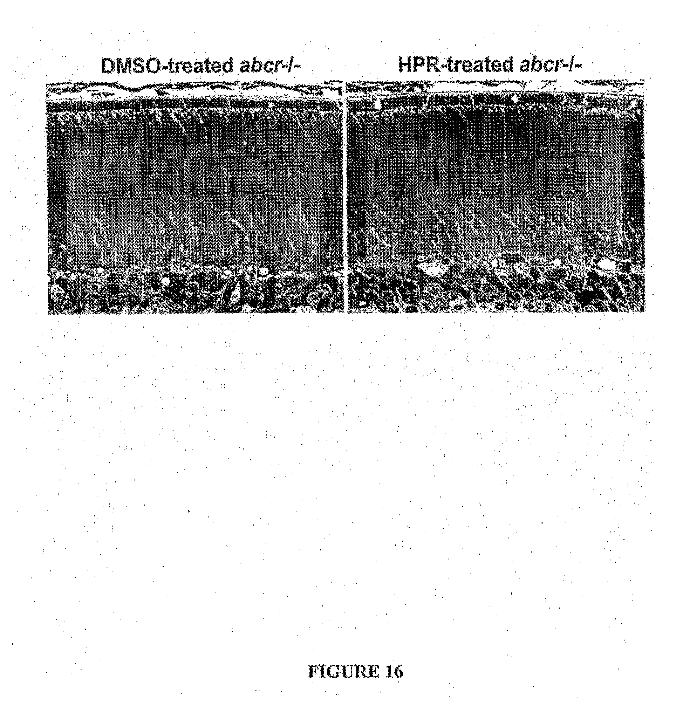

Panels G - I in FIG. 13 show morphological/histological evidence that HPR significantly reduces lipofuscin autofluorescence in the RPE of abcr null mutant mice (Stargardt's animal model). Treatment conditions are as described above. The level of autofluorescence in the HPR-treated animal is comparable to that of an age-matched wild-type animal. FIG. 16 shows light microscopy images of the retinas from DMSO- and HPR-treated animals. No aberrant morphology or compromise of the integrity in retinal cytostructure was observed.

Accumulation of lipofuscin in the retinal pigment epithelium (RPE) is a common pathological feature observed in various degenerative diseases of the retina. A toxic vitamin A-based fluorophore (A2E) present within lipofuscin granules has been implicated in death of RPE and photoreceptor cells. In these experiments, we employed an animal model which manifests accelerated lipofuscin accumulation to evaluate the efficacy of a therapeutic approach based upon reduction of serum vitamin A (retinol). Fenretinide potently and reversibly reduces serum retinol. Administration of HPR to mice harboring a null mutation in the Stargardt's disease gene (*ABCA4*) produced profound reductions in serum retinol/retinol binding protein and arrested accumulation of A2E and lipofuscin autofluorescence in the RPE. Physiologically, HPR-induced reductions of visual chromophore were manifest as modest delays in dark adaptation; chromophore regeneration kinetics were normal. Importantly, specific intracellular effects of HPR on vitamin A esterification and chromophore mobilization were also identified. These findings demonstrate the vitamin A-dependent nature of A2E biosynthesis and validate a therapeutic approach which is readily transferable to human patients suffering from lipofuscin-based retinal diseases.

### Example 21: Benefits of HPR Therapy Persist During Drug Holiday

HPR (10 mg/kg in DMSO) was administered to *ABCA4-*/*-* mice daily for a period of 28 days. Control *ABCA4-*/*-* mice received only DMSO for the same period. Biochemical (HPLC) analysis of the A2E precursor (A2PE-H₂) and A2E following a 28-day treatment period revealed a reduction of these fluorophores in the eyes of HPR-treated mice (FIG. 13). Further analysis by fluorescence microscopy corroborated the biochemical data and revealed that lipofuscin autofluorescence levels of HPR-treated *ABCA4-*/*-* mice were comparable to levels observed in untreated wild type mice (FIG. 13). Histological examinations by light microscopy showed no alteration of retina cytostructure or morphology (FIG. 16). Importantly, the observed reductions in lipofuscin autofluorescence persist long after cessation of HPR therapy. HPR (10 mg/kg), or DMSO, administration was discontinued following 28 days of treatment and re-evaluated A2E and precursor levels after 2 weeks and after 4 weeks.

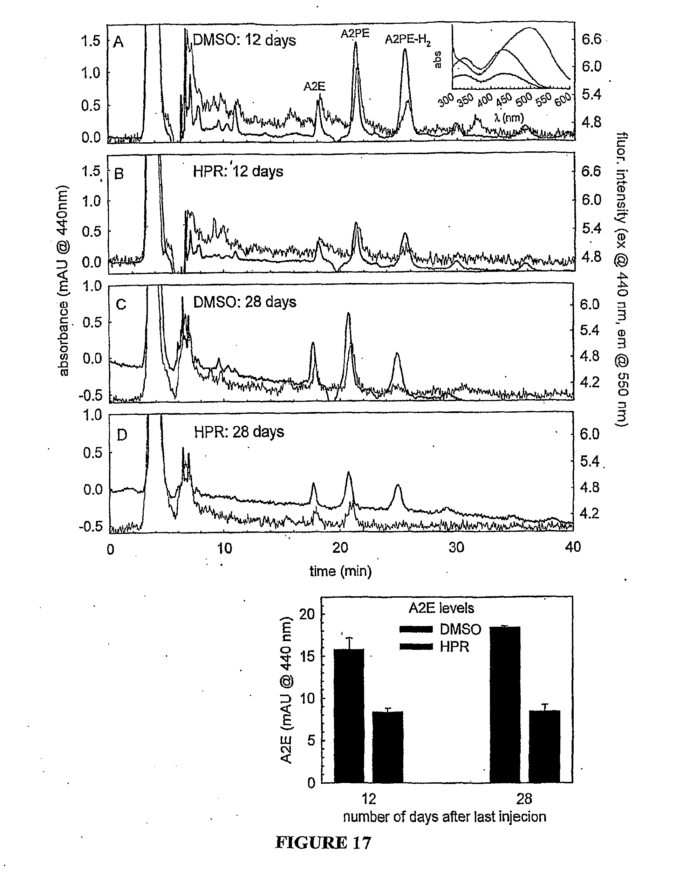

We examined eyecup extracts by HPLC and employed detection by absorbance and fluorimetry. Identity of the indicated peaks was confirmed by on-line spectral analysis and by co-elution with authentic standards. The data show that in animals that had been previously maintained on HPR therapy (FIG. 17, panel A), A2E and precursor (A2PE-H₂ and A2PE) levels remain significantly reduced relative to control mice (FIG. 17, panel B) even after 12 days without receiving a dose of HPR (i.e., a 12-day drug holiday). Similar results were observed in mice following a 28-day drug holiday: A2E and precursor (A2PE-H₂ and A2PE) levels remain significantly reduced relative to control mice (compare FIG. 17, panel C, treated mice, with FIG. 17, panel D, control mice). Further, the A2E and precursor (A2PE-H₂ and A2PE) levels after a 12- or 28-day drug holiday remained at or near the levels immediately following 28 days of treatment (i.e., ca. 50% reduction relative to control), although after the 28-day drug holiday, the amount of A2E and precursor (A2PE-H₂ and A2PE) had increased by a few percentage points relative to the 12-day drug holiday levels. Despite the persistent reduction in the levels of A2E and precursor (A2PE-H₂ and A2PE) in the eyes of animals on an HPR drug holiday, we were unable to detect either HPR or HPR metabolites (e.g., MPR) in the eyes of the animals on a 28-day drug holiday. The trace in FIG. 17, panels C and D, shows the intensity of autofluorescence associated with the indicated peaks. It is clear that peak fluorescence tracks with the abundance of A2E, A2PE and A2PE-H₂.

These data bear on toxicity during clinical trials by maintaining patients on a reduced HPR dose following proof of clinical efficacy at a higher dose. This analysis may obviate the need for additional corroboration by microscopy. To our knowledge this effect has not been observed with other methods for treating an ophthalmic condition or trait selected from the group consisting of Stargardt Disease, dry-form age-related macular degeneration, a lipofuscin-based retinal degeneration, photoreceptor degeneration, and geographic atrophy. Nor has this effect been observed with methods for reducing the formation of *N*-retinylidene-*N*-retinylethanolamine in an eye of a mammal, or methods for reducing the formation of lipofuscin in an eye of a mammal. HPR reduces serum retinol levels, which leads to a reduction in the level of retinol in the eyes of treated animals. Once the level of retinol has been reduced in the eye, there is a time lag in the subsequent increase in retinol levels in the eye. Alone or in combination, the production of A2E, A2PE and A2PE-H₂ in the eye remains low despite the absence of HPR in the serum or the eye.

### Example 22: Validation of RBP as a therapeutic target for arresting accumulation of A2E

We have explored a non-pharmacological means of reducing lipofuscin fluorophores in order to validate our therapeutic approach based upon reduction of RBP levels in a patient. In this study, RBP protein levels have been reduced through genetic manipulation. Two new lines of mice expressing heterozygous mutations in retinol binding protein (*RBP*4) have been generated. The first line carries a heterozygous mutation only at the RBP locus (RBP+/-); the second line carries heterozygous mutations at both *ABCA*4 and RBP loci (*ABCA*4+/-/*RBP*4 +/-). Thus, both lines demonstrate a ∼ 50% reduction in RBP expression and serum retinol. The RBP+/- mice will be wild type at the *ABCA*4 locus and, therefore, do not accumulate excessive amounts of A2E fluorophores. However, *ABCA*4+/- mice will accumulate A2E fluorophores at levels which are approximately 50% of that observed in *ABCA*4-/- (null homozygous) mice. At issue is whether the reduced expression of RBP in the *ABCA*4+/-/RBP+/- mice will have an effect on the accumulation of A2E fluorophores.

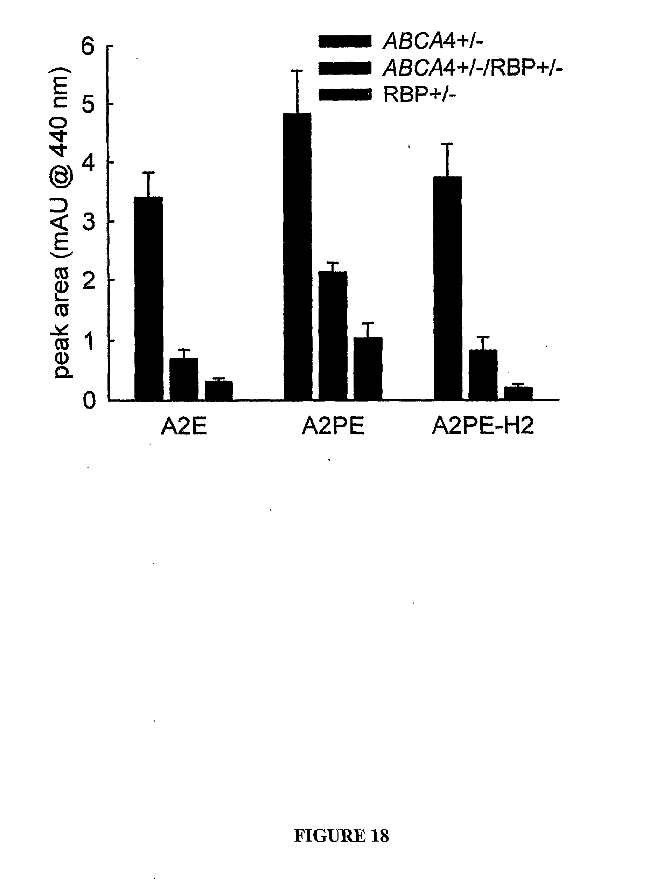

The levels of A2E and precursor fluorophores (A2PE and A2PE-H₂) in these mice have been monitored monthly over a three month period and compared to the fluorophore levels in *ABCA*4+/- mice. The data provide fluorophore levels in the three lines of mice at three months of age (FIG. 18). Overall, the *ABCA*4+/-/RBP+/- mice demonstrate a ∼ 70% reduction in total fluorophore level relative to the levels present in *ABCA*4+/- mice. In fact, the measured fluorophore levels in the *ABCA*4+/-/RBP+/- mice approach that observed in RBP+/- mice. These data validate RBP as a therapeutic target for reducing fluorophore levels in the eye. Further, these data demonstrate that agents or methods that inhibit the transcription or translation of RBP in a patient will also (a) reduce serum retinol levels in that patient, and (b) provide a therapeutic benefit in the retinol-related diseases described herein. Further, agents or methods that enhance the clearance of RBP in a patient will also produce such effects and benefits.

All of the methods disclosed and claimed herein can be made and executed without undue experimentation in light of the present disclosure. It will be apparent to those of skill in the art that variations may be applied without departing from the concept, spirit and scope of the invention. More specifically, it will be apparent that certain agents that both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### RELEVANT PARAGRAPHS:

1. A method for treating retinol-related diseases in a mammal, comprising administering to the mammal at least once an effective amount of at least one of the compounds chosen from the group consisting of: an RBP transcription inhibitor, a TTR transcription inhibitor, an RBP translation inhibitor, a TTR translation inhibitor, an RBP clearance agent, a TTR clearance agent, an RBP antagonist, an RBP agonist, a TTR antagonist, a TTR agonist and a retinol binding receptor antagonist.
2. The method of paragraph 1, wherein the RBP transcription inhibitor is chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, Zn-finger binding proteins, ribozymes and monoclonal antibodies.
3. The method of paragraph 1, wherein the TTR transcription inhibitor is chosen from the group consisting of RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, HNF-4 agonists, HNF-4 antagonists, aptamers, Zn-finger binding proteins, ribozymes and monoclonal antibodies.
4. The method of paragraph 1, wherein the RBP translation inhibitor is chosen from the group consisting of: RXR/RAR agonists, RXR/RAR antagonists, estrogen agonists, estrogen antagonists, testosterone agonists, testosterone antagonists, progesterone agonists, progesterone antagonists, dexamethasone agonists, dexamethasone antagonists, antisense oligonucleotides, siRNA, HNF-4 agonists, HNF-4 antagonists, aptamers, ribozymes and monoclonal antibodies.
5. The method of paragraph 1, wherein the TTR translation inhibitor is chosen from the group consisting of: antisense oligonucleotides, siRNA, fatty acid binding protein antagonists, C/EBP agonists, C/EBP antagonists, HNF-1 agonists, HNF-1 antagonists, HNF-3 agonists, HNF-3 antagonists, HNF-4 agonists, HNF-4 antagonists, HNF-6 agonists, HNF-6 antagonists, aptamers, ribozymes and monoclonal antibodies.
6. The method of paragraph 1, wherein the RBP clearance agent is chosen from the group consisting of: a retinyl derivative, a polyhalogenated aromatic hydrocarbon, thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.
7. The method of paragraph 6, wherein said retinyl derivative is N-(4-hydroxyphenyl)retinamide or N-(4-methoxyphenyl)retinamide.
8. The method of paragraph 1, wherein the TTR clearance agent is chosen from the group consisting of: a thyroid hormone agonist, thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, a polyhalogenated aromatic hydrocarbon and an antibody.
9. The method of paragraph 1, wherein the RBP agonist or antagonist inhibits the binding of retinol to RBP.
10. The method of paragraph 9, wherein said RBP agonist or antagonist is selected from N-(4-hydroxyphenyl)retinamide and N-(4-methoxyphenyl)retinamide.
11. The method of paragraph 1, wherein the TTR agonist or antagonist is chosen from the group consisting of: a polyhalogenated aromatic hydrocarbon, a thyroid hormone agonist, a thyroid hormone antagonist, diclofenac, a diclofenac analogue, a small molecule compound, an endocrine hormone analogue, a flavonoid, a non-steroidal anti-inflammatory drug, a bivalent inhibitor, a cardiac agent, a peptidomimetic, an aptamer, and an antibody.
12. The method of paragraph 1, wherein the retinol binding protein receptor antagonist is an inhibitor of retinyl palmitate hydrolase.
13. The method of paragraph 1, further comprising administration of a second compound selected from the group consisting of an inducer of nitric oxide production, an antioxidant, an anti-inflammatory agent, a mineral, an anti-oxidant, a carotenoid, a negatively charged phospholipid and a statin.
14. The method of paragraph 1, wherein administration of the compound prevents age-related macular degeneration or dystrophy in an eye of the mammal.
15. The method of paragraph 1, wherein the retinol-related disease is hyperostosis, idiopathic intracranial hypertension, amyloidosis, Alzheimer's disease, and Alström-Hallgren syndrome.
16. A method of treating age-related macular degeneration or dystrophy, comprising administering to a mammal at least once an effective amount of a first compound, wherein said first compound modulates RBP or TTR levels or activity in the mammal.
17. The method of paragraph 16 wherein the first compound inhibits the binding of retinol to RBP.
18. The method of paragraph 16, wherein said first compound inhibits transcription of RBP or TTR in the mammal.
19. The method of paragraph 16, wherein said first compound inhibits translation of RBP or TTR in the mammal.
20. The method of paragraph 16, wherein said first compound increases RBP or TTR clearance in the mammal.
21. The method of paragraph 16, wherein said first compound inhibits RBP binding to TTR.
22. The method of paragraph 16, further comprising administration of a second compound selected from the group consisting of an inducer of nitric oxide production, an antioxidant, an anti-inflammatory agent, a mineral, an anti-oxidant, a carotenoid, a negatively charged phospholipid, a complement inhibitor, a fish oil, and a statin.
23. The method of paragraph 16, wherein the effective amount of the compound is systemically administered to the mammal.
24. The method of paragraph 16, wherein the mammal is a human.
25. The method of paragraph 16, wherein the macular degeneration is dry form age-related macular degeneration.
26. The method of paragraph 16, wherein the macular degeneration comprises geographic atrophy in at least one eye of the mammal.
27. The method of paragraph 16, wherein the first compound has the structure of Formula (II).
28. The method of paragraph 27, wherein the first compound is N-(4-hydroxyphenyl)retinamide or N-(4-methoxyphenyl)retinamide.
29. A method for treating type I or type II diabetes in a mammal, comprising administering to the mammal at least once an effective amount of a first compound, wherein said first compound modulates RBP or TTR levels or activity in the mammal.
30. The method of paragraph 29 wherein the first compound inhibits the binding of retinol to RBP.
31. The method of Paragraph 29, wherein the first compound inhibits transcription of RBP or TTR in the mammal. 32. The method of Paragraph 29, wherein the first compound inhibits translation of RBP or TTR in the mammal.
33. The method of Paragraph 29, wherein the first compound increases RBP or TTR clearance in the mammal.
34. The method of Paragraph 29, wherein the first compound antagonizes or inhibits RBP binding to TTR.
35. The method of paragraph 29 wherein the first compound has the structure of Formula (II).
36. The method of paragraph 35, wherein the first compound is N-(4-hydroxyphenyl)retinamide or N-(4-methoxyphenyl)retinamide.
37. The method of paragraph 29, further comprising administration of a second agent selected from the group consisting of: comprise administration of a second compound selected from the group consisting of (a) a glucose-lowering hormone or hormone mimetic, (b) a glucose-lowering sulfonylurea, (c) a glucose- lowering biguanide, (d) a glucose-lowering meglitinide, (e) a glucose-lowering thiazolidinedione or other PPAR-gamma agonist, (f) a glucose-lowering dual-acting PPAR agonist with affinity for both PPAR- gamma and PPAR-alpha, (g) a glucose-lowering alpha-glucosidase inhibitor, (h) a glucose-lowering antisense compound not targeted to glucose-6-phosphatase translocase, (i) an anti-obesity appetite suppressant, (j) an anti-obesity fat absorption inhibitor, (k) an anti-obesity modified form of ciliary neurotrophic factor which inhibits hunger signals that stimulate appetite, (I) a lipid-lowering bile salt sequestering resin, (m) a lipid-lowering HMGCoA-reductase inhibitor, (n) a nicotinic acid, (o) a lipid-lowering fibric acid derivative, (p) an agent selected from probucol, neomycin, and dextrothyroxine, (q) a plant-stanol ester, (r) a cholesterol absorption inhibitor, (s) a CETP inhibitor, (t) a MTP inhibitor, (u) an inhibitor of bile acid transporters, (v) a regulator of hepatic CYP7a, (w) an ACAT inhibitor, (x) a lipid-lowering estrogen replacement therapeutic, (y) a synthetic HDL, and (z) a lipid-lowering anti-inflammatory agent.
38. The method of paragraph 29, wherein a decrease in the RBP level or activity in the mammal decreases insulin levels in the patient.

## Claims

1. A compound of Formula (II) for use for the manufacture of a medicament for modulating serum retinol binding protein (RBP) or transthyretin (TTR) levels or activity in a mammal with hyperostosis, or Alzheimer's disease, or Idiopathic Intracranial Hypertension or Bone-Related Disorders or Protein Misfolding and Aggregation Diseases or Alström-Hallgren Syndrome, wherein the compound of Formula (II) has the structure: 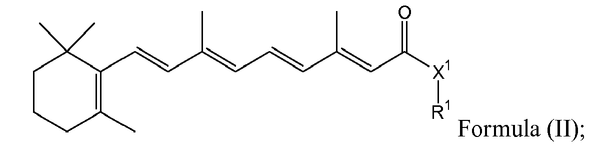 wherein X¹ is selected from the group consisting of NR²; R¹ is (CHR²)ₓ-L¹-R³, wherein x is 0, 1, 2, or 3; L¹ is a single bond or -C(O)-; R² is H or (C₁-C₄)alkyl; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloakenyl, and a heterocycle; or a pharmaceutically acceptable salt thereof.

2. A compound of Formula (II) for use as claimed in claim 1 the manufacture of a medicament for treating hyperostosis, or Alzheimer's disease, Idiopathic Intracranial Hypertension or Bone-Related Disorders or Protein Misfolding and Aggregation Diseases or Alström-Hallgren Syndrome, wherein the compound of Formula (II) has the structure: 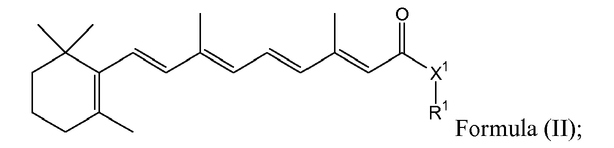 wherein X¹ is selected from the group consisting of NR²; R¹ is (CHR²)ₓ-L¹-R³, wherein x is 0, 1, 2, or 3; L¹ is a single bond or -C(O)-; R² is H or (C₁-C₄)alkyl; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloakenyl, and a heterocycle; or a pharmaceutically acceptable salt thereof.

3. A compound of Formula II for use as claimed in claim 1 or 2, wherein the mammal is a human.

4. A compound of Formula II for use as claimed in any of claims 1 to 3, wherein the compound of Formula (II) inhibits the binding of retinol to RBP; or wherein said compound of Formula (II) inhibits transcription of RBP or TTR; or wherein said compound of Formula (II) inhibits translation of RBP or TTR; or wherein said compound of Formula (II) increases RBP or TTR clearance; or wherein said compound of Formula (II) inhibits RBP binding to TTR.

5. A compound of Formula II for use as claimed in any of claims 1 to 4, wherein said medicament is formulated for systemic administration.

6. A compound of Formula II for use as claimed in any of claims 1 to 4, wherein the medicament is formulated for oral administration.

7. A compound of Formula II for use as claimed in claim 6, wherein the medicament is in the form of a tablet, powder, pill, dragee, capsule, liquid, gel, syrup, elixir, slurry or suspension.

8. A compound of Formula II for use as claimed in any of claims 1 to 7, wherein x is 0, X¹ is NH and R³ is an optionally substituted aryl group, wherein the aryl group has one substituent selected from the group consisting of halogen, -OH, -O(C₁-C₄)alkyl, -NH(C₁-C₄)alkyl, -O(C₁-C₄)fluoroalkyl, and -N[(C₁-C₄)alkyl]₂.

9. A compound of Formula II for use as claimed in any of claims 1 to 7, wherein the compound is selected from among N-(4-hydroxyphenyl)retinamide (HPR); N-(4-methoxyphenyl)retinamide (MPR); N-(4-hydroxyphenyl) retinmide-O-glucuronide; 4-(retinamido)phenyl-C-glucuronide; 4-(retinamido)phenyl-C-glucoside; 4-(retinamido)phenyl-C-xyloside; 4-(retinamido)benzyl-C-glucuronide; 4-(retinamido)benzyl-C-glucoside; 4-(retinamido)benzyl-C-xyloside; 1-(β-D-glucopyranosyl)retinamide; and 1-(D-glucopyranosyluronosyl)retinamide; or a pharmaceutically acceptable salt thereof.

10. A compound of Formula II for use as claimed in any of claims 1 to 7, wherein the compound of Formula (II) is N-(4-hydroxyphenyl)retinamide.

11. A compound of Formula II for use as claimed in any of claims 1 to 7, wherein the compound of Formula (II) is N-(4-methoxyphenyl)retinamide.
